(19) Europäisches Patentamt
European Patent Office
Office européen des brevets

(11) Numéro de publication : **0 113 599 B1**

(12) **FASCICULE DE BREVET EUROPÉEN**

(45) Date de publication du fascicule du brevet : 01.02.89

(21) Numéro de dépôt : 83402109.9

(22) Date de dépôt : 27.10.83

(51) Int. Cl.⁴ : C 07 H 3/04, C 07 H 3/06, C 07 H 11/00, A 61 K 31/70, A 61 K 31/725, C 08 B 37/10, G 01 N 33/48, C 07 H 7/02

**ERRATUM**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | LAUTET BERICHTIGT:<br>SHOULD READ :<br>DEVRAIT ETRE LU : |
|---|---|---|---|
| un motif U comportant | 27 | 33 | un motif A comportant |
| un motif A comportant ledit | 27 | 34 | un motif U comportant ledit |
| is present on the U unit | 33 | 26 | is present on the A unit |
| to the left on the A unit, | 33 | 26 | to the left on the U unit, |
| U-A nach rechts einen Rest U, | 39 | 14 | U-A nach rechts einen Rest A, |
| links einen Rest A, | 39 | 15 | links einen Rest U, |

Tag der Entscheidung über die Berichtigung / Date of decision on rectification / Date de décision portant sur modification: 8.1.90

Ausgabe- und Veröffentlichungstag / Issue and publication date / Date d'edition et de publication: 14.3.90

Patbl.Nr / EPB no / Bull. no: 90/11

(19) Europäisches Patentamt
European Patent Office
Office européen des brevets

(11) Numéro de publication : **0 113 599**
**B1**

(12) **FASCICULE DE BREVET EUROPÉEN**

(45) Date de publication du fascicule du brevet : **01.02.89**

(21) Numéro de dépôt : **83402109.9**

(22) Date de dépôt : **27.10.83**

(51) Int. Cl.⁴ : **C 07 H 3/04**, C 07 H 3/06, C 07 H 11/00, A 61 K 31/70, A 61 K 31/725, C 08 B 37/10, G 01 N 33/48, C 07 H 7/02

(54) **Procédé de synthèse organique d'oligosaccharides renfermant des motifs galactosamine-acide-uronique, nouveaux oligosaccharides obtenus et leurs applications biologiques.**

(30) Priorité : **27.10.82 FR 8218003**

(43) Date de publication de la demande : **18.07.84 Bulletin 84/29**

(45) Mention de la délivrance du brevet : **01.02.89 Bulletin 89/05**

(84) Etats contractants désignés : **AT BE CH DE FR GB IT LI LU NL SE**

(56) Documents cités :
**EP-A- 0 014 184**
**EP-A- 0 027 089**
**EP-A- 0 048 231**
**EP-A- 0 064 012**
**EP-A- 0 084 999**
**CHEMICAL ABSTRACTS, vol. 55, no. 26, 25 décembre 1961, colonne 27533e, Columbus, Ohio, US. S.A. BARKER et al.: "Some enzyme transfer reactions involving 2-acetamido-2-deoxy-D-glucose"**
**CARBOHYDRATE RESEARCH, vol. 78, 1980, pages 249-256, Elsevier Scientific Publishing Company, Amsterdam, NL. U. KLEIN et al.: "A 3H-labelled trisaccharide from heparin as substrate for acetyl-CoA: 2-amino-2-deoxy-alpha-D-glucoside N-acetyl-transferase"**

(73) Titulaire : **CHOAY S.A.**
**48, Avenue Théophile-Gautier**
**F-75782 Paris Cédex 16 (FR)**

(72) Inventeur : **Jacquinet, Jean-Claude**
**1, Allée André Gide**
**F-45100 Orleans la Source (FR)**
Inventeur : **Petitou, Maurice**
**Appart. 210 27, rue du Javelot**
**F-75645 Paris Cédex 13 (FR)**
Inventeur : **Sinay, Pierre**
**5, rue Jacques Monot**
**F-45100 Orleans (FR)**
Inventeur : **Choay, Jean**
**21, rue Saint-Guillaume**
**F-75007 Paris (FR)**

(74) Mandataire : **Peaucelle, Chantal et al**
**S.C. Ernest Gutmann - Yves Plasseraud 67, boulevard Haussmann**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 113 599 B1

Jouve, 18, rue St-Denis, 75001 Paris, France

CARBOHYDRATE RESEARCH, vol. 103, no. 1, mai 1982, pages 190-194, Elsevier Scientific Publishing Company, Amsterdam, NL. N. SENO et al.: "Structure of disulfated disaccharides from chondroitin polysulfates, chondroitin sulfate D and K"
CHARBOHYDRATE RESEARCH, vol. 105, no. 1, juillet 1982, pages 69-85, Elsevier Scientific Publishing Company, Amsterdam, NL. A. OGAMO et al.: "Preparation and properties of fluorescent glycosaminoglycuronans labled with 5-aminofluorescein"
PURE & APPL. CHEM., vol. 50, 1978, pages 1437-1452, Pergamon Press Ltd, GB. P. SINAY: "Recent advances in glycosylation reactions"
METHODS CARBOHYDR. CHEM., vol. 8, 1980, pages 305-311, GB. P.A. MANTHORPE et al.: "Allylethers as protecting groups. Synthesis of 3-0-[6-0-(alpha-D-galactopyranosy 1)-beta-D-galactopyranosyl]-1,2-d i-0-stearoyl-L-glycerol a ""digalactosyl diglyceride"""
TETRAHEDRON LETTERS, no. 30, 1972, pages 3055-2058, Pergamon Press, GB. J. KISS et al.: "Syntheses of heparin saccharides. Stereospecific synthesis of derivatives of 2-amino-2-deoxy-4-0-(alpha-D-glucopyranuronosyl)-D-glucose"
HELVETICA CHIMICA ACTA, vol. 58, Fasc. 6, 1975, no. 204-205, pages 1847-1864. P.C. WYSS et al.: "Synthesis of heparin saccharides IV: Synthesis of disaccharides possessing the structure of a repeating unit of heparin"
TETRAHEDRON LETTERS, no. 5, 1972, pages 431-433, Pergamon Press, GB. A. KLEMER et al.: "Synthese von Athyl-2-Amino-2-Desoxy-4-0-(beta-D-Glucuronopyranosyl)-alpha, beta-D-Glucopyranoside", page 431
CARBOHYDRATE RESEARCH, vol. 87, 1980, pages 297-301, Elsevier Scientific Publishing Company, Amsterdam, NL. L. AYOTTE et al.: "Fractionation of heparin and heparan sulfate as barium salts: high-field, n.m.r.-spectal observations on heterogeneity"

(56) Documents cités :
THE JOURNAL OF BIOCHEMISTRY, vol. 92, no. 1, juillet 1982, pages 295-303,Pub. The Japanese Biochemical Society. M. KOSAKAI et al.: "High performance liquid chromatography of pyridylamino derivatives of sulfated oligosaccharides in the deamination products of porcine and whale heparins"
CHEMICAL ABSTRACTS, vol. 97, no. 19, 8 novembre 1982, page 512, no. 160637u, Columbus, Ohio, US. J.J. HOPWOOD et al.: "Diagnosis of Sanfilippo type A syndrome by estimation of sulfamidase activity using a radiolabeled tetrasaccharide substrate"

**Description**

L'invention est relative à un procédé de synthèse organique d'oligosaccharides constituant ou comportant des fragments de mucopolysaccharides acides. Elle concerne également la synthèse de dérivés de ces oligosaccharides.

L'invention se rapporte, en outre, à de nouveaux oligosaccharides du genre indiqué ci-dessus et à leurs dérivés, possédant, notamment, des propriétés biologiques leur conférant, en particulier, un intérêt en tant que médicaments et/ou utilisables, par exemple, comme réactifs de laboratoire.

Elle vise également leurs applications notamment biologiques et biochimiques.

Par le terme « mucopolysaccharide acide », on désigne des dérivés également couramment appelés glycosaminoglycuronoglycanes. Il s'agit d'oligosaccharides et polysaccharides rencontrés plus spécialement dans les chaînes de dérivés biologiquement actifs tels que des dérivés du type dermatane-sulfate, chondroïtines, chondrosine, chondroïtines-sulfates.

Dans les produits naturels, les mucopolysaccharides en question sont essentiellement formés de motifs alternés sucre aminé-acide uronique, ou inversement. Dans ces motifs, le sucre aminé, désigné ci-après par A présente plus spécialement une structure D-galactosamine. L'acide uronique, qu'on appellera U, présente, plus spécialement, une structure acide D-glucuronique ou acide L-iduronique.

Les structures de base pour A et U répondent respectivement aux formules x, y et z ci-dessous :

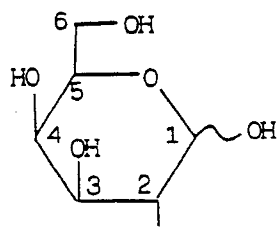

dérivé d'amine (x) D-galactosamine

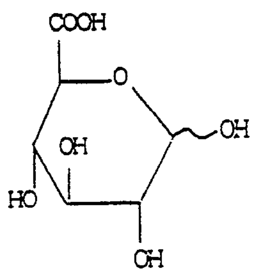

(y) acide D-glucuronique

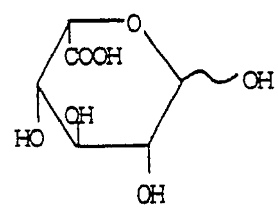

(z) acide L-iduronique

Dans les produits naturels en question, ces différents motifs sont liés entre eux de manière stéréospécifique généralement par des liaisons 1 $\xrightarrow{\alpha}$ 3, 1 $\xrightarrow{\beta}$ 3 et 1 $\xrightarrow{\beta}$ 4.

Dans les chondroïtines et les chondroïtines-sulfates, on rencontre des liaisons de type 1 $\xrightarrow{\beta}$ 3 (entre les motifs y et x) et 1 $\xrightarrow{\beta}$ 4 (entre les motifs x et y).

Des liaisons de type 1 $\xrightarrow{\alpha}$ 3 (entre les motifs z et x et 1 $\xrightarrow{\beta}$ 4 (entre les motifs x et z) existent, en outre, dans le dermatane-sulfate.

On notera, en outre, toujours en se référant aux produits naturels, que les motifs ci-dessus comportent des substitutions spécifiques, c'est-à-dire des substitutions déterminées en des positions données. Les chaînes des produits naturels renferment, ainsi, par exemple, des motifs O-substitués 4 ou 6 ou 4,6-di-O-sulfate-D-galactosamine et des motifs non-O-substitués ; ainsi, par exemple, des motifs acide-D-glucuronique, L-iduronique et D-galactosamine. En outre, le motif x est N-substitué en position 2 par des groupes -N-acétyle.

On connaît l'importance des applications thérapeutiques des mucopolysaccharides acides ci-dessus, en particulier, pour la prévention et pour le traitement des troubles de la coagulation et de la paroi vasculaire, et en particulier des thromboses et des arthéroscléroses et artérioscléroses, ou pour lutter contre le vieillissement des tissus, ou des manifestations de type dégénératif, telles que des alopécies.

Les procédés proposés à ce jour pour obtenir ce type de produits mettent en jeu des techniques d'extraction à partir de sources naturelles, par exemple, d'organes d'animaux.

L'état d'avancement des travaux des inventeurs dans ce domaine les a conduits à rechercher de nouveaux moyens permettant d'accéder à ce type de produits et plus spécialement à étudier les

possibilités de les obtenir par voie de synthèse.

A cet égard, il convient de mesurer le nombre des problèmes soulevés par une telle synthèse. En effet, d'une part, ces produits renferment dans leurs chaînes plusieurs types de motifs A et U. D'autre part, certaines liaisons entre ces motifs répondent à une stéréochimie donnée et sont de type 1,4, dont les difficultés de réalisation particulières sont bien connues. En outre, chaque motif comporte une ou plusieurs substitutions spécifiques selon le type de produit considéré.

Tous ces éléments fixent des impératifs contraignants dont il est aisé d'apprécier les difficultés qu'ils engendrent pour l'élaboration d'un processus général et du processus de synthèse.

Il s'ensuit que de telles synthèses n'ont pratiquement jamais été envisagées jusqu'à présent dans la littérature scientifique, tout particulièrement, en ce qui concerne l'acide L-iduronique.

Les oligosaccharides avec des enchaînements sucre aminé-acide uronique ou l'inverse décrits dans l'art antérieur, sont obtenus pour un grand nombre d'entre eux par des procédés enzymatiques ou par dépolymérisation nitreuse.

Il s'ensuit qu'il ne peut s'agir de produits purs mais de mélanges, qui, en outre, sont constitués par des chaînes modifiées, par rapport aux chaînes naturelles, sous l'action des enzymes ou de l'acide nitreux utilisé dans ces procédés. De telles compositions d'oligosaccharides sont décrites notamment dans les demandes EP-A-0 048 231 et EP-A-0 014 184 au nom de KABI, l'article de Barker et al. visé dans le Chemical Abstract, vol. 55, n° 27533e, l'article de Klein et al. dans Carbohydrate Research, vol. 78, 1980, p. 249-256, la demande EP-A-0 027 089 au nom de la demanderesse, l'article de Seno et al. dans Carbohydrate Research, vol. 103, n° 1, 1982, p. 190-194, l'article d'Ogamo et al. dans Carbohydrate Research, vol. 105, n° 1, 1982, p. 69-85, l'article de Ayotte et al. dans Carbohydrate Research, vol. 87, 1980, p. 297-301, l'article de Kosakai et al. dans J. of Biochemistry, vol. 92, 1982, p. 295-303 et l'article de Hopwood et al. rapporté dans le résumé de Chemical Abstract, vol. 97, 1982, n° 160637u.

D'autres dérivés d'oligosaccharides ont été décrits mais les réactifs et conditions de synthèse utilisés ne constituent pas des solutions aux problèmes que se propose de résoudre l'invention (voir notamment dans Methods Carbohydrate Chem. vol. 8, 1980, p. 305-311, Tetrahedron Letters, n° 30, 1972, p. 3055-58, Helvetica Chimica Acta, vol. 58, 1975, n° 204-205, p. 1847-1864 et Tetrahedron Letters, n° 5, 1972, p. 431-433).

En recherchant des conditions de synthèse osidique appropriées à l'élaboration de ce type de composés, les inventeurs ont développé une stratégie en choisissant certains types de protection particuliers pour les produits mis en œuvre.

Les travaux effectués ont alors montré qu'avec de tels produits ainsi protégés, il était possible de réaliser un enchaînement stéréospécifique puis d'introduire, si souhaité, sur les séquences formées, des substitutions données en des positions déterminées.

Selon un aspect présentant un intérêt dont on mesurera l'importance, le procédé mis au point présente une grande souplesse. Il est ainsi possible d'accéder, avec les avantages, notamment de spécificité et de pureté, liés à un processus de synthèse, à de nombreux dérivés d'oligosaccharides comportant les substitutions spécifiques rencontrées avec les produits naturels, ou même des substitutions différentes et/ou encore des motifs de structure analogue avec des configurations différentes.

Grâce à ce procédé, les inventeurs ont obtenu des produits biologiquement actifs. Le procédé de l'invention permet également d'accéder à un grand nombre d'oligosaccharides particulièrement précieux, notamment, comme réactifs biologiques et/ou comme composés de référence pour des études de structures.

L'invention a donc pour but de fournir un procédé d'obtention, par voie de synthèse, d'oligosaccharides et de leurs dérivés ou analogues, comportant ou correspondant à des fragments de mucopolysaccharides acides.

Elle a également pour but de fournir des moyens permettant d'établir entre des motifs de type A et U des liaisons glycosidiques selon la stéréospécificité souhaitée.

Elle vise également à fournir des moyens permettant d'introduire sur les motifs de la chaîne glycosidique des groupes fonctionnels donnés, en particulier des substituants spécifiques tels que rencontrés dans les chaînes de molécules biologiquement actives, notamment celles du type dermatane-sulfate, chondroïtines-sulfates ou chondroïtines.

Elle a également pour but de fournir des moyens permettant d'obtenir des oligosaccharides tels qu'évoqués ci-dessus, mais dont les substituants et/ou la nature chimique des sucres et/ou la position et la configuration des liaisons interglycosidiques et/ou la configuration des monosaccharides et/ou l'ordre des enchaînements sont différents de ceux des produits naturels.

Selon un autre aspect, l'invention a également pour but de fournir de nouveaux oligosaccharides constituant des produits intermédiaires du procédé de synthèse en question dans lesquels tous les groupes —OH des différents motifs sont bloqués par des groupements protecteurs et les groupements précurseurs des radicaux fonctionnels éventuellement présents ; le cas échéant, ces radicaux eux-mêmes sont également protégés.

Selon encore un autre aspect, l'invention vise à fournir de nouveaux oligosaccharides présentant la structure des produits naturels ci-dessus ainsi que des oligosaccharides correspondant à des fragments de ces produits.

Elle vise également à fournir de nouveaux oligosaccharides possédant les substitutions spécifiques des produits naturels.

L'invention vise, en outre, à fournir de nouveaux oligosaccharides portant des substitutions différentes de celles des substitutions spécifiques en question et/ou comportant des motifs différents par rapport aux produits naturels considérés ci-dessus.

L'invention vise également les applications biologiques de ces oligosaccharides, notamment en tant que substances actives de médicaments, réactifs de laboratoire ou produits de référence pour l'étude, en particulier, de composés comportant ce type de structure.

Le procédé de synthèse de l'invention est caractérisé par le fait

— qu'on met en œuvre, dans une réaction de glycosylation, deux composés terminés ou constitués respectivement par un motif A de structure D-galactosamine et un motif U de structure acide D-glucuronique ou acide L-iduronique, l'un des motifs A ou U étant un alcool dans lequel le groupe —OH de la fonction alcool occupe la position 3 ou 4, l'autre motif possédant un carbone anomère activé, substitué par un groupe réactif compatible avec les autres groupements présents sur les motifs, toutes les autres positions de A et U, excepté celle dont le carbone anomère est activé et celle occupée par le groupe —OH de la fonction alcool, portant, soit des groupes amino, soit des groupes carboxyle, ou des précurseurs de ces groupes, soit encore des groupes —OH, ces groupes occupant des positions déterminées, les groupes amino et carboxyle lorsqu'ils sont présents, étant bloqués respectivement par des groupements protecteurs de fonction amino et carboxyle, les groupes —OH étant bloqués par au moins deux types de groupes protecteurs, éliminables séquentiellement en permettant l'introduction de groupes désirés en certaines positions, puis la libération de groupes —OH en d'autres positions,

— qu'on répète, éventuellement, l'opération de glycosylation afin d'allonger la chaîne oligosaccharidique,

— qu'on élimine séquentiellement les groupements protecteurs des groupes —OH en introduisant des groupements de substitution désirés dans des positions spécifiques, puis en libérant les groupements —OH d'autres positions spécifiques ainsi que les groupes amino et carboxyle.

Grâce aux dispositions ci-dessus, il est ainsi possible de réaliser une liaison covalente entre des motifs de structure A et U et ce, selon la stéréochimie que présente ce type d'enchaînement dans les molécules biologiquement actives déjà considérées.

Il est même possible à l'aide de l'invention de réaliser les enchaînements souhaités selon un ordre donné et/ou possédant une stéréospécificité donnée.

Les moyens proposés selon l'invention permettent ainsi d'établir notamment une liaison de type $1 \xrightarrow{\alpha} 3$ entre un motif acide L-iduronique et un motif galactosamine, une liaison de type $1 \xrightarrow{\beta} 4$ entre un motif D-galactosamine et un motif soit acide D-glucuronique, soit L-iduronique et une liaison de type $1 \xrightarrow{\beta} 3$ entre un motif acide D-glucuronique et un motif D-galactosamine.

Les intermédiaires mono ou oligosaccharides de cette synthèse sont des produits semi-ouverts ou ouverts. On appellera composé semi-ouvert à droite un composé activé ou potentiellement activable sur son carbone anomère, permettant ainsi son transfert sur l'extrémité non réductrice d'un monosaccharide ou d'un oligosaccharide. L'expression « composé semi-ouvert à gauche » désignera un monosaccharide ou un oligosaccharide possédant une seule fonction —OH libre ou potentiellement libre, permettant sa glycosylation spécifique. A titre illustratif, on indique ci-après la formule 1 d'un exemple de composé semi-ouvert à gauche et celle 2 d'un exemple de composé semi-ouvert à droite :

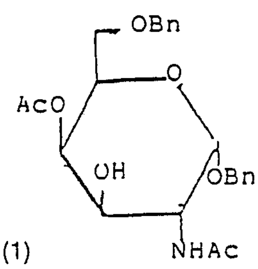
(1)

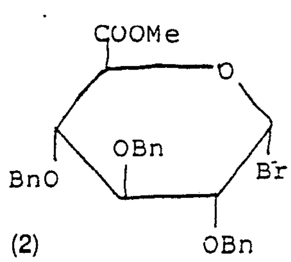
(2)

Ac représentant un groupe acétyle, Bn, un groupe benzyle et Me méthyle.

Il s'ensuit qu'on appellera dérivés ouverts un dérivé semi-ouvert à la fois à droite et à gauche selon la définition ci-dessus, de tels dérivés autorisant une élongation de chaîne dans les deux directions. Un dérivé de ce type répond par exemple à la formule 3 :

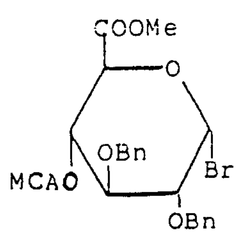
(3)

dans laquelle Bn et Me sont tels que définis ci-dessus et MCAO représente un groupe monochloroacétyle.

Quant aux dérivés fermés, il s'agit de produits dont les motifs ne peuvent pas donner lieu à élongation de chaînes en raison de la nature de leurs substituants.

Selon une disposition supplémentaire pour pouvoir ajouter des motifs à la séquence A-U ou U-A formée dans l'étape précédente, les motifs A ou U de la séquence formée doivent renfermer des groupements protecteurs temporaires, c'est-à-dire des groupements capables de bloquer sélectivement une position du motif A ou U destinée à intervenir dans une nouvelle réaction de glycosylation. Ces groupements sont éliminables en présence des autres groupements présents sur les motifs des produits de départ en recréant un alcool, ce qui permet en répétant l'étape précédente de glycosylation d'allonger le squelette glucidique.

L'invention donne donc accès à la synthèse d'oligosaccharides à enchaînements variés, qu'il s'agisse de stéréospécificité α ou β et/ou d'ordre d'enchaînement entre les motifs x, z et/ou y, l'élongation pouvant être effectuée à volonté.

Selon encore une autre disposition du procédé de l'invention, la chaîne glucidique élaborée est soumise à une ou plusieurs réactions chimiques afin d'introduire un type de groupements fonctionnels donnés ou, successivement, plusieurs types de groupements, puis de former, si on le désire, des dérivés de ces groupements fonctionnels.

Cette étape de fonctionnalisation peut être réalisée en n'éliminant que certains groupements protecteurs et/ou certains groupements précurseurs des dérivés aminés ou encore la totalité des groupements protecteurs et/ou des groupements précurseurs et en introduisant à leur place un type de substituants donné, ou, successivement, des substituants différents, puis en libérant une partie ou la totalité des groupes —OH encore bloqués, si on le désire.

Il est entendu alors que les différents groupes présents sur les motifs de la chaîne sont compatibles avec les substituants introduits à chaque étape.

La ou les réactions chimiques mises en œuvre au cours des étapes de fonctionnalisation sont réalisées de manière à ne pas altérer la structure de la chaîne et les groupes que l'on désire éventuellement maintenir et/ou ceux qui ont déjà été introduits.

Selon un mode préféré de réalisation de l'invention, pour obtenir des oligosaccharides à substitutions spécifiques comme définies ci-dessus, on utilise avec avantage des produits de départ renfermant plusieurs types de groupements protecteurs, à savoir (1) un ou plusieurs groupements semi-permanents et (2) un ou plusieurs groupements permanents.

Par groupements semi-permanents, on entend des groupements éliminables en premier lieu après les réactions de glycosylation lorsque le squelette glucidique comporte le nombre de motifs désirés, sans enlèvement ou altération des autres groupes présents, permettant alors l'introduction de groupements fonctionnels souhaités aux positions qu'ils occupent.

Les groupements permanents sont des groupements capables de maintenir la protection des radicaux —OH durant l'introduction de groupements fonctionnels à la place des groupements semi-permanents.

Ces groupements sont choisis parmi ceux compatibles avec les groupes fonctionnels introduits après élimination des groupes semi-permanents. Il s'agit, en outre, de groupements inertes vis-à-vis des réactions effectuées pour la mise en place de ces groupes fonctionnels et qui sont éliminables sans que ces groupements fonctionnels ne soient altérés.

D'une manière avantageuse, la mise en œuvre de ces dispositions permet d'élaborer une chaîne glucidique dans laquelle les motifs A et U sont sélectivement substitués.

Pour préparer plus particulièrement des oligosaccharides renfermant les motifs A et/ou U des molécules biologiquement actives évoquées ci-dessus, on a avantageusement recours à des groupements protecteurs tels que les radicaux acyle, alcoyle éventuellement substitués ou aryle.

Les motifs des produits mis en œuvre de type A comportent, en position 2, un groupe azoté autorisant le maintien de la présence d'une fonction azotée durant les opérations mises en œuvre dans le procédé. Ce groupe azoté est avantageusement constitué par des groupes tels que $—N_3$ ou N-phtalimido, ou tout autre groupe constituant un précurseur de fonction amine ou d'un dérivé d'amine, en particulier de —NH-acyle, plus spécialement de $—NH—COCH_3$ et éventuellement $—NHSO_3^-$.

Quant aux fonctions carboxyle des motifs U, elles sont bloquées par des groupes inertes vis-à-vis des réactions mises en jeu pour le remplacement des groupes protecteurs et éliminables en fin de synthèse pour libérer les groupes carboxyle, éventuellement aux fins de salification. Ces groupes protecteurs de fonction carboxyle sont choisis avantageusement parmi les radicaux alcoyle, ou les radicaux aryle.

Les fonctions carboxyle peuvent aussi être obtenues après glycosylation à l'aide d'un sucre neutre puis déblocage sélectif et oxydation de la fonction alcool primaire.

La structure du produit mis en œuvre dans la réaction de glycosylation est choisie en fonction des motifs du squelette glucidique recherché ainsi que des substitutions recherchées.

Pour former, par exemple, un disaccharide de type —U—A—, on utilise deux composés respectivement à structure acide uronique et sucre aminé, répondant, par ailleurs, aux définitions données ci-dessus.

En vue d'une élongation de chaîne, ces composés tels que mis en œuvre pour former le disaccharide en question, renferment, en outre, un groupement temporaire sur la position destinée à être impliquée

dans la nouvelle réaction de glycosylation. Pour une élongation du disaccharide U—A vers la gauche, ce groupement temporaire est présent sur le motif U et pour une élongation à droite sur le motif A.

Il est ainsi possible d'obtenir, notamment, des enchaînements $U_wA_xU_yA_z$ dans lesquels la somme des indices est comprise entre 2 et 12, ces valeurs étant incluses dans l'intervalle, w et y ne pouvant être nuls simultanément. Des enchaînements réguliers sont du type U $(AU)_n$, $(AU)_nA$, $(UA)_n$ ou encore $(AU)_n$ avec n compris entre 1 et 6.

Selon une variante de réalisation du procédé de l'invention, l'alternance de type A—U ou U—A rencontrée dans les structures des produits naturels peut être modifiée en utilisant, à la place de l'un ou plusieurs des motifs A ou U, un sucre constituant un analogue structural de motif A ou U, tel qu'un sucre neutre ou un désoxy-sucre, ou encore d'autres motifs acides uroniques ou sucres aminés U ou A de configurations différentes.

Selon un mode préféré de réalisation du procédé de l'invention, on fait réagir l'alcool ci-dessus avec un dérivé réactif tel qu'un halogénure, un imidate ou un orthoester. Ces condensations sont réalisées dans des conditions anhydres.

La réaction de condensation entre l'halogénure et l'alcool est avantageusement du type Koenigs-Knorr. L'halogénure est avantageusement constitué par un bromure ou un chlorure en raison de commodités d'obtention.

On opère en milieu solvant, plus spécialement dans un solvant organique, notamment du type dichlorométhane ou dichloroéthane.

Avantageusement, on utilise un catalyseur, en général un sel d'argent ou de mercure, par exemple, le trifluorométhane sulfonate d'argent, communément appelé triflate d'argent, le carbonate d'argent, l'oxyde d'argent, le bromure mercurique ou le cyanure mercurique. On utilise également un accepteur de protons tel que la sym-collidine de même qu'un capteur pour l'eau éventuellement présente et/ou pour l'acide halogénohydrique formé, par exemple des tamis moléculaires 0,4 nm.

L'étude des conditions de réaction montre qu'il est approprié d'opérer à température ambiante ou encore à une température inférieure pouvant atteindre 0 °C ou moins, sous atmosphère d'un gaz inerte tel que l'azote ou l'argon.

Ces conditions permettent de condenser les motifs de structure x et y ou z (ou l'inverse), selon la stéréochimie souhaitée. Elles permettent également l'établissement de liaisons covalentes avec des sucres neutres ou des désoxy-sucres.

Une variante comportant l'utilisation, comme catalyseur, de dérivés mercuriques, en particulier de cyanure et/ou de bromure mercurique, s'avère appropriée pour réaliser des liaisons covalentes entre des alcools de structures variées et un précurseur L-idose du motif de structure z (acide L-iduronique). Selon cette variante, on utilise également des tamis moléculaires 0,4 nm. Le solvant organique est choisi selon la réactivité de l'alcool. Ainsi, on utilise avantageusement un solvant du type du nitrobenzène lorsque la condensation requiert une température supérieure à 100 °C. Pour des températures inférieures, on utilise des solvants, tels que le benzène ou le dichlorométhane. Des mélanges de solvants conviennent également pour réaliser la réaction de condensation.

Avec les motifs de type U, il est possible d'utiliser, comme groupe réactif, un orthoester. On réalise alors de préférence la réaction à une température supérieure à 100 °C.

Le milieu solvant est du type du chlorobenzène ou tout autre solvant dont le point d'ébullition dépasse 100 °C et se trouve avantageusement entre 100 et 150 °C. Pour activer la réaction, on utilise un catalyseur tel que du perchlorate de 2,6-diméthyl pyridinium.

Ce mode de réalisation de l'étape de condensation s'avère d'un grand intérêt pour établir une liaison interglycosidique entre un motif de structure U (acide uronique et un motif de structure A (D-galactosamine).

L'utilisation du groupement orthoester présente, en particulier, un double avantage.

D'une part, elle permet de conférer au carbone anomère de U la réactivité nécessaire pour la réaction de glycosylation. D'autre part, l'ouverture de ce groupe assure la mise en place en position 2 de U d'un groupe protecteur, éliminable sélectivement, en permettant l'introduction, à sa place, d'un groupe de substitution spécifique.

Ainsi, par réaction d'un groupe 1,2-o-méthoxyéthylidène d'un motif U avec le radical —OH d'un motif x il est possible à la fois d'établir une liaison interglycosidique entre les deux produits utilisés et de disposer en position 2 de U d'un groupe —OAc (Ac représentant un groupe acétyle) qui pourra être éliminé sélectivement aux fins d'introduction d'un groupe fonctionnel donné, par exemple $—SO_3^-$. Cette disposition permet également de laisser toute liberté pour traiter la position 4 du motif U.

Lorsqu'on utilise comme groupe réactif un groupe imidoyle, il s'avère approprié d'opérer à basse température, plus spécialement à une température inférieure ou égale à 0 °C environ, en milieu solvant, tel que du dichlorométhane, en présence de tamis moléculaire 0,4 nm et d'un catalyseur tel que de l'éthérate de trifluorure de bore.

Dans l'alcool de départ, le groupe —OH libre occupe la position que l'on souhaite engager dans la liaison de glycosylation.

En choisissant l'alcool de manière appropriée, il est ainsi possible d'établir des liaisons du type 1-2, 1-3, 1-4 ou 1-6.

A partir de la séquence formée à l'issue de la réaction de condensation, on élabore une chaîne comportant le nombre de motifs désirés en répétant l'étape de glycosylation.

La fonction alcool de l'un des motifs A ou U impliqué dans la séquence glucidique déjà constituée est alors avantageusement libérée de son groupement protecteur temporaire. Le choix de ce groupement sera aisément déterminé par l'homme de l'art selon la nature des autres groupements présents sur la chaîne glucidique.

Parmi les divers groupements pouvant être utilisés, on citera le groupe allyle qui, par traitement, par exemple d'abord avec un agent isomérisant tel que des dérivés de Pd, Rh et Ir, en particulier le chlorure de tristriphénylphosphine rhodium (I), ou encore le tertio-butoxyde de potassium, puis dans des conditions acides, en particulier avec un mélange d'oxyde mercurique et de chlorure mercurique, permet aisément de recréer un alcool à la position qu'il occupe.

De même, il est possible d'obtenir un groupe —OH par saponification à partir d'un groupe —O-acyle, en particulier —O-acétyle, ou O-chloroacétyle ou O-lévulinoyle.

Ces radicaux peuvent être éliminés pour libérer une fonction —OH, par exemple, à l'aide de thiourée en milieu solvant, avantageusement à une température supérieure à 80 °C, de préférence de l'ordre de 100 °C.

Les dispositions qui précèdent permettent d'obtenir une chaîne glucidique à motifs alternés A—U ou U—A.

Cette alternance régulière peut être modifiée en mettant en œuvre les produits appropriés dans la réaction de glycosylation. Il est ainsi possible d'élaborer une structure irrégulière avec incorporation de motifs autres que U ou A, en particulier des sucres neutres ou encore des désoxy-sucres. Un autre type de structure irrégulière peut être obtenu en ajoutant plusieurs motifs A ou plusieurs motifs U consécutifs entre deux motifs de structure A—U ou U—A.

Il est entendu que les différentes dispositions de l'invention concernant les motifs A et U s'appliquent également aux autres motifs que peut comporter la chaîne glucidique tels que les sucres neutres ou les désoxy-sucres.

Comme déjà indiqué, les différents groupements présents sur les motifs A et U sont choisis de manière à conférer à ces derniers une réactivité suffisante pour réaliser la liaison glycosidique en question.

Les groupements protecteurs de radicaux —OH, mis à part les groupements temporaires déjà considérés, sont généralement choisis dans le groupe comprenant les radicaux acyle (notamment acétyle, alcoyle, alcoyle substitué tel que benzyle), et pour deux positions voisines, parmi les groupes acétals ou cétals, par exemple benzylidène. Une autre forme de protection consiste à effectuer un blocage de deux groupes —OH sous forme époxyde ou de pont 1,6-anhydro.

Avantageusement, les produits utilisés dans la réaction de glycosylation renferment plusieurs types de groupements protecteurs, ce qui permet au cours de l'étape de fonctionnalisation d'introduire successivement un ou plusieurs groupements fonctionnels et de libérer un ou plusieurs radicaux —OH si on le désire.

D'une manière générale, les groupements protecteurs peuvent déjà occuper des positions déterminées sur les produits mis en œuvre dans la réaction de glycosylation.

Ils peuvent également être introduits à partir d'autres groupements une fois le squelette glucidique constitué. Cette variante comporte, par exemple, l'utilisation pour la glycosylation d'un produit A dans lequel les groupes —OH en positions 2 et 3 et en positions 1 et 6 sont bloqués sous forme anhydro, respectivement 2,3-époxyde et 1,6-anhydro. Grâce à ce blocage, on dispose durant l'élaboration du squelette glucidique d'un élément constituant potentiellement un motif A mais n'interférant pas avec les réactions mises en jeu dans la synthèse. Cette disposition présente l'avantage de laisser une large liberté pour effectuer les réactions désirées sur les groupements des autres motifs.

On remarquera, en outre, dans le cas considéré, que l'ouverture de la fonction époxyde par de l'acide de sodium permet d'introduire, en position 2, un groupe $N_3$ qui constitue donc un précurseur de fonction amine.

D'une manière préférée, pour disposer d'une chaîne glucidique permettant d'introduire sélectivement un ou plusieurs types de substituants au cours de l'étape de fonctionnalisation, en particulier les substitutions spécifiques ci-dessus, on met en œuvre des produits comportant plusieurs types de groupements protecteurs, à savoir les groupes semi-permanents et les groupes permanents définis plus haut.

Comme déjà indiqué, les substitutions des produits naturels considérés, mises à part celles des positions 2 des motifs A, sont essentiellement constituées par des groupes sulfate.

Les travaux des inventeurs pour mettre au point des conditions de sulfatation appropriées ont montré qu'il est possible et même avantageux d'effectuer une réaction de sulfatation en présence de groupements benzyle. Contrairement aux opinions admises dans ce domaine, l'élimination de groupements permanents benzyle, en présence de groupements —O-sulfate, peut être réalisée.

D'une manière préférée, les radicaux —OH des produits de départ destinés à être sulfatés sont alors protégés par des groupes acyle, en particulier acétyle, tandis que les radicaux —OH destinés à être libérés en fin de synthèse sont protégés par un groupe permanent tel que le groupe benzyle.

Selon une grande souplesse du procédé de l'invention, il est possible de soumettre l'ensemble de la

chaîne glucidique élaborée à une réaction chimique donnée afin d'introduire un type de substituant déterminé.

Ce traitement peut consister par exemple en une estérification, notamment une sulfatation à l'aide d'un agent approprié, réalisée dans des conditions n'altérant pas la structure osidique. Cette sulfatation peut être réalisée de manière spécifique ou non, le cas échéant sur le glycoside totalement déprotégé.

Selon un mode préféré de réalisation de l'invention, l'étape de fonctionnalisation est cependant réalisée sélectivement de manière à introduire sur la chaîne, successivement, plusieurs types de substituants puis de libérer certains radicaux —OH.

Selon des conditions particulièrement avantageuses, permettant d'introduire des groupes sulfate sur des positions déterminées des motifs, de libérer des radicaux —OH sur d'autres positions, de former en position 2 des motifs A un dérivé d'amine et en position 6 des motifs U des dérivés d'acide, on met en œuvre des motifs répondant aux caractéristiques suivantes.

Les groupes semi-permanents de ces motifs occupent des positions destinées à être sulfatées et sont constitués par des groupes —O-acétyle.

Quant aux positions correspondant à un groupe —OH destinées à être libérées, elles sont occupées par des groupes semi-permanents constitués par des groupes benzyle ou permanents.

Les positions 2 des motifs A sont substituées par des groupes tels que $N_3$ ou NH-phtalimidoyl ou —NH-acétyle et les positions 6 des motifs U sont occupées par des groupes carboxyles protégés par un radical alcoyle, en particulier méthyle.

Ce jeu de conditions permet de réaliser l'étape de fonctionnalisation, par exemple comme suit :

On introduit tout d'abord sélectivement les groupes sulfate après avoir éliminé les groupes —O-acétyle de blocage. Cette réaction est réalisée de manière à ne pas affecter les groupes benzyle et les groupes azotés et carboxyle présents.

A cet égard, on effectue avantageusement une réaction de saponification à l'aide d'une base forte telle que la soude.

Cette réaction est réalisée de préférence à une température inférieure à l'ambiante et plus spécialement voisine de 0 °C.

On soumet le produit résultant de l'hydrolyse à l'action d'un agent d'alcoylation afin d'introduire, sur le groupe carboxyle, les groupes alcoyle protecteurs qui se sont trouvés éliminés lors de l'hydrolyse.

Par réaction avec un agent de sulfatation, on obtient alors l'introduction de groupes sulfate aux positions libérées par l'hydrolyse et laissées libres après l'action de l'agent d'alcoylation.

Des conditions de réaction satisfaisantes pour la conduite de la sulfatation comprennent la mise en œuvre d'un agent de sulfatation, tel qu'un complexe triméthylamine/$SO_3^-$. Cette réaction est avantageusement réalisée en milieu solvant, plus spécialement dans un solvant tel que le diméthylformamide. De préférence, on opère à une température supérieure à l'ambiante, généralement voisine de 50 °C, ce qui correspond à une durée de réaction d'environ 12 heures.

Après l'introduction des groupes sulfate sur les fonctions alcool, on procède à la libération des groupes —OH bloqués par les radicaux benzyle.

L'élimination de groupes benzyle est avantageusement réalisée par hydrogénation catalytique dans des conditions compatibles avec le maintien des groupes sulfate et la transformation des groupes azotés en groupes fonctionnels amine.

On opère de préférence sous pression d'hydrogène en présence d'un catalyseur du type Pd/C.

Cette réaction est avantageusement réalisée en milieu solvant organique, en particulier alcoolique, additionné d'eau.

Pour obtenir l'hydrogénation des groupes azotés précurseurs et l'élimination des radicaux protecteurs des groupes —OH, la réaction est avantageusement réalisée sur une durée d'environ 3 à 4 jours.

Comme déjà indiqué, les groupes fonctionnels amine se présentent sous forme de dérivés de type N-acétyle dans les molécules biologiquement actives considérées.

Pour former des groupes N-acétyle, on soumet le produit résultant de la réaction d'hydrogénation à l'action d'un agent d'acétylation. A cet égard, l'anhydride acétique constitue un agent particulièrement approprié.

Pour réaliser cette réaction d'acétylation sélective sans affecter les autres substituants présents sur les motifs, il convient, notamment, d'opérer à pH basique, en particulier voisin de 8 en milieu aqueux.

On peut également souhaiter former des groupes N-sulfate, ce qui peut être réalisé à l'aide d'un agent de sulfatation du type indiqué ci-dessus. Des pH supérieurs à 9, avantageusement de l'ordre de 9-10, sont utilisés pour la sulfatation.

Après la réaction de sulfatation ou d'acétylation, l'addition d'une base forte permet de libérer les groupes carboxyle.

Les produits formés peuvent être aisément salifiés à l'aide de résines échangeuses d'un cation approprié. Dans les produits naturels, le cation en particulier est constitué par du sodium. On utilise donc avantageusement des résines échangeuses de cations sodium.

On peut également former des sels de potassium, lithium, magnésium, calcium. On utilise alors une résine échangeuse de protons, puis on neutralise l'acide formé avec la base du cation.

L'invention vise également les oligosaccharides constituant des intermédiaires dans les différentes étapes du procédé de synthèse défini ci-dessus.

Dans une famille, ces oligosaccharides renferment au moins un motif binaire A-U et U-A complètement protégé et possédant soit un groupe réactif sur le carbone anomère du motif à l'extrémité réductrice, soit un seul groupe —OH libre sur le motif à l'extrémité non réductrice, ce groupe —OH occupant la position 3, 4 ou 6 dans le cas d'un motif A et la position 2, 3 ou 4 dans le cas d'un motif U.

Dans une autre famille, les oligosaccharides sont constitués par des motifs complètement protégés tels qu'obtenus à l'issue de l'étape de glycosylation. Une autre famille encore comprend les produits dans lesquels un ou plusieurs groupes —OH sont libérés.

Ces différents oligosaccharides comportent une chaîne à base de motifs binaires de structure $(A\text{-}U)_n$ ou $(U\text{-}A)_n$ dans laquelle n est un nombre de 1 à 6.

Ces oligosaccharides correspondent à un enchaînement de type x-y ou encore x-z.

Dans un groupe d'oligosaccharides intermédiaires de l'invention, la chaîne glycosidique est constituée par un seul type de ces enchaînements binaires.

Dans un autre groupe, plusieurs de ces types sont présents.

Des oligosaccharides correspondants comportent dans leurs chaînes des motifs x-y et x-z.

Il est entendu que l'ordre des enchaînements considéré ci-dessus dans l'un ou plusieurs des motifs binaires peut être inversé conformément à l'invention.

Selon une variante, les oligosaccharides intermédiaires définis ci-dessus renferment un ou plusieurs motifs consécutifs x ou encore y ou z.

Selon une autre variante, les oligosaccharides intermédiaires renferment un ou plusieurs motifs de sucres neutres et/ou plusieurs désoxy-sucres dans leur structure. Les différents groupements protecteurs de ces sucres répondent aux définitions données ci-dessus pour les motifs A et U.

Dans ces oligosaccharides, les motifs constitutifs sont reliés entre eux par des liaisons de type 1-2, 1-3, 1-4 ou 1-6 selon la nature de l'alcool mis en œuvre dans l'étape de glycosylation.

Les oligosaccharides possédant la structure des fragments de chondroïtines, de chondroïtines-sulfates ou dermatane-sulfate renferment des liaisons de type 1 $\xrightarrow{\beta}$ 3, et comportent respectivement des motifs y 1 $\xrightarrow{\beta}$ 3x, z 1 $\xrightarrow{\alpha}$ 3x, x 1 $\xrightarrow{\beta}$ 4z et x 1 $\xrightarrow{\beta}$ 4y.

Un groupe d'oligosaccharides préférés renferme au moins un motif binaire possédant une structure de type x 1 $\xrightarrow{\beta}$ 4y c'est-à-dire [D-galactosamine] 1 $\xrightarrow{\beta}$ 4 [acide D-glucuronique] répondant à la formule I :

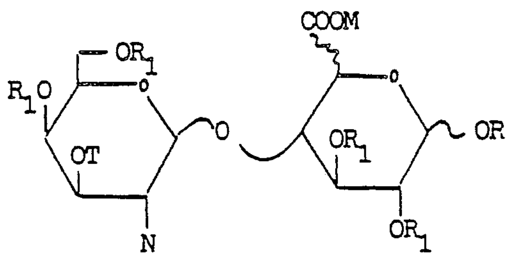

(I)

dans laquelle :

— les radicaux $R_1$, identiques ou différents les uns des autres, éventuellement conjointement avec R, représentent un groupe protecteur, en particulier un groupement sp semi-permanent ou un groupement p permanent,

— T, un groupement temporaire t, ou un groupement permanent p, ou un atome d'hydrogène,

— N, un groupe azoté précurseur d'amine ou de dérivé d'amine,

— R, un radical aliphatique ou aromatique, notamment un radical alcoyle comportant de 1 à 4 atomes de carbone, ou encore R un radical alcoyle substitué notamment benzyle, ou OR représente un groupe réactif tel qu'un halogénure et

— M, un groupement bloquant la fonction acide, ces différents symboles présentant les significations données ci-dessus.

Dans un sous-groupe, tous les radicaux R, $R_1$ et T sont identiques et représentent un groupement p ou sp.

Dans un autre sous-groupe, les radicaux $R_1$ sont différents les uns des autres, l'un au moins représentant un groupement de type sp, éventuellement conjointement avec R, le ou les autres radicaux $R_1$ représentant un groupe p.

On notera que les significations générales des symboles de la formule I s'appliquent également aux formules des différents groupes considérés ci-après. De même, on retrouve dans chacun de ces groupes, notamment, les deux sous-groupes évoqués ci-dessus.

Des oligosaccharides préférés comprennent des motifs aux formules II à V suivantes :

(Voir formules page 11)

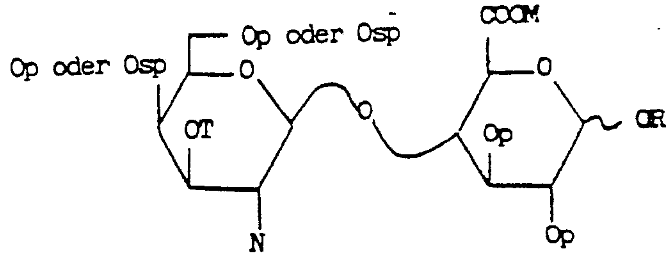

(II)

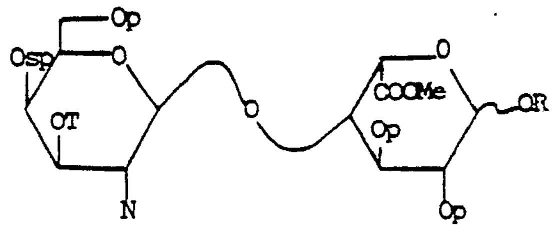

(III)

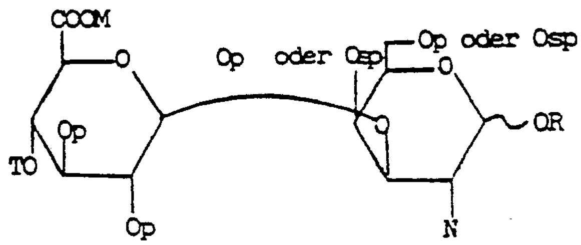

(IV)

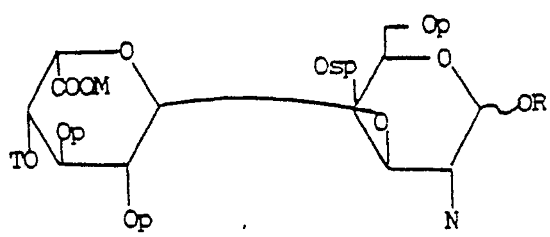

(V)

On notera que les oligosaccharides (II) et (IV) permettent d'effectuer des réactions d'estérification, en particulier de sulfatation, en positions 4 ou 6 respectivement du motif galactosamine et acide glucuronique tout en pouvant rallonger la chaîne.

Ceux de formules (III) et (V) permettent d'effectuer les réactions en question respectivement en position 4 de la galactosamine ou de l'acide iduronique et ce, également, tout en pouvant rallonger la chaîne.

De préférence, dans les formules (II) à (V), les symboles donnés présentent indépendamment, ou en combinaison, les significations suivantes :

— M représente un atome d'hydrogène ou un radical alcoyle, en particulier méthyle,

— sp un groupe acyle, en particulier acétyle,

— p, un groupe alcoyle substitué, en particulier benzyle,

— R, un groupe acyle en α ou β, en particulier un groupe acétyle, un radical alcoyle, en particulier méthyle ou alcoyle substitué, notamment benzyle, ou —OR un halogène, en particulier un bromure, ou encore un radical imidoyle,

— N, un groupe azido ou phtalimido,

— T, le groupe t représentant un radical acyle, en particulier acétyle, un radical acyle halogéné, en particulier, un radical monochloro ou trichloroacétyle, ou le groupe p représentant un radical alcoyle substitué en particulier le radical benzyle, le cas échéant lui-même paraméthoxy ou encore un atome d'hydrogène.

Une autre famille d'oligosaccharides intermédiaires préférée entrant dans le cadre de l'invention correspond aux produits dont les groupes protecteurs ont été éliminés partiellement en cours de synthèse. En particulier, de tels produits comportent un groupe —OH à la place des groupes sp.

Un groupe préféré de trisaccharides intermédiaires présente une structure répondant à l'une des formules VI et IX

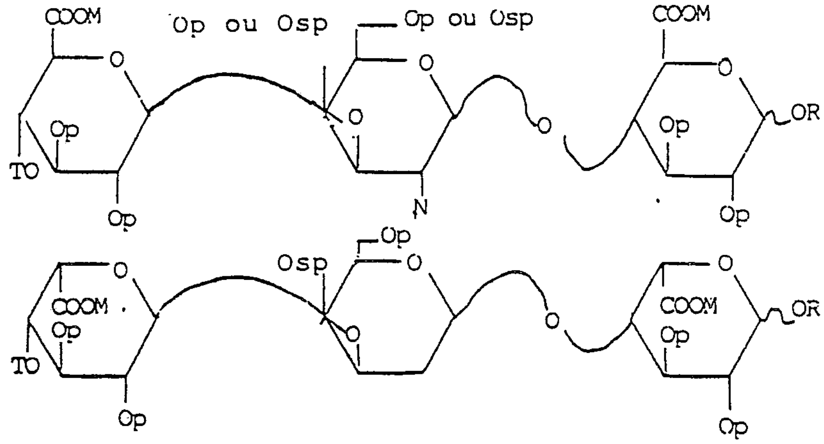

(VI)

(VII)

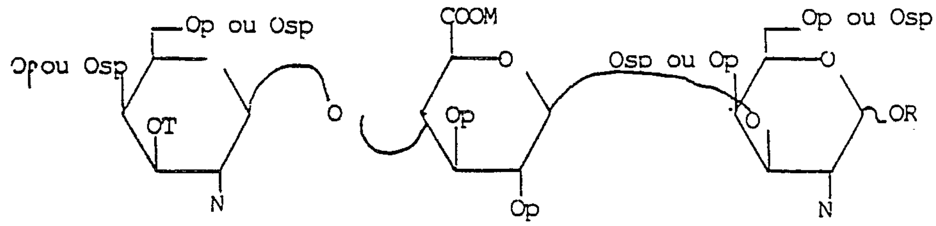

(VIII)

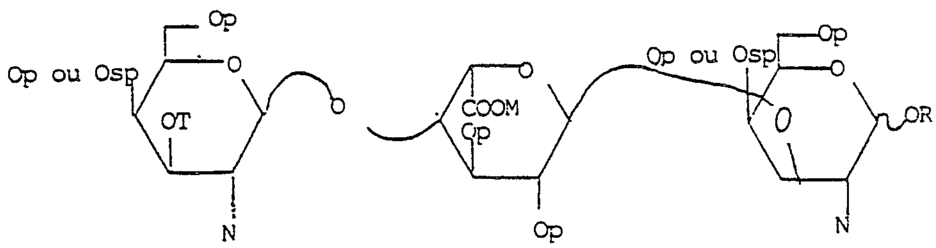

(IX)

dans laquelle les différents symboles présentent les significations données ci-dessus.

D'autres oligosaccharides intermédiaires préférés sont constitués par des tétrasaccharides. Un tétrasaccharide plus spécialement avantageux possède la structure (X) :

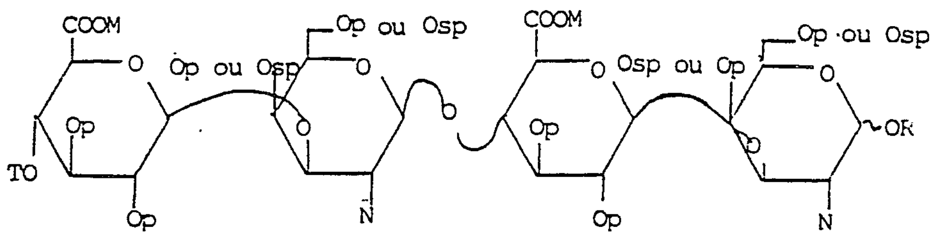

(X)

Comme évoqué ci-dessus pour les motifs binaires, l'invention concerne également les oligosaccharides ci-dessus dans lesquels un, plusieurs ou le cas échéant, la totalité des groupes —OH se trouvent libérés en cours de synthèse.

L'invention vise, en outre, en tant que produits nouveaux, les oligosaccharides répondant respectivement aux différentes définitions données ci-dessus, mais renfermant un ou plusieurs groupements fonctionnels.

Ces groupements fonctionnels sont constitués, de préférence, par des esters, et se présentent plus spécialement sous forme d'anions minéraux.

Des esters particulièrement préférés, en raison de leur présence dans les molécules biologiquement actives de type chondroïtines et chondroïtines-sulfates et dermatane-sulfate sont constitués par des esters sulfates.

D'autres esters avantageux correspondent à des esters phosphates.

Ces groupements fonctionnels sont portés par une ou plusieurs fonctions alcool primaire et/ou alcool secondaire et/ou amine primaire.

Une famille préférée d'oligosaccharides de l'invention renferme ainsi des motifs x comportant un anion tel que défini ci-dessus en position 6 et/ou 4.

Des oligosaccharides de cette famille renferment en position 2 de ces motifs x un groupe fonctionnel amine primaire avantageusement substitué par un sulfate ou par un autre groupe substituant.

Dans les oligosaccharides de l'invention renfermant au moins deux motifs x, les groupements

fonctionnels amine en position 2 peuvent être substitués par un même groupement ou par des groupements différents.

Un groupe préféré d'oligosaccharides de la famille considérée renferme des motifs x comportant des groupes sulfate sur les fonctions alcool secondaire et/ou alcool primaire.

Des oligosaccharides préférés de ce groupe, comportent en position 2 de ces motifs un groupe NH-acyle, en particulier NH-acétyle. D'autres oligosaccharides comportent un groupe $NH—SO_3^-$.

D'une manière préférée, les esters ci-dessous se présentent sous forme de sels avec un cation minéral ou organique, en particulier un cation métallique, notamment un cation alcalin, ou encore un cation dérivé d'une base organique azotée, par exemple du triéthylammonium.

Les cations utilisés sont constitués par du sodium. D'autres cations sont appropriés tels que les cations potassium, magnésium ou calcium.

Dans une autre famille préférée d'oligosaccharides de l'invention, les groupes carboxyle des motifs y ou z sont libres ou se présentent de préférence sous forme de sels avec un cation organique ou minéral tel que défini ci-dessus. Ils peuvent également être protégés comme rapporté plus haut.

D'autres produits préférés présentent des sulfates sur le motif y.

Dans ces différentes familles d'oligosaccharides, les fonctions hydroxyle des cycles pyraniques et plus particulièrement l'hydroxyle anomère (pour des raisons de stabilité) sont soit libres, soit protégés par des groupements permanents de type alcoyle, en particulier par des groupes méthyle.

Des produits préférés de ces différentes familles renferment, en combinaison, des motifs A et U répondant aux caractéristiques ci-dessus.

L'étude pharmacologique d'oligosaccharides de ce type de structure a montré chez certains de ces composés des activités biologiques leur permettant de contrôler de manière spécifique certaines étapes de la coagulation sanguine (voir Griffith M.J. and Marber G.A., Biochem. Biophys. Res. commun ; 112 (1983) 663-670).

L'invention concerne donc également leur application à la constitution de réactifs biologiques, utilisables en laboratoire, notamment comme éléments de comparaison pour l'étude d'autres substances dont on souhaite tester l'activité sur la coagulation.

Les travaux de la demanderesse ont montré que ce type de produit est capable d'exercer une activité antithrombotique puissante. (E.G. Vairel et al. Ann. Pharm. Franc. en impression.)

En outre, des dérivés selon l'invention présentent un grand intérêt pour lutter contre les troubles de la paroi vasculaire (athéroscléroses et artérioscléroses) et le vieillissement des tissus. Ils ont aussi une influence sur l'adhésion cellulaire. De plus, ils présentent l'avantage de ne pas avoir d'effet d'activation de thrombocytopénie. Ils présentent également l'avantage d'être pratiquement dénués d'effet sur le temps de saignement, ce qui élimine les risques d'hémorragie. Ces deux propriétés sont extrêmement importantes pour les applications médicales.

Les oligosaccharides de l'invention sont, en outre, avantageusement dépourvus de toxicité.

Ces produits sont donc particulièrement précieux pour l'élaboration de médicaments utilisables, notamment, pour le traitement de désordre de la coagulation, du vieillissement des tissus, et de troubles de la prolifération cellulaire.

L'invention est donc relative également à des préparations pharmaceutiques qui renferment lesdits oligosaccharides.

Elle est plus particulièrement relative à des préparations pharmaceutiques dépourvues de substances pyrogènes, contenant une quantité efficace de principes actifs en association avec des excipients pharmaceutiques.

Elle concerne également les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes, des pilules, ou encore présentées sous forme de liposomes ou de solutions buvables. Ces préparations renferment avantageusement de 50 mg à 5 g par unité de poids, de préférence 100 à 250 mg pour des gélules, tablettes et pilules et de 1 à 5 g pour les solutés buvables.

D'autres compositions pharmaceutiques comprennent ces oligosaccharides en association avec des excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables pour l'administration tant par voie intraveineuse qu'intramusculaire ou sous-cutanée.

Ces solutions renferment avantageusement 50 à 250 mg d'oligosaccharides, de préférence de 100 à 150, par exemple de 150 mg/ml, lorsque ces solutions sont destinées à l'injection par voie sous-cutanée. Elles peuvent contenir, par exemple, de 25 à 250, notamment 150 mg/ml d'oligosaccharides lorsqu'elles sont destinées à l'injection par voie intraveineuse ou par perfusion.

Avantageusement, de telles préparations pharmaceutiques sont présentées sous la forme de seringues non récupérables, prêtes à l'emploi.

D'autres préparations se présentent sous forme de solutés buvables renfermant avantageusement de 500 mg à 5 g de produit actif.

L'invention concerne également les compositions pharmaceutiques contenant lesdits oligosaccharides en association avec un autre principe actif, utilisable en particulier pour la prophylaxie et le traitement de thrombose, tel qu'un agent veinotonique comme la dihydroergotamine, un sel d'acide nicotinique ou un agent thrombolytique comme l'urokinase.

Les compositions pharmaceutiques de l'invention sont particulièrement adaptées pour le contrôle (préventif ou curatif) de certaines étapes de la coagulation du sang (chez l'homme ou l'animal), notamment dans le cas où le patient est soumis à des risques d'hypercoagulabilité résultant notamment d'opérations chirurgicales, de processus athéromateux, de développement de tumeurs et de troubles de la coagulation par des activateurs bactériens ou enzymatiques, etc.

Les compositions de l'invention sont plus spécialement appropriées pour lutter contre le vieillissement des tissus ou des manifestations de type dégénératif telles que les alopécies. Elles sont utilisées également pour le traitement des troubles de l'athérosclérose et de la prolifération cellulaire.

Afin d'illustrer l'invention, on indique ci-après, un exemple de posologie utilisable chez l'homme : cette posologie comprend par exemple, l'administration au patient de 50 mg à 150 g par voie sous-cutanée, une à trois fois par jour, selon le niveau des risques d'hypercoagulabilité ou la condition thrombotique du patient, ou de 150 mg/24 heures, par voie intraveineuse, en administrations discontinues à intervalles réguliers, ou continues par perfusion, ou encore de 150 mg (trois fois par semaine) par voie intramusculaire ou sous-cutanée (ces titres sont exprimés en unités Yin-Wessler). Ces doses peuvent être naturellement ajustées pour chaque patient en fonction des résultats et des analyses de sang effectuées auparavant, la nature des affections dont il souffre et, d'une manière générale, son état de santé.

Outre les compositions pharmaceutiques renfermant les oligosaccharides tels quels, l'invention vise également les compositions pharmaceutiques renfermant au moins un oligosaccharide tel que défini ci-dessus, conjugué, par liaison covalente, à un support soluble ou à un support insoluble, avantageusement au moyen du sucre terminal réducteur.

D'autres conjugués préférés avec supports solubles sont formés d'un oligosaccharide fixé à un véhicule tel qu'une protéine, notamment la polylysine, ou le sérum albumine bovine.

Ces produits sont utilisables comme immunogènes eux-mêmes sources d'anticorps circulants produits in vivo ou d'anticorps monoclonaux, clonés in vitro selon les techniques appropriées.

Dans d'autres conjugués préférés, les oligosaccharides de l'invention sont conjugués à des supports insolubles. On utilisera avantageusement les supports classiques.

Ces conjugués sont utilisables comme immunoabsorbants, par exemple pour son dosage ou pour l'élaboration, par fixation sur des polymères biocompatibles, de nouveaux polymères hémocompatibles athrombotiques.

L'invention vise également l'application des oligosaccharides considérés en médecine nucléaire, en tant que produits radiopharmaceutiques. Ces produits sont alors marqués par des traceurs choisis parmi ceux couramment utilisés dans ce domaine, et notamment à l'aide de technétium 99 m.

A cet effet, on transforme le technétium 99 m obtenu à partir de générateurs du commerce, sous forme de pertechnétate de sodium de valence 7 non réactif, en technétium réduit de valence 4 qui serait la forme la plus réactive du technétium. Cette transformation est effectuée grâce à un système réducteur réalisé à partir de sels d'étain (chlorure stanneux), de sels de fer (sulfate ferreux), de sels de titane (trichlorure de titane) ou autres sels.

La plupart du temps, cette simple réduction du technétium suffit, dans des conditions de pH données, à réaliser la fixation du technétium sur la molécule considérée.

Pour l'élaboration de ces réactifs radiopharmaceutiques, on peut opérer conformément à la méthode de P.V. KULKARNI et al. dans The Journal of Nuclear Medecine 21, n° 2 p. 117-121.

Les produits ainsi marqués sont avantageusement utilisés dans des tests in vivo pour la détection et le diagnostic d'extension des thromboses et des états thrombotiques.

Les oligosaccharides de l'invention peuvent être également utilisés pour la détermination de la spécificité des nombreuses enzymes impliquées dans le métabolisme des glycosaminoglycanes.

Grâce à leur structure, les produits de l'invention constituent en outre des intermédiaires de synthèse de grand intérêt permettant d'obtenir des fragments donnés, ou des dérivés de fragments, de molécules biologiquement actives. Ils constituent, notamment, des composés de référence pour des études de structure.

D'autres caractéristiques avantageuses de l'invention apparaîtront dans les exemples qui suivent et en se reportant aux figures 1 à 12 illustrant les produits mis en œuvre dans les synthèses décrites.

Dans ces figures, les références numériques des formules sont utilisées également dans les exemples pour désigner les mêmes produits.

Les abréviations utilisées dans ces formules présentent les significations suivantes :
Ac : un groupe acétyle ; Me : méthyle ; Bn : benzyle ;
Bz : benzoyle ; MCAO : monochloroacétyle ; Tr : trityle ;
but. : butyle ; S un groupe $SO_3^-$ ; Pht : phtalimidoyle ; et
L : lévulinoyle.

(Voir formules page 15)

Exemple 1

Synthèse des disaccharides 11 et 12 de formule (voir figures 1 et 2)

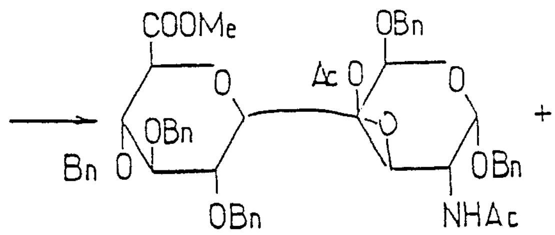

(11)

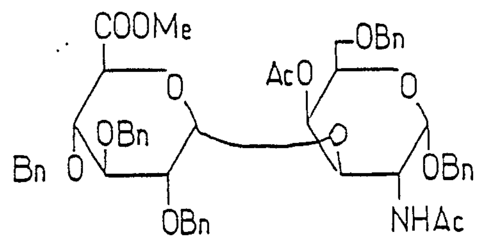

(12)

1) Préparation du benzyl-2-acétamido—2-désoxy-α-D-galactopyranoside 2

Une suspension de N-acétyl-D-galactosamine 1 (3 g) dans de l'alcool benzylique anhydre (40 ml) contenant 2 % d'acide chlorhydrique (gaz, séché) est agitée à 70 °C à l'abri de l'humidité pendant 16 heures. Après refroidissement, la solution limpide est versée lentement dans de l'éther froid (400 ml). Le précipité est alors refroidi 2 heures à — 20 °C, puis essoré. Les solides sont rincés avec de l'éther, puis dissout dans un mélange méthanol-eau (4 : 1, v/v, 100 ml) et portés à ébullition pendant 1/2 heure en présence de charbon actif (1 g). La solution chaude est filtrée, puis évaporée à sec. Le résidu est soumis à une cristallisation fractionnée dans le 2-propanol pour donner le composé 2 (2,54 g, 60 %) ; PF = 205-206° ; $[\alpha]_D$ + 210° (c7, $H_2O$).

(Lit. : H.M. FLOWERS and D. SHAPIRO, J. Org. Chem., 30 (1965) 2041-43, PF = 203-205°, $[\alpha]_D$ + 204° (c0.98, $H_2O$)).

2) Acétalation du composé 2

a) Acétalation par l'acétone en milieu acide.

Une suspension du composé 2 (311 mg, 1 mM) dans de l'acétone anhydre (20 ml) est agitée à l'abri de l'humidité en présence d'acide para-toluènesulfonique (monohydrate, 40 mg). Le mélange devient homogène après 1 heure, et est agité 3 heures 30 au total. De la triéthylamine (0,5 ml) est ajoutée et le mélange réactionnel est évaporé à sec. Le résidu est repris avec du chloroforme (50 ml), la phase organique est lavée avec une solution aqueuse à 5 % d'hydrogénocarbonate de sodium, avec de l'eau, séchée (sulfate de sodium), filtrée et évaporée. Le résidu est chromatographié sur une colonne de gel de silice (20 g). L'élution par l'acétate d'éthyle donne :

— le composé 3, sirop (222 mg, 63 %), $[\alpha]_D$ + 193° (c1, méthanol), R.M.N. (90 $MH_z$, $CDCl_3$) : δ : 7,30 (s, 5H, Ph) ; 5,95 (d, 1H, NH, J = 8,5 $H_z$), 4,92 (d, 1H, $H_1$, $J_{1,2}$ = 3,5 $H_z$), 2,80 (1H, OH, échangé avec $D_2O$), 1,97 (s, 3H, NAc), 1,55 et 1,32 (2s, 2 × 3H, Isopropyl) ;

— le composé 4, sirop (110 mg, 31 %) ; $[\alpha]_D$ + 154° (c1, chloroforme), R.M.N. (90 $MH_z$, $CDCl_3$) : δ : 7,32 (s, 5H, Ph), 5,80 (d, 1H, NH, J = 8,5 $H_z$), 5,0 (d, 1H, $H_1$, $J_{1,2}$ = 3,5 $H_z$) ; 2,75 (1H, OH, échangé avec $D_2O$) ; 1,96 (s, 3H, NAc) ; 1,46 (s, 6H, Isopropyl).

b) Acétalation par le 2-méthoxypropène (contrôle cinétique).

Ce composé 2 (311 mg, 1 mM) est dissous dans du N,N-diméthylformamide anhydre (8 ml). Du 2-méthoxypropène (0,3 ml) et de l'acide para-toluènesulfonique (monohydrate, 3 mg) sont ajoutés successivement, et le mélange réactionnel est agité à l'abri de l'humidité pendant 3 heures.

Un traitement identique à celui décrit au paragraphe a, suivi d'une chromatographie sur colonne de gel de silice (20 g), donne, par élution avec le mélange dichlorométhane-méthanol (15 : 1, v/v, contenant 0,1 % de triéthylamine).

— Le composé 3, sirop (34 mg, 10 %)

— Le composé 4, sirop (299 mg, 85 %)

— Benzoylation du composé 4.

Une solution du composé 4 (90 mg, 0,25 mM) dans un mélange de dichlorométhane anhydre (5 ml) et de pyridine anhydre (1 ml) est traité à 0 °C à l'abri de l'humidité par du chlorure de benzoyle (60 μl, 0,5 mM) pendant 4 heures. Du méthanol (1 ml) est alors ajouté, et après 15 minutes, le mélange réactionnel est dilué avec du dichlorométhane (20 ml). La phase organique est lavée avec une solution aqueuse à 10 % d'hydrogénosulfate de sodium, avec de l'eau, avec une solution aqueuse à 5 % d'hydrogénocarbonate de sodium, avec de l'eau, séchée (sulfate de sodium), filtrée et évaporée. Le résidu gélatineux est cristallisé dans un mélange acétate d'éthyle-éther-hexane pour donner le composé 5 (105 mg, 90 %), PF = 185-186 °C ; $[\alpha]_D$ + 189° (c1, chloroforme), R.M.N. (90 $MH_z$, $CDCl_3$) : δ : 8,05 (m, 2H, H ortho de benzoyle) ; 7,40 (m, 8H, 1 Ph + H méta, para de benzoyle), 5,73 (d, 1H, NH, J = 9 $H_z$), 5,33 (d. de d., 1H, $H_3$, $J_{2,3}$ = 10 $H_z$, $J_{3,4}$ = 3,5 $H_z$), 1,83 (s, 3H, NAc), 1,48 et 1,39 (2s, 2 × 3H, isopropyl).

N.B. : La présence à δ = 5,33 ppm d'un doublet de doublet ayant des constantes de couplage de 10 et 35 $H_z$ montre de manière non ambiguë la présence d'un groupement électro-attracteur (benzoate) en C-3, et donc assure la position en 4 et 6 de l'isopropylydène.

— Hydrolyse et benzoylation sélective en 6 du composé 5.

Un mélange du composé 5 (72 mg) et d'acide acétique à 80 % (5 ml) est chauffé à 100 °C sous agitation pendant 30 minutes. Après refroidissement à la température ambiante, le mélange réactionnel est évaporé à sec, évaporé avec de l'eau (3 fois 10 ml), puis avec du toluène. Le résidu solide est séché au dessicateur sous haut-vide.

Le diol brut est dissous dans un mélange de pyridine anhydre (0,5 ml) et de dichlorométhane (3 ml). Du cyanure de benzoyle (33 mg) est ajouté et le mélange réactionnel est agité pendant 16 heures. Du méthanol (5 ml) est ajouté, et, après 1 heure sous agitation, le mélange réactionnel est évaporé à sec. Le résidu est cristallisé dans un mélange acétate d'éthyle-hexane pour donner le composé 6 (71 mg, 86 % à partir du composé 5) ; PF = 180-181 °C ; $[\alpha]_D$ + 109° (c1, chloroforme) ; R.M.N. (90 $MH_z$, $CDCl_3$) : δ : 8,02 (m, 4H, H ortho des 2-benzoyle) ; 7,40 (m, 11H, 1 Ph + H méta, para des 2-benzoyle) ; 5,88 (d, 1H, NH, J = 9 $H_z$) ;

5,38 (d. de d., 1H, H3, $J_{2,3}$ = 10$H_z$, $J_{3,4}$ = 3$H_z$), 5,02 (d, 1H, $H_1$, $J_{1,2}$ = 35$H_z$),

3,30 (1H, OH, échangé avec $D_2O$, 1,81 (s, 3H, NAc).

On notera que par benzylation, O-débenzoylation, puis acylation sélective en C-6, le composé 6 peut conduire à un dérivé du type

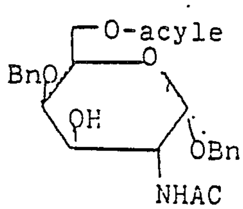

qui est un précurseur convenablement protégé utilisable pour la synthèse de la chondroïtine-6-sulfate.

— Benzylation du dérivé 3.

Le composé 2 (200 mg, 057 mM) est dissous dans du N,N-diméthylformamide anhydre (5 ml). De la baryte anhydre (700 mg, 4,5 mM), de l'hydroxyde de baryum octahydraté (158 mg, 0,5 mM) et du bromure de benzyle (120 μl, 1 mM) sont ajoutés successivement. Le mélange réactionnel est agité à l'abri de l'humidité pendant 20 heures. Du méthanol (0,5 ml) est ajouté, puis, après 30 minutes, le mélange réactionnel est filtré, les solides sont rincés avec du chloroforme (50 ml). La phase organique est lavée avec une solution froide d'acide acétique à 50 %, avec de l'eau, avec une solution aqueuse à 5 % d'hydrogénocarbonate de sodium, avec de l'eau, séchée (sulfate de sodium), filtrée et évaporée. Le résidu est lavé sur une colonne de gel de silice (10 g). L'élution par le mélange acétate d'éthyle-héxane (3 : 1, v/v) donne le composé 7 sous formé d'un verre incolore qu'il n'a pas été possible de cristalliser (228 mg, 91 %; $[\alpha]_D$ + 136° (c 1,5, chloroforme), R.M.N. (90 $MH_z$, $CDCl_3$) : δ : 7,30 (m, 10H, 2 Ph), 5,86 (d, 1H, NH, J = 8,5 $H_z$), 4,89 (d, 1H, $H_1$, $J_{1,2}$ = 3,5 $H_z$); 1,93 (s, 3H, NAC), 1,55 et 1,31 (2s, 2 × 3 H, Isopropyl.).

— Hydrolyse acide du dérivé 7.

Un mélange du composé 7 (150 mg) et d'acide acétique à 80 % (5 ml) est agité à 100 °C pendant 1/2 heure. Après refroidissement à la température ambiante, le mélange réactionnel est évaporé à sec, évaporé avec de l'eau (3 fois 10 ml), puis avec du toluène. Le résidu gélatineux est cristallisé dans l'éthanol pour donner le diol 8 (121 mg, 89 %), PF = 183-184 °C $[\alpha]_D$ + 171° (c1, méthanol).

— Préparation du dérivé 4-0-acétylé 9.

Un mélange du composé 8 (100 mg), de toluène anhydre (5 ml), de triméthylorthoacétate (0,5 ml) et d'acide para-toluène sulfonique (monohydrate, 1 mg) est agité à l'abri de l'humidité pendant 1 heure (le milieu devient homogène après environ 45 minutes). De la triéthylamine (0,2 ml) est ajoutée et le mélange réactionnel est dilué avec du toluène (20 ml). La phase organique est lavée avec de l'eau (2 fois), séchée (sulfate de sodium), filtrée et évaporée. Le spectre de R.M.N. du produit brut est en accord avec la structure attendue (δ : 3,24 (s, 3H, OMe); 1,65 (s, 3H, CMe), mais l'orthoester instable est engagé

immédiatement dans la réaction suivante:

Un mélange de l'orthoester précédent et d'acide acétique à 80 % (5 ml) est agité 10 minutes à la température ambiante, puis évaporé à sec. Ce résidu est évaporé avec de l'eau, puis avec du toluène. Une cristallisation dans un mélange acétate d'éthyle-hexane donne le composé 9 (95 mg, 85 % à partir du composé 8) PF = 146° – 147 °C, $[\alpha]_D$ + 94°, (c1, chloroforme), R.M.N. (90 MHz, $CDCl_3$) : δ : 7,32 (m, 10H, 2 Ph),

5,92 (d, 1H, NH, J = 8,5 $H_z$), 5,37 (d. de d., 1 H, $H_4$, $J_{3,4}$ = 3 $H_z$, $J_{4,5}$ = 1 $H_z$),

4,96 (d, 1H, H1, $J_{1,2}$ = 3,5 $H_z$), 3,60 (1H, OH, échangé avec $D_2o$),

2,11 et 1,95 (2s, 2 × 3 H, OAc et NAc).

(La présence à δ = 5,37 d'un doublet de doublet ayant des constantes de couplage de 3 et 1$H_z$ montre de manière non ambiguë la présence d'un groupement acylé (acétate) en C-4).

Le dérivé 9 est un précurseur de choix pour la préparation du disaccharide de base des:

— chondroïtine-4-sulfate,

— (acide L-iduronique 1 $\xrightarrow{d}$ 3 N-Ac-D-galactosamine-4-0-sulfate).

— Condensation entre l'alcool 9 et l'imidate 10.

Imidate 10 : Lit. : R.R. Schmidt and G. Grundler, Synthesis, (1981), 885-87.

Une solution de l'alcool 9 (76 mg, 0,17 mM) et de l'imidate 10, (175 mg, 0,28 mM) dans le dichlorométhane anhydre (2,5 ml) est agitée à l'abri de l'humidité en présence de tamis moléculaire 0,4 nm (poudre, 100 mg). Le mélange réactionnel est refroidi à 0 °C, et de l'éthérate de trifluorure de bore ($BF_3$ : $Et_2$°,.4 μl, 32 μM) est ajouté en une seule fois. Après agitation 1 heure à 0 °C, puis 3 jours à la température ambiante, de l'hydrogénocarbonate de sodium (100 mg) est ajouté. Après 15 minutes, les solides sont essorés, rincés avec du dichlorométhane (50 ml) et la phase organique est lavée avec une solution aqueuse à 5 % d'hydrogénocarbonate de sodium, avec de l'eau, séchée (sulfate de sodium), filtrée et évaporée.

Le résidu est chromatographié sur une colonne de gel de silice (18 g). L'élution par le mélange acétate d'éthyle-hexane (1 : 1, v/v) permet d'isoler :

— une fraction disaccharidique (62 mg),

— le produit de départ qui n'a pas réagi (47 mg, 60 %).

La fraction disaccharidique est rechromatographiée sur une colonne de gel de silice (5 g, gel 230-400 mesh). L'élution par le mélange dichlorométhane-acétate d'éthyle (5 : 1, v/v) permet d'isoler (par ordre d'élution) :

— le disaccharide 1 $\xrightarrow{\alpha}$ 3 12, sirop incolore (24 mg, 15 %), $[\alpha]_D$ + 98° (c1, chloroforme) ; R.M.N. (90 $MH_z$, $CDCl_3$) : δ : 7,30 (m, 25H, 5 Ph) ;

5,65 (d, 1H, NH, J = 9,5 $H_z$) ; 5,52 (d. de d., 1 H, $H_4$, $J_{3,4}$ = 3$H_z$) ;

5,05 (d, 1H, $H'_1$, $J_{1',2'}$ = 3,5 $H_z$) ; 4,95 (d, 1H, H1, $J_{1,2}$ = 3,5 $H_z$) ; 3,63 (s, 3H, COOMe), 1,92 et 1,82 (2s, 2 × 3 H, OAc et NAc) ;

— le disaccharide 1 $\xrightarrow{\beta}$ 3 11, sirop incolore (24 mg, 15 %), $[\alpha]_D$ + 80° (c1, chloroforme) ; R.M.N. (90 $MH_z$, $CDCl_3$) : δ : 7,30 (m, 25 H, 5 Ph) ; 5,48 (d, 1H, NH, J = 9$H_z$) ; 5,46 (d. de d., 1 H, $H_4$, $J_{3,4}$ = 3$H_z$) ; 4,97 (d, 1H, H1, $J_{1,2}$ = 3,5 $H_z$) ; 3,78 (s, 3H, COOMe) ; 2,04 et 1,61 (2s, 2 × 3H, OAc et NAc).

## Exemple 2

Synthèse d'un disaccharide de type

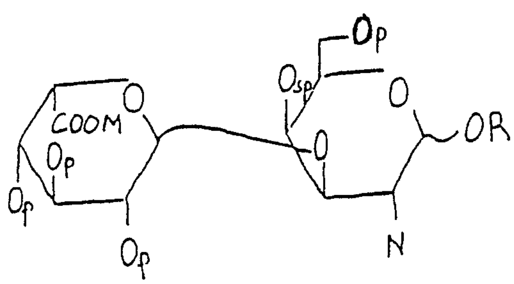

Le disaccharide 22 obtenu par voie de synthèse répond à la formule ci-dessus dans laquelle M représente Me, p et R, un groupe benzyle, sp un groupe -OH, et N un groupe -NH-acétyle.

On effectue cette synthèse à partir du 1,6 anhydro-α-L-idopyranose selon les étapes 1 à 6 suivantes (voir figure 3).

1) — Préparation du 1,6-anhydro-2,3,4-tri-0-benzyl-α-L-idopyranose

A une solution de 1,6-anhydro-α-L-idopyranose (1g) dans le DMF anhydre (60 ml), on ajoute 1,5 g de NaH (émulsion à 50 %) dans l'huile) puis du 4 ml de bromure de benzyle.

Après une heure d'agitation à température ambiante, on ajoute 5 ml de méthanol puis le mélange est concentré à sec. Ce produit est extrait par du dichlorométhane.

La phase organique est lavée à l'eau et reséchée sur sulfate de sodium.

Le sirop obtenu est passé sur colonne de gel de silice éluée d'abord par de l'hexane puis un mélange hexane acétate d'éthyle 8/1, v/v, puis 4/1 v/v. Le sirop obtenu cristallise au cours du séchage (2,5 g-88 %).

On recristallise le produit dans un mélange éther-hexane $[\alpha]_{20}^{D} = +30°$ (c = 1 chloroforme) - PF : 69-70 °C.

2) — Acétolyse —

On soumet à agitation pendant 4 heures à la température ambiante et à l'abri de l'humidité une solution de 13 (405 mg) dans un mélange de 10 ml d'anhydride acétique et de 2 ml d'acide fluoroacétique.

On ajoute 20 ml de xylène puis on évapore à sec le mélange réactionnel, on réalise une co-évaporation avec du xylène (2 fois 5 ml) et on effectue un séchage sous vide. On obtient 455 mg du composé 14 (rendement 91 %) sous forme d'un sirop incolore. Le spectre RMN est conforme avec la structure attendue. $[\alpha]_D^{20\,°C} = +2{,}9°$ (c = 1, $CHCl_3$)

3) Elimination des groupes acétyle du composé 14 — On traite une solution du composé 14 (534 mg) dans 10 ml de méthanol anhydre à 4 °C par une solution de 0,1 ml de méthylate de sodium M pendant environ 14 heures.

Le mélange réactionnel est neutralisé par de la résine amberlite IR 120($H^+$), la résine est filtrée puis le solvant est évaporé.

Le résidu est cristallisé dans un mélange éther-hexane ce qui conduit à 412 mg du composé 15 (rendement 91 %) PF : 81-82 °C ; $[\alpha]_D^{20\,°C} = +11°$ (c = 1, $CHCl_3$).

Le spectre RMN et l'analyse centésimale sont conformes avec la structure attendue.

4) Monochloacétylation du composé 15 — Une solution de 740 mg du diol 15 dans un mélange de 8 ml de dichlorométhane anhydre et 1 ml de pyridine anhydre est refroidie sous agitation à − 20 °C sous atmosphère d'argon sec.

On ajoute goutte à goutte en 10 minutes une solution de 0,40 ml de chlorure de monochloroacétyle dans 2 ml de dichlorométhane anhydre.

Le mélange est soumis à agitation 40 minutes à — 20 °C puis versé toujours sous agitation dans un mélange eau-glace (100 ml).

Après une heure, le mélange est extrait avec du dichlorométhane (3 fois 20 ml) les phases organiques sont lavées avec KH $SO_4$ à 10 %, avec de l'eau, avec $NaHCO_3$ saturé, avec de l'eau puis sont séchées sur du sulfate de sodium, filtrées et évaporées.

Le résidu est lavé sur une colonne de gel de silice de 20 g. On effectue l'élution à l'aide d'un mélange hexane-acétate d'éthyle 7 : 2 (v/v).

On obtient 854 mg du composé 16 sous forme d'un sirop incolore (rendement 87 %, $[\alpha]_D^{20\,°C} = +5°$ (c = 1 $CHCL_3$). Le spectre RMN est conforme avec la structure attendue.

5) Préparation du chlorure d'idopyranosyle 17 —

Une solution de 260 mg du composé 16 dans 2 ml de dichlorométhane anhydre est traitée à 0 °C par une solution saturée de HCl (gaz séché) dans 10 ml de dichlorométhane anhydre pendant 3 heures.

Le mélange réactionnel est alors évaporé à sec, coévaporé avec du toluène (3 fois 20 ml) et séché sous vide élevé. On obtient 216 mg du composé 17 sous forme d'un sirop incolore instable (rendement 92 %). $[\alpha]_D^{20\,°C} = -41°$ (c = 1, $CHCl_3$). Le spectre RMN confirme la structure attendue.

6) Condensation entre le chlorure 17 et l'alcool 18 —

On soumet à agitation un mélange de 153 ml de chlorure 17 (0,28 mml) et de 80 mg d'alcool 18 (0,18 mmole) dans 3,5 ml de dichlorométhane anhydre en présence de 100 mg de tamis moléculaire 0,4 nm à la température ambiante sous atmosphère d'argon sec.

On refroidit le mélange à − 20 °C et on ajoute alors 66 microlitres de sym-collidine et 108 mg de triflate d'argent.

Le mélange réactionnel est soumis à agitation à l'abri de la lumière et on laisse la température remontée lentement vers la température ambiante pendant environ 14 heures.

On dilue ensuite le mélange avec 50 ml de dichlorométhane puis on le filtre sur un lit de célite 545.

Le filtre est lavé HCL 0,1N glacé, avec de l'eau, $NaHCO_3$ saturé puis de l'eau. Le filtrat est alors séché sur du sulfate de sodium filtré puis évaporé.

Le résidu est chromatographié sur une colonne de 30 gr de gel de silice.

L'élution par le mélange acétate d'éthyle-hexane 1 : 1 (v/v) donne par ordre d'élution de disaccharide 19 et le disaccharide 20.

Le disaccharide 19 cristallise dans un mélange éther-hexane, on récupère 45 mg du produit, ce qui correspond à un rendement de 26 %. $[\alpha]_D^{20\,°C} = +48°$ (c = 1 $CHCl_3$). Le spectre RMN confirme la structure attendue.

Le disaccharide 20 se présente sous forme d'un sirop (117 mg 68 %). $[\beta]_D^{20\,°C} = +92°$ (c = 1 $CHCl_3$).

L'anomérie des deux disaccharides est déduite de spectres de RMN ($J_{1',2'}$ 0,5 Hz pour et $J_{1',2'}$ = 2Hz pour et de la valeur du pouvoir rotatoire P.F. : 73-74 °C.

7) Elimination du groupe monochloroacétyle en position 6'.

Une solution des 19 et 20 (40 mg) dans un mélange de pyridine (1 ml) et d'éthanol (0,2 ml) est agitée 20 minutes à 100 °C en présence de thiourée (7 mg). Après refroidissement et traitement habituel, le

résidu est lavé sur une colonne de gel de silice (2 g). L'élution par le mélange hexane-AcOEt (3 : 1 v/v) donne le composé 21 (30 mg, 81 %), sirop incolore.

Les spectres RMN sont en accord avec les structures attendues.

8) Oxydation pour le passage idose-acide iduronique.

Le composé 21 (30 mg) est oxydé à 0 °C dans l'acétone (3 ml) par le mélange $CrO_3/H_2SO_4$ 3,5 M pendant 1 heure. Après traitement habituel, le résidu est dissout dans THF: eau (4 : 1, v/v) est traité par de la soude 2M (1 ml).

Après 3 heures, le mélange réactionnel est acidifié à pH 1 avec HCl M, extrait au chloroforme. Les phases organiques sont lavées avec de l'eau, séchées et évaporées. Le résidu est traité par une solution éthérée de diazo-méthane pendant une demi-heure. Après évaporation à sec, le résidu est lavé sur une colonne de gel de silice (2 g). L'élution par le mélange hexane-AcOEt (5 : 2, v/v) donne le composé 22 (22 mg, 75 %), sirop incolore.

RMN ($CDCl_3$) : δ 3,40 (s, 3H, COOMe ido), 2,85 (1H, OH). Le reste du spectre est en accord avec la structure attendue.

Par O-sulfatation (par exemple avec un complexe $SO_3 : Me_3N$ dans le DMF) puis hydrolyse (par exemple à l'aide de Pd/c dans $MeOH{\cdot}H_2O$) ; on obtient le motif de base du dermatane-sulfate.

Exemple 3

Synthèse d'un dérivé A du type

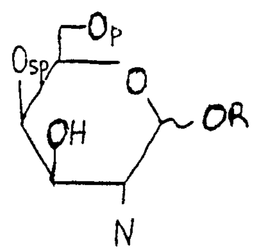

utilisable dans une séquence de type $(U\text{-}A)_n$ et représentant le motif A de la partie terminale réductrice de la séquence.

On prépare ainsi le motif A de formule 26 selon les étapes a) à c) suivantes, à partir du dérivé de formule 23, lui-même obtenu selon la méthode de H. Paulsen et al dans Tetrahedron. Lett., 24 (1983) 1759-1762. (voir figure 4).

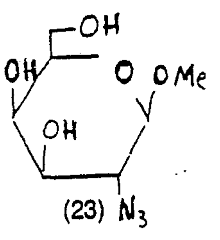

(23)

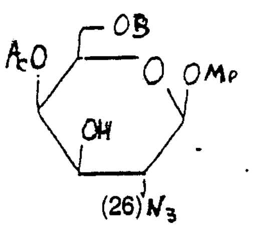

(26)

a) Préparation du dérivé 3,4-O-isopropylidène 24

Une solution de 23 (635 mg) dans l'acétone anhydre (20 ml) est agitée à la température ambiante en présence d'acide p-toluènesulfonique (monohydrate, 40 mg) pendant 5 heures. De la triéthylamine (1 ml) est ajoutée, et le mélange réactionnel est évaporé à sec. Le résidu est chromatographié sur une colonne de gel de silice (40 g). L'élution par le mélange $CH_2Cl_2$-AcOEt (5 : 1, v/v) contenant 0,2 % de triéthylamine donne, par ordre d'élution:

— le dérivé 4,6-O-isopropylidène, (202 mg, 27 %) qui est hydrolysé et recristallisé pour redonner 23.

— le dérivé attendu 24 (488 mg, 65 %), F 84-85° (éther-hexane), $(\alpha)_D + 43°$ (cl, $CHCl_3$). (Spectre RMN conforme avec la structure attendue). (AcOEt représente l'acétate d'éthyle).

b) Benzylation et hydrolyse de 25.

Une solution de 24 (520 mg) dans le DMF anhydre (8 ml) est traitée successivement par de l'hydrure de sodium (196 mg, à 50 % dans l'huile) et du bromure de benzyle (0,36 ml). Après 1 heure, du méthanol (1 ml) est ajouté avec précaution, et le mélange réactionnel est évaporé à sec. Le résidu est repris dans du chloroforme (50 ml) et la phase organique est lavée avec de l'eau, séchée (sulfate de sodium) et évaporée.

Le résidu est dissous dans de l'acide trifluoroacétique à 90 % (10 ml), agité 10 minutes à la température ambiante et évaporé à sec. Le résidu est lavé sur une colonne de gel de silice (10 g). L'élution

par le mélange AcOEt : hexane (1 : 1, v/v) donne le dérivé 25, 540 mg, 87 %), sirop incolore, $(\alpha)_D$ + 5° (cl, chloroforme). (Spectre RMN conforme avec la structure attendue).

c) Acétylation sélective pour donner le dérivé semi-ouvert 26.

Le diol 25 (450 mg) est agité dans un mélange de toluène anhydre (10 ml) et de triméthyl orthoacétate (1,5 ml) à la température ambiante. On ajoute alors l'acide p-toluène-sulfonique (monohydrate, 3 mg) et agite encore 1 heure. De la triéthylamine (1 ml) est ajoutée, le mélange est dilué avec du toluène (30 ml) et lavé avec de l'eau, séché (sulfate de sodium) et évaporé.

Le résidu est dissout dans de l'acide acétique à 80%, agité 10 minutes à la température ambiante, puis évaporé à sec pour donner quantitativement le composé 26 (485 mg, 95 % à partir de 25, sirop, $(\alpha)_D$ - 45° (cl, $CHCl_3$). RMN ($CDCl_3$) δ : 7,25 (s, 5H, Ph), 5,26 (d. de d., 1H, $J_{3,4}$ : 3 Hz, $J_{4,5}$ : 1Hz, H-4), 4,14 (d, 1H, $J_{1,2}$ : 7Hz, H-1), 3,52 (s, 3H, OMe), 2,90 (d, 1H, J, 3Hz, OH).

Exemple 4

Préparation d'un composé A ouvert du type

Le dérivé préparé répond à la formule 31.

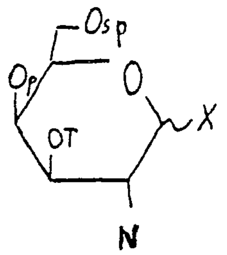

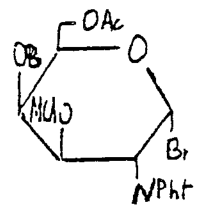

et est obtenu selon les étapes 1 à 4 suivantes à partir du dérivé 27

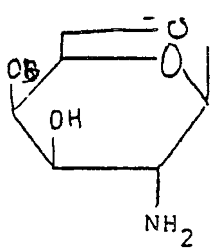

dont la synthèse est rapportée par R.W. Jeanloz et al dans J.A.C.S., 76 (1954) 5682 (voir figure 5).

1) N-phtalimidoylation de 28.

Une solution de 27 (100 mg) dans le méthanol (1 ml) est traitée à 60° pendant 1 heure par de l'anhydride phtalique (75 mg) et de la triéthylamine (60 µl). Après refroidissement, le mélange est évaporé à sec et cristallisé dans un mélange $CH_2Cl_2$-éther-hexane. Le composé intermédiaire 2-(2'-carboxybenzamido) est dissout dans l'anhydride acétique (5 ml) et chauffé à 100 °C pendant 2 heures en présence d'acétate de sodium (100 mg). Après refroidissement, le mélange est versé dans de l'eau glacée et agité 1 heure. Après extraction avec du chloroforme, les phases organiques sont lavées à l'eau, séchées et évaporées. Le résidu est désacétylé par le méthylate de sodium pour donner 28 (101 mg, 66 %), sirop cristallisé dans l'éther, PF : 157 °C $(\alpha)_D$ + 82° (cl, chloroforme).

Le spectre RMN est en accord avec la structure attendue.

2) Monochloroacétylation de 28

Le composé 28 (100 mg) est traité à — 20 °C par le chlorure de monochloroacétyle de la manière habituelle. Le mélange réactionnel est traité de façon classique pour donner 29 (115 mg, 96 %), sirop, $(\alpha)_D$ + 55° (cl, $CH_2Cl_2$). Le spectre RMN est en accord avec la structure attendue.

3) Acétolyse du 1,6-anhydro 29

Le composé 29 (100 mg) est traité par le mélange anhydride acétique : acide trifluoroacétique (9 : 1,

v/v, 10 ml) pendant 24 heures, puis évaporé à sec et séché sous haut vide pour donner le mélange d'acétates α, β 30 (115 mg, 99 %) sous forme d'un sirop.

Le spectre RMN ($CDCl_3$) montre la présence de 3s (6H au total) à δ 2,05, 1,99 et 1,97 attribués au 6-OAc, 1-OAc α et β.

4) Préparation du bromure 31.

Une solution du mélange d'acétates 30 (100 mg) dans un mélange de dichlorométhane (4,5 ml) et d'acétate d'éthyle (0,5 ml) est traitée par du tétrabromure de titane ($TiBr_4$, 100 mg) pendant la nuit. Le traitement habituel donne le bromure 31 (90 mg, 84 %), sirop incolore, RMN ($CDCl_3$) 6,69 (d, 1H, $J_{1,2}$ : 3,5 Hz, H-1). Le reste du spectre est en accord avec la structure attendue.

Ce composé instable est immédiatement utilisé pour les réactions de glycosylation.

Exemple 5

Préparation d'un composé A de formule 39

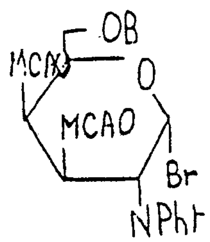

à partir du dérivé 32 de formule:

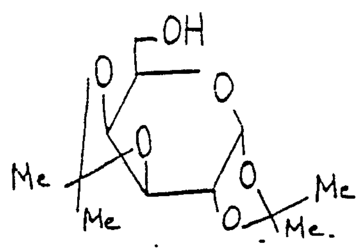

On opère selon les étapes 1 à 7 suivantes : (voir figure 6).

1-Benzylation du 1,2 : 3,4-di-O-isopropylidène-α-D-galactopyranose 32.

Le composé 32 (2,60 g) est benzylé dans le DMF (20 ml) par action du bromure de benzyle (2 ml) et de l'hydrure de sodium (1 g). Le traitement habituel donne un résidu qui est distillé sous vide pour donner 33 (3,16 g, 90 %), $Eb^{0,1}$ : 130-132 °C.

Le spectre RMN est en accord avec la structure attendue.

2-Hydrolyse et acétylation du dérivé 33

Le dérivé 33 (1 g) est dissous dans l'acide trifluoroacétique à 90 % (20 ml), agité 30 minutes à la température ambiante, puis évaporé à sec. Le résidu est évaporé avec de l'eau (3 fois 10 ml) puis séché sous vide.

Le résidu est dissous dans l'anhydride acétique (10 ml) et chauffé à 100 °C cependant 3 heures avec de l'acétate de sodium anhydre (500 mg). Après refroidissement et traitement habituel, le résidu est chromatographié sur une colonne de gel de silice (100 g). L'élution par le mélange hexane-acétate d'éthyle (3 : 2, v/v) donne, par ordre d'élution :

— une fraction contenant les isomères furanniques (480 mg, 38 %), pour laquelle l'isomère α est majoritaire (RMN : δ 6,32 (d, 1H, $J_{1,5}$ Hz, $H_1^{\alpha}$).

— une fraction contenant l'acétate 34 (612 mg, 49 %), sirop incolore, RMN : δ 5,68 (d, 1H, $J_{1,2}$ 7,5 Hz, $H_1$). Le reste du spectre est en accord avec la structure attendue.

3-Préparation du « galactal » 35

— Une solution de l'acétate 34 (1 g) dans l'éther (10 ml) est agitée à la température ambiante en présence de benzylamine (1 ml) pendant 2 heures. Après dilution au dichlorométhane (50 ml), la phase organique est lavée avec HCl 0,1 M glacé, avec de l'eau, séchée (sulfate de sodium) et évaporée.

— Le résidu (produit réducteur, libre sur le carbone anomère) est dissout dans le dichlorométhane anhydre (10 ml) et traité par un excès de bromure de diméthylbromoformiminium (réactif de Vilsmeier

bromé) en présence de symcollidine (0,5 ml) pendant 1 heure à la température ambiante. Le traitement habituel donne le dérivé α-bromo intermédiaire, instable, qui est directement utilisé pour la réaction d'élimination (885 mg, 81 %). RMN : δ : 7,25 (m, 5H, Ph), 6,31 (d, 1H, $J_{1,2}$ 3,5 Hz, H-1).

— Une solution du bromure (885 mg, fraîchement préparé) dans l'acide acétique (5 ml) est ajoutée goutte à goutte à 0 °C à un mélange d'acide acétique (4 ml), d'eau (15 ml), d'acétate de sodium (2 g), de zinc en poudre (1 g) et de $CuSO_4$, $5H_2O$ (0,1 g). Après 4 heures d'agitation violente à 0 °C, le mélange est filtré, le filtrat dilué avec de l'eau, et extrait au chloroforme (3 fois 20 ml). La phase organique est lavée à l'eau, séchée (sulfate de sodium) et évaporée. Le résidu est lavé sur une colonne de gel de silice (50 g). L'élution par le mélange hexane-acétate d'éthyle (5 : 3, v/v) donne le galactal 35 sirop incolore (595 mg, 65 %), le spectre RMN est en accord avec la structure attendue.

4-Azidonitration du galactal 35

Une solution du galactal 35 (1 g) dans l'acétonitrile anhydre (10 ml) est agitée à — 20 °C sous atmosphère d'argon sec en présence d'azoture de sodium (200 mg) et de cérium-ammonium nitrate (3,5 g) pendant 12 heures. Après dilution avec de l'éther froid (50 ml), la phase organique est lavée à l'eau, séchée (sulfate de sodium) et évaporée. Le résidu (contenant principalement le mélange de nitrates α,β) est immédiatement dissous dans l'acide acétique (10 ml) et chauffé à 100 °C en présence d'acétate de sodium anhydre (400 mg) pendant 1 heure. Le traitement habituel donne un résidu qui est chromatographié sur une colonne de gel de silice (50 g). L'élution par le mélange toluène-acétate d'éthyle (8 : 1, v/v) donne le mélange d'acétates 36 (760 mg, 51 % à partir de 35, qui contient majoritairement l'α-acétate, RMN δ 7,28 (s, 5H, Ph), 6,22 (d, 1H, $J_{1,2}$ 3,5 Hz, $H_1^{\alpha}$).

5-Préparation du dérivé N-phtalimido 37

— Le mélange d'acétates 36 (200 mg) est dissous dans de l'éthanol (10 ml) contenant $NiCl_2$, $6H_2O$ (0,4 g) et de l'acide borique $H_3BO_3$ (0,2 g). On ajoute alors goutte à goutte une solution de $NaBH_4$ dans l'éthanol (10 mg/ml) jusqu'à disparition de la couleur verte et apparition d'une couleur gris-noir. Le mélange est alors évaporé à sec et dilué avec de l'eau, puis extrait au chloroforme (5 fois 10 ml). La phase organique est lavée à l'eau, séchée (sulfate de sodium) et évaporée.

— Le résidu (dérivé 2-amino, donnant une tache rose par vaporisation de ninhydrine en c.c.m) est immédiatement traité dans le méthanol (3 ml) par l'anhydride phtalique (150 mg) en présence de triéthylamine (120 µl) à 60 °C pendant 2 heures. Après traitement habituel, le résidu est traité à 100 °C pendant 2 heures par l'anhydride acétique (10 ml) et l'acétate de sodium (200 mg). Après traitement, le résidu obtenu est chromatographié sur une colonne de gel de silice (15 g). L'élution par le mélange acétate d'éthylehexane (1 : 1, v/v) donne le mélange d'acétates 37 (152 mg, 66 %). Le spectre RMN est en accord avec la structure attendue. Le spectre IR montre l'absence totale de bande correspondant au groupe azide.

6-Préparation du dérivé 38

Le mélange d'acétates 37 (150 mg) est désacétylé de manière classique à 0 °C (MeONa dans le méthanol). Le résidu est monochloroacétylé à − 20 °C dans un mélange pyridine-dichlorométhane de manière habituelle, pour donner le mélange de monochloroacétates anomères α,β, sirop (155 mg, 87 %). Le spectre RMN est en accord avec la structure attendue.

7-Préparation du bromure 39

Une solution du mélange de monochloroacétates 38 (100 mg) dans le dichlorométhane (5 ml) et d'acétate d'éthyle (0,5 ml) est traitée à la température ambiante par du tétrabromure de titane ($TiBr_4$, 100 mg) pendant 16 heures. Le traitement habituel donne le bromure 39 (76 mg, 75 %), sirop instable. RMN δ 8,0-7,10 (m, 9H, aromatique), 6,59 (d, 1H, $J_{1,2}$ : 3,5 Hz, H-1). Le reste du spectre est en accord avec la structure attendue.

Ce composé « activé » est utilisé immédiatement pour les réactions de glycosylation ultérieures.

Exemple 6

Préparation d'un dérivé « U-A » semi-ouvert du type

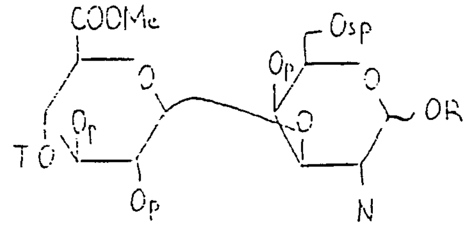

— La partie « A » est un motif semi-ouvert utilisable dans le cas des chondroïtines-6-sulfate et est préparée selon le schéma :

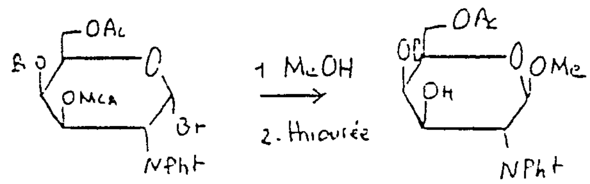


— La partie U dérive de :

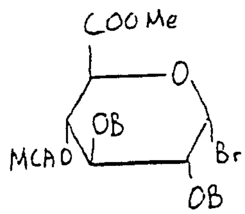

— Le couplage est effectué selon le schéma de la figure 7 pour donner le disaccharide 43.

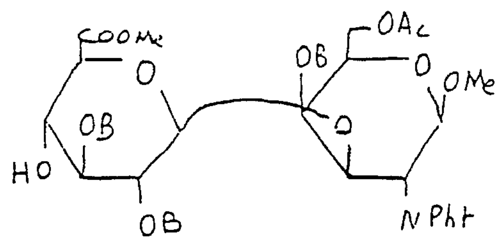

— Synthèse de 40 à partir du bromure 27

— Une solution du bromure 27 (200 mg) dans le dichlorométhane anhydre (3 ml) est agitée à l'abri de la lumière et de l'humidité en présence de silicate d'argent (300 mg), de tamis moléculaire 0,4 nm en poudre (100 mg) et de méthanol (0,25 ml) pendant 5 heures à la température ambiante. Les solides sont filtrés, rincés avec du dichlorométhane, et le filtrat est évaporé.

— Le résidu est dé-O-monochloroacétylé par la thiourée dans le mélange pyridine-éthanol de façon habituelle. Le résidu est lavé sur une colonne de gel de silice (15 g). L'élution par le mélange AcOEt-hexane (2 : 1, v/v) donne le dérivé 40 (122 mg, 78 %), sirop incolore. RMN : δ 3,60 (s, 3H, OMe), 2,30 (1H, OH, échangeable avec $D_2O$), 2,06 (s, 3H, OAc).

— Condensation des motifs 40 et 41

Une solution du bromure 41 (158 mg) et d'alcool 40 (91 mg) dans le dichlorométhane anhydre (5 ml) est agitée à la température ambiante à l'abri de la lumière et de l'humidité en présence de carbonate d'argent fraîchement préparé (120 mg) et de tamis moléculaire 0,4 nm en poudre (200 mg) pendant 3 jours.

Les solides sont filtrés, le filtrat est évaporé et le résidu est chromatographié sur une colonne de gel de silice (20 g). L'élution par le mélange AcOEt : hexane (1 . 1, v/v) donne le disaccharide 42 (106 mg, 52 %), sirop.

Le spectre RMN est en accord avec la structure : δ : 3,70 (s, 3H, COO Me), 3,50 (s, 3H, OMe), 2,04 (s, 3H, OAc).

— dé-O-monochloroacétylation du dérivé 42

Le disaccharide 42 (80 mg) est dé-O-monochloroacétylé par l'hydrazine-dithiocarbonate (H.D.T.C.) selon le procédé décrit par C.A. van BOECHEL and T. BEETZ, Tetrahedron, Letters, 24 (1983) 3775. Après traitement, le résidu est lavé sur une colonne de gel de silice (5 g). L'élution par le mélange AcOEt-hexane (2 : 1, v/v) donne le disaccharide semi-ouvert 43 (67 mg, 90 %), sirop. RMN : δ 8,0-7,10 (m, 19H, aromatique), 3,74 (s, 3H, COOMe), 3,46 (s, 3H, OMe), 2,70 (1H, OH, échangeable avec $D_2O$), 1,98 (s, 3H, OAc).

Exemple 7

Synthèse de dérivés A-U semi-ouverts du type

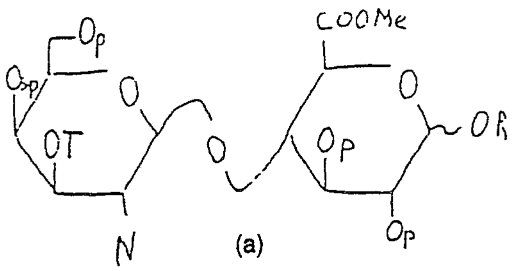
(a)

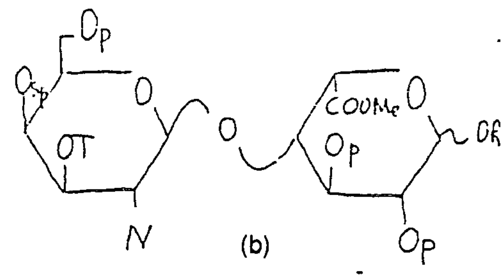
(b)

Ces dérivés sont utilisables dans le cas des chondroïtines-4-sulfate (a) et du dermatane-sulfate (b).

— Les motifs A sont préparés à partir du dérivé 39.

— Les motifs U correspondent aux produits 13 et 115 de la demande de brevet FR 82 18003 du 27.10/1982 au nom de la Demanderesse et répondent selon la numérotation de la présente demande aux formules 44 et 45.

On se référera à la figure 8.

a) Préparation d'un dérivé type (a).

On procède selon les étapes 1 et 2 suivantes :

1) Réaction de condensation pour la préparation de 46

Une solution de 39 (124 mg) et de 44 (64 mg) dans le dichlorométhane anhydre (3 ml) est agitée à — 20 °C en présence de tamis moléculaire 0,4 nm en poudre (100 mg) sous atmosphère d'argon sec. On ajoute alors successivement de la sym-collidine (0,10 ml) et du triflate d'argent (70 mg), et laisse remonter la température vers la température ambiante pendant la nuit. Les solides sont essorés, lavés au dichlorométhane, le filtrat est lavé avec HCl 0,1 M glacé, avec de l'eau, avec $NaHCO_3$ à 5 %, avec de l'eau, séché (sulfate de sodium) et évaporé. Le résidu est chromatographié sur une colonne de gel de silice (20 g). L'élution par le mélange hexane : AcOEt (2 : 1, v/v) donne le dissaccharide 46 (110 mg, 70 %), sirop. RMN : δ 3,98 et 3,96 (2s, 4H, $2Cl\text{-}CH_2COO$), 3,72 (s, 3H, COOMe). Le reste du spectre est en accord avec la structure attendue.

2) Synthèse du disaccharide semi-ouvert 47

Le disaccharide 46 (100 mg) est dé-O-monochloroacétylé par l'hydrazine dithiocarbonate comme décrit précédemment. Le disaccharide diol obtenu est trouvé dans le toluène (5 ml) par le triméthylorthoacétate (1 ml) en présence d'acide p-toluènesulfonique (1 mg) pendant 2 heures, puis l'orthoester formé est traité par l'acide acétique à 80 % pendant 10 minutes à la température ambiante. Après évaporation à sec, on obtient le dérivé 47 semi-ouvert (72 mg, 80 %), sirop incolore. RMN : δ : 8,0-7,10 (m, 19H, aromatiques), 5,25 (d. de d., 1H, $J_{3',4'}$ : 3 Hz, $J_{4',5'}$ : 1 Hz, H'-4), 3,73 (s, 3H, COOMe), 2,70 (1H, OH, échangeable avec $D_2O$), 2,01 (s, 3H, OAc).

b) Préparation d'un dérivé type (b).

— Réaction de condensation pour la préparation de 48

Les composés 39 (124 mg) et 45 (60 mg) sont condensés et traités de la manière décrite pour la préparation de 46 pour donner le disaccharide 47 (102 mg, 72 %), sirop. RMN : δ 8,0-7,15 (m, 19H, aromatiques), 3,98 et 3,94 (2s, 4H, $2Cl.CH_2COO$), 3,65 (s, 3H, COOMe), 3,46 (s, 3H, OMe).

— Synthèse du dérivé semi-ouvert 49

Le disaccharide 48 (80 mg) est traité comme précédemment pour la préparation du dérivé 47 pour donner 49 (56 mg, 78 %), sirop. RMN : δ 5,22 (d. de d., 1H, $J_{3',4'}$ : 3 Hz, $J_{4',5'}$ : 1 Hz, H'-4), 3,62 (s, 3H, COOMe), 3,42 (s, 3H, OMe), 2,55 (1H, OH, échangeable avec $D_2O$), 1,97 (s, 3H, OAc).

(Voir formule page 25)

Exemple 8

Synthèse d'un dérivé A-U semi-ouvert du type

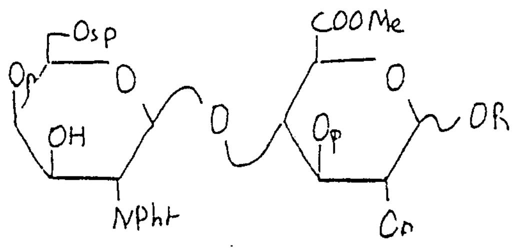

Les dérivés de ce type sont utilisables dans le cas des chondroïtines-6-sulfate. Le motif A est préparé à partir du composé 27 et le motif U correspond au composé 13 de la demande FR 82 18003 portant ici le numéro 44. On prépare le disaccharide 51 en procédant comme suit (voir figure 9).

Réaction de condensation pour la préparation du disaccharide 50.

Un mélange de 44 (64 mg) et du bromure 27 (116 mg) est traité exactement de la manière décrite pour la préparation du disaccharide 46. Le résidu est chromatographié sur une colonne de gel de silice (25 g). L'élution par le mélange hexane-AcOEt (5 : 2, v/v) donne le disaccharide 50 (112 mg, 74 %), sirop. RMN : δ 8,05-7,15 (m, 19H, aromatiques), 3,97 (s, 2H, $Cl-CH_2COO$), 3,75 (s, 3H, COOMe), 2,07 (s, 3H, OAc). Le reste du spectre est en accord avec la structure attendue.

— Dé-O-monochloroacétylation du disaccharide 50

Le disaccharide 50 (95 mg) est dé-O-monochloroacétylé par la thiourée dans le mélange pyridine-éthanol. Après purification sur une colonne de gel de silice (5 g) et élution par le mélange hexane-AcOEt (4 : 3 v/v), on obtient le disaccharide semi-ouvert 51 (64 mg, 72 %), sirop. RMN : δ 8,0-7,10 (m, 19H, aromatiques), 3,78 (s, 3H, COOMe), 2,85 (1H, OH, échangeable avec $D_2O$), 2,06 (s, 3H, OAc).

Exemple 9

Synthèse d'un trisaccharide U-A-U du type

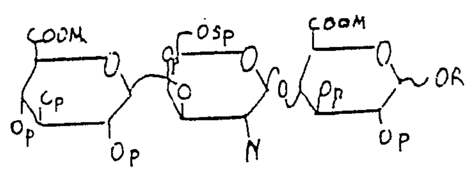

Les trisaccharides de ce type sont utilisables dans le cas des chondroïtines-6-sulfate. Le schéma de synthèse est représenté sur les figures 10 et 11. Le composé 52 est préparé selon la technique de R. R. SCHMIDT et al dans Tetrahedron Letters, 21 (1980) 1421.

— Synthèse du disaccharide 53

Une solution de l'alcool 29 (76 mg) et du bromure 52 (216 mg) dans le dichlorométhane anhydre (5 ml) est agitée pendant 4 jours à la température ambiante à l'abri de la lumière en présence de carbonate d'argent fraîchement préparé (160 mg) et de tamis moléculaire 0,4 nm en poudre (200 mg). Après filtration et évaporation, le résidu est chromatographié sur une colonne de gel de silice (30 g). L'élution par le mélange hexane-acétate d'éthyle (2 : 1, v/v) donne le disaccharide 53 (87 mg, 52 %), sirop. RMN : δ 8,0-7,15 (m, 24H, aromatiques), 3,74 (s, 3H, COOMe), 5,32 (s, large, H-1).

— Acétolyse et bromuration du disaccharide 53

Le disaccharide 53 (100 mg) est acétolysé par le mélange anhydride acétique : acide trifluoroacétique (9 : 1, v/v, 5 ml) pendant la nuit. Après évaporation à sec, le résidu (mélange d'acétates anomères α, β) est traité par la benzylamine dans l'éther comme décrit précédemment pour donner le disaccharide réducteur (libre sur le carbone anomère de A) qui est immédiatement traité par le bromure de diméthylbromoformiminium en présence de sym-collidine pour donner le bromure 54 (78 mg, 68 %), sirop instable utilisé immédiatement. RMN : δ 6,41 (d, 1H, $J_{1,2}$ 3,5 Hz, H-1), 2,05 (s, 3H, OAc). Le reste du spectre est en accord avec la structure attendue.

— Condensation pour la préparation du trisaccharide 55

L'alcool 44 (43 mg) (43 mg) et l'halogénure 54 (144 mg) sont condensés par la méthode au sulfate d'argent comme décrit pour le disaccharide 46. Le résidu est chromatographié sur une colonne de gel de silice (20 g). L'élution par le mélange AcOEt : hexane (1 : 1, v/v) donne le trisaccharide 55 (163 mg, 53 %). RMN : δ 7,95-7,10 (m, 34H, aromatiques), 3,74 et 3,71 (2s, 2 × 3H, 2 COOMe), 2,05 (s, 3H, OAc). Le reste du spectre est en accord avec la structure attendue.

Exemple 10

Synthèse d'un tétrasaccharide du type U-A-U-A

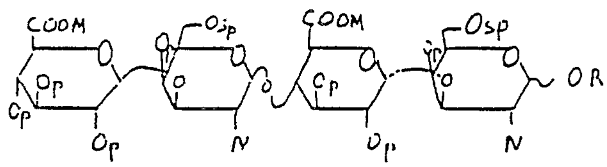

On prépare le tétrasaccharide 56 à partir des disaccharides 54 et 43 en opérant comme suit (voir figure 12) :

Les composés 54 (96 mg) et 43 (90 mg) sont condensés par la méthode au sulfate d'argent comme décrit précédemment pour le disaccharide 46. Le résidu est chromatographié sur une colonne de gel de silice (20 g). L'élution par le mélange hexane-AcOEt (4 : 3, v/v) donne le tétrasaccharide 56 (82 mg, 46 %), verre incolore. RMN : δ : 8,10-7,05 (m, 43H, aromatiques), 3,77 et 3,70 (2s, 2 × 3H, 2 COOMe), 3,46 (s, 3H, OMe), 2,08 et 2,04 (2s, 2 × 3H, 2 OAc).

**Revendications**

1. Procédé de synthèse organique d'oligosaccharides comprenant des enchaînements [D-galactosamine]-[acide D-glucuronique ou acide L-iduronique], ou l'inverse, tels que rencontrés dans le dermatane-sulfate, les chondroïtines, la chondrosine ou les chondroïtines-sulfates, caractérisé par le fait

qu'on met en œuvre, dans une réaction de glycosylation, deux composés terminés ou constitués respectivement par un motif A de structure D-galactosamine et un motif U de structure acide D-glucuronique ou acide L-iduronique, l'un des motifs A ou U étant un alcool dans lequel le groupe —OH de la fonction alcool occupe la position 3 ou 4, l'autre motif possédant un carbone anomère activé, substitué par un groupe réactif compatible avec les autres groupements présents sur les motifs, toutes les autres positions de A et U, excepté celle dont le carbone anomère est activé et celle occupée par le groupe —OH de la fonction alcool, portant, soit des groupes amino, soit des groupes carboxyle, ou des précurseurs de ces groupes, soit encore des groupes —OH, ces groupes occupant des positions déterminées, les groupes amino et carboxyle lorsqu'ils sont présents, étant bloqués respectivement par des groupements protecteurs de fonction amino et carboxyle, les groupes —OH étant bloqués par au moins deux types de groupes protecteurs, éliminables séquentiellement en permettant l'introduction de groupes désirés en certaines positions, puis la libération de groupes —OH en d'autres positions, cette réaction de glycosylation donnant :

soit une liaison 1,3, avec stéréospécificité α, entre un motif acide L-iduronique et un motif D-galactosamine, ou une liaison 1,3 avec une stéréospécificité β entre un motif acide D-glucuronique et un motif D-galactosamine,

soit une liaison 1,4, avec une stéréospécificité β, entre un motif D-galactosamine et un motif acide D-glucuronique ou L-iduronique,

qu'on répète, éventuellement, l'opération de glycosylation afin d'allonger la chaîne oligosaccharidique,

qu'on élimine séquentiellement les groupements protecteurs des groupes —OH en introduisant des groupements de substitution désirés dans des positions spécifiques, puis en libérant les groupements —OH d'autres positions spécifiques ainsi que les groupes amino et carboxyle.

2. Procédé selon la revendication 1, caractérisé en ce que les produits avec les motifs A et U mis en œuvre renferment plusieurs types de groupes protecteurs de radicaux —OH à savoir (1) un ou plusieurs groupes semi-permanents, c'est-à-dire des groupements éliminables en premier lieu après les réactions de glycosylation lorsque le squelette glucidique comporte le nombre de motifs désirés, sans enlèvement ou altération des autres groupes présents, et (2) un ou plusieurs groupes permanents, c'est-à-dire des groupements capables de maintenir la protection des radicaux —OH durant l'introduction de groupements fonctionnels à la place des groupements semi-permanents, ces groupements protecteurs étant choisis parmi des radicaux tels que les radicaux acyle, alcoyle, le cas échéant alcoyle substitué, ou aryle.

3. Procédé selon la revendication 1 ou 2, caractérisé en ce que les groupements protecteurs de

radicaux —OH, sont choisis dans le groupe comprenant les radicaux acyle, notamment acétyle, alcoyle, alcoyle substitué tel que benzyle, et pour deux positions voisines, parmi les groupes acétals ou cétals, par exemple benzylidène, une autre forme de protection consistant à effectuer un blocage de deux groupes —OH sous forme époxyde et/ou de pont 1-6-anhydro.

4. Procédé selon l'une quelconque des revendications précédentes, caractérisé en ce que les produits utilisés dans la réaction de glycosylation renferment plusieurs types de groupements protecteurs, les groupements protecteurs occupant déjà des positions déterminées sur les produits mis en œuvre dans la réaction de glycosylation, ou en variante étant introduits à partir d'autres groupements une fois le squelette glucidique constitué, cette variante comportant, par exemple, l'utilisation pour la glycosylation d'un produit A dans lequel les groupes —OH en position 2 et 3 et en positions 1 et 6 sont bloqués sous forme anhydre, respectivement 2,3-époxyde et 1,6-anhydro, l'ouverture de la fonction époxyde par de l'azide de sodium permettant d'introduire, en position 2, un groupe $N_3$ qui constitue un précurseur de fonction amine.

5. Procédé selon l'une quelconque des revendications précédentes, caractérisé en ce que les radicaux —OH des produits de départ destinés à être sulfatés sont protégés par des groupes acyle, en particulier acétyle tandis que les radicaux —OH destinés à être libérés en fin de synthèse sont protégés par un groupe permanent tel que le groupe benzyle.

6. Procédé selon l'une quelconque des revendications 1 à 5, caractérisé en ce que A comprend en position 2, un groupe azoté, avantageusement constitué par des groupes tels que $—N_3$ ou $NHCOO—CH_2—C_6H_5$, ou tout autre groupe constituant un précurseur de fonction amine ou d'un dérivé d'amine, en particulier de $—NHSO_3^-$ ou de —NH-acyle, plus spécialement de $—NH—COCH_3$.

7. Procédé selon l'une quelconque des revendications 1 à 6, caractérisé en ce que les fonctions carboxyle des motifs U sont bloqués par des groupes inertes vis-à-vis des réactions mises en jeu pour le remplacement des groupes protecteurs et éliminables en fin de synthèse pour libérer les groupes carboxyle, éventuellement aux fins de salification, ces groupes protecteurs de fonction carboxyle étant choisis avantageusement parmi les radicaux alcoyle, ou les radicaux aryle.

8. Procédé selon la revendication 1, caractérisé en ce que les motifs A et U de la séquence formée renferment des groupements protecteurs temporaires, c'est-à-dire des groupements capables de bloquer sélectivement une position du motif A ou U destinée à intervenir dans une nouvelle réaction de glycosylation, ces groupements étant éliminables en présence des autres groupements présents sur les motifs des produits de départ en recréant un alcool.

9. Procédé selon la revendication 8, caractérisé en ce qu'aux fins d'élongation d'une unité disaccharidique U-A vers la droite on met en œuvre un motif U comportant un groupe temporaire et qu'aux fins d'une élongation à gauche on utilise un motif A comportant ledit groupe temporaire, ce qui permet, en effectuant des réactions de glycosylation successives d'effectuer des enchaînements $U_wA_xU_yA_z$ dans lesquels la somme des indices est comprise entre 2 et 12, ces valeurs étant incluses dans l'intervalle, w et y ne pouvant être nuls simultanément, les enchaînements réguliers étant du type $U\ (AU)_n$, $(AU)_n\ A$, $(UA)_n$ ou encore $(AU)_n$ avec $1 \leq n \leq 6$.

10. Procédé selon l'une quelconque des revendications 1 à 9, caractérisé en ce que la fonction alcool de l'un des motifs A ou U impliqué dans la séquence glucidique déjà constituée est avantageusement libérée de son groupement protecteur temporaire par exemple

à partir d'un groupe allyle par un traitement du type comportant l'utilisation d'un agent isomérisant tel que des dérivés de Pd, Rh et Ir, en particulier le chlorure de tris-triphénylphosphine rhodium (I) ou encore le tertiobutoxyde de potassium, puis dans des conditions acides, en particulier avec un mélange d'oxyde mercurique et de chlorure mercurique, ou

par saponification à partir d'un groupe —O-acyle, en particulier —O-acétyle, ou O-chloroacétyle, ces radicaux étant éliminés pour libérer une fonction —OH, par exemple, à l'aide de thiourée en milieu solvant, avantageusement à une température supérieure à 80 °C, de préférence de l'ordre de 100 °C.

11. Procédé selon l'une quelconque des revendications 1 à 10, caractérisé en ce qu'on fait réagir ledit alcool avec un dérivé réactif tel qu'un halogénure, avantageusement un chlorure ou un bromure, un imidate ou un orthoester,

la réaction de condensation entre l'halogénure et l'alcool étant avantageusement du type Koenigs-Knorr, et étant effectuée en milieu solvant, plus spécialement dans un solvant organique, notamment du type dichlorométhane ou dichloroéthane,

avantageusement en présence d'un catalyseur, en général un sel d'argent ou de mercure, par exemple, le trifluorométhane sulfonate d'argent, communément appelé triflate d'argent, le carbonate d'argent, l'oxyde d'argent, le bromure mercurique ou le cyanure mercurique et également avec un accepteur de protons tel que la sym-collidine de même qu'un capteur pour l'eau éventuellement présente et/ou pour l'acide halogénohydrique formé, par exemple des tamis moléculaires 0,4 nm.

à température ambiante ou encore à une température inférieure pouvant atteindre 0 °C ou moins, sous atmosphère d'un gaz inerte tel que l'azote ou l'argon, ou en variante, pour former des liaisons covalentes entre des alcools de structures variées et un précurseur L-iduronique, on réalise la réaction de condensation en utilisant comme catalyseur des dérivés mercuriques, en particulier de cyanure et du bromure mercurique, de tamis moléculaire, en particulier de tamis moléculaire 0,4 nm dans un solvant organique choisi selon la réactivité de l'alcool,

la condensation avec un orthoester tel qu'un groupe 1,2-O-méthoxy-éthylidène étant de préférence effectuée à une température supérieure à 100 °C dans un milieu solvant, du type chlorobenzène ou un solvant analogue avec un point d'ébullition supérieur à 100 °C et avantageusement entre 100 et 150 °C, en présence d'un catalyseur tel que le perchlorate de 2,6-diméthylpyridinium.

la condensation avec un imidate étant réalisée à basse température, en particulier à une température inférieure ou égale à environ 0 °C, en milieu solvant, tel que le dichlorométhane, en présence de tamis moléculaire 0,4 nm et d'un catalyseur tel que de l'éthérate de trifluorure de bore.

12. Procédé selon la revendication 1, caractérisé en ce que la chaîne glucidique élaborée est soumise à une ou plusieurs réactions chimiques afin d'introduire un type de groupements fonctionnels donnés ou, successivement, plusieurs types de groupements, puis de former, si on le désire, des dérivés de ces groupements fonctionnels.

13. Procédé selon la revendication 12, caractérisé en ce que l'étape de fonctionnalisation est réalisée en n'éliminant que certains groupements protecteurs et/ou certains groupements précurseurs des dérivés aminés ou encore la totalité des groupements protecteurs et/ou des groupements précurseurs et en introduisant à leur place un type de substituants donné, ou, successivement, des substituants différents, puis en libérant une partie ou la totalité des groupes —OH encore bloqués, si on le désire.

14. Procédé selon la revendication 12 ou 13, caractérisé en ce que l'étape de fonctionnalisation est réalisée sélectivement de manière à introduire sur la chaîne, successivement, plusieurs types de substituants, en particulier des groupes sulfate, en des positions déterminées des motifs, pour former en position 2 des motifs A un dérivé aminé et en position 6 des motifs U, un dérivé d'acide, puis de libérer les fonctions —OH à d'autres positions, cette étape de fonctionnalisation étant réalisée en utilisant des dérivés dans lesquels les groupes semi-permanents occupent des positions destinées à être sulfatées et sont constitués par des groupes —O-acétyle, les positions correspondant à un groupe —OH destinées à être libérées sont occupées par des groupes permanents constitués par des groupes benzyle et les positions 2 des motifs A sont substituées par des groupes tels que $N_3$ ou $NH—COO—CH_2—C_6H_5$ et les positions 6 des motifs U sont occupées par des groupes carboxyle protégés par un radical alcoyle, en particulier méthyle.

15. Procédé selon la revendication 14, caractérisé en ce que l'étape de fonctionnalisation comprend l'introduction sélective de groupes sulfate après l'élimination des groupes de blocage —O-acétyle à l'aide d'une réaction de saponification réalisée avec une base forte telle que la soude, de préférence à une température inférieure à l'ambiante et plus spécialement voisine de 0 °C, le produit résultant de l'hydrolyse étant alors soumis à l'action d'un agent d'alcoylation afin d'introduire, sur le groupe carboxyle, les groupes alcoyle protecteurs qui se sont trouvés éliminés lors de l'hydrolyse, cette alcoylation étant suivie par un traitement de sulfatation aux fins d'introduction de groupes sulfate aux positions libérées par l'hydrolyse et laissées libres après l'action de l'agent d'alcoylation, la conduite de la sulfatation comprenant la mise en œuvre d'un agent de sulfatation, tel qu'un complexe triméthylamine/$SO_3^-$, en milieu solvant, plus spécialement dans un solvant tel que le diméthylformamide, de préférence, à une température supérieure à l'ambiante, généralement voisine de 50 °C, ce qui correspond à une durée de réaction d'environ 12 heures,

la libération des groupes —OH bloqués par les radicaux benzyle, par l'élimination de ces derniers avantageusement réalisée par hydrogénation catalytique dans des conditions compatibles avec le maintien des groupes sulfate et la transformation des groupes azotés en groupes fonctionnels amine, de préférence sous pression d'hydrogène en présence d'un catalyseur du type Pd/C, en milieu solvant organique, en particulier alcoolique, additionné d'eau,

la formation de groupes N-acétyle en soumettant le produit résultant de la réaction d'hydrogénation à l'action d'un agent d'acétylation tel que de l'anhydride acétique, cette réaction étant avantageusement réalisée à un pH basique, en particulier voisin de 8, en milieu aqueux, ou la formation de groupes N-sulfate à l'aide d'un agent de sulfatation du type indiqué ci-dessus, à un pH supérieur à 9, avantageusement de l'ordre de 9-10,

la libération des groupes carboxyle par addition d'une base forte,

la salification des groupes carboxyle en utilisant par exemple des résines échangeuses comportant le cation désiré, en particulier du sodium ou encore du potassium, du lithium, du magnésium ou du calcium.

16. Procédé selon l'une quelconque des revendications précédentes, caractérisé en ce qu'on met en œuvre, à la place de l'un ou plusieurs des motifs A ou U, un sucre constituant un analogue structural de motif A ou U, tel qu'un sucre neutre ou un désoxy-sucre.

17. Oligosaccharides constituant des intermédiaires dans le procédé selon l'une quelconque des revendications précédentes, caractérisés en ce qu'ils comprennent une chaîne à base de motifs binaires de structure $(A\text{-}U)_n$ ou $(U\text{-}A)_n$ correspondant à des enchaînements x-y ou x-z (ou l'inverse), x, y, z répondant respectivement aux structures :

(Voir formule page 29)

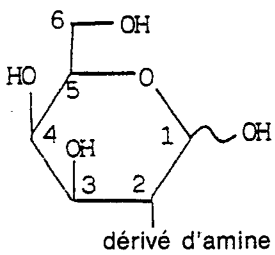

(x) D-galactosamine

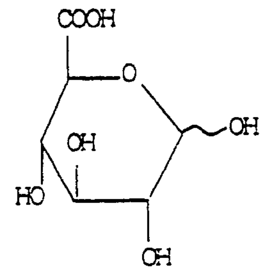

(y) acide D-glucuronique

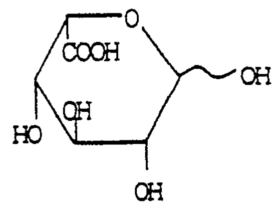

(z) acide L-iduronique

n étant un nombre de 1 à 6, ces motifs binaires étant (1) complètement protégés et possédant soit un groupe réactif sur le carbone anomère du motif à l'extrémité réductrice, soit un seul groupe —OH libre sur le motif à l'extrémité non réductrice, ce groupe —OH occupant la position 3, 4 ou 6 dans le cas d'un motif A et la position 2, 3 ou 4 dans le cas d'un motif U, ou (2) étant constitués par des motifs complètement protégés tels qu'obtenus à l'issue de l'étape de glycosylation, ou (3) comprenant les produits dans lesquels un ou plusieurs groupes —OH sont libérés, ces oligosaccharides intermédiaires comportant le cas échéant un ou plusieurs motifs consécutifs x, y ou z et/ou plusieurs motifs de sucres neutres et/ou plusieurs désoxy-sucres dans leur structure.

18. Oligosaccharides selon la revendication 17, caractérisés en ce qu'ils possèdent la structure de fragments de dermatane-sulfate chondroïtines-sulfates, chondroïtines ou chondrosine et comportent des liaisons z1 $\xrightarrow{\alpha}$ 3x, x1 $\xrightarrow{\beta}$ 4y ou z et y1 $\xrightarrow{\beta}$ 3x.

19. Oligosaccharides selon la revendication 18, caractérisés en ce qu'ils renferment au moins un motif disaccharidique possédant une structure du type x1 $\xrightarrow{\beta}$ 4y ou z, répondant à la formule I :

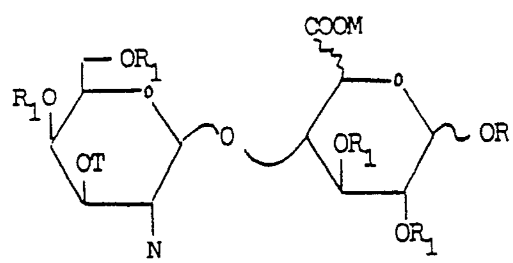

ou un motif disaccharidique possédant une structure du type z1 $\xrightarrow{\alpha}$ 3x ou y1 $\xrightarrow{\beta}$ 3x de formule Ia :

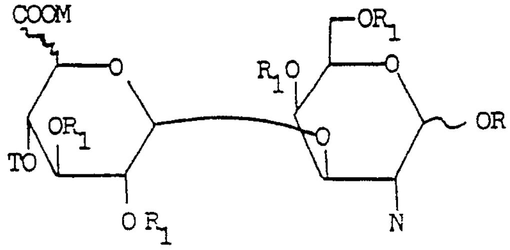

dans laquelle :

les radicaux $R_1$, identiques ou différents les uns des autres, éventuellement conjointement avec R, représentent un groupe protecteur, en particulier un groupement sp semi-permanent ou un groupement p permanent.

T, un groupement temporaire t, un groupement permanent p, ou un atome d'hydrogène,

N, un groupe azoté précurseur d'amine ou de dérivé d'amine,

R, un radical aliphatique ou aromatique, notamment un radical alcoyle comportant de 1 à 4 atomes de carbone, ou encore R un radical alcoyle substitué notamment benzyle ou OR représente un groupe réactif tel qu'un halogénure et

M, un groupement bloquant la fonction acide, des motifs disaccharidiques préférés répondant aux formules suivantes :

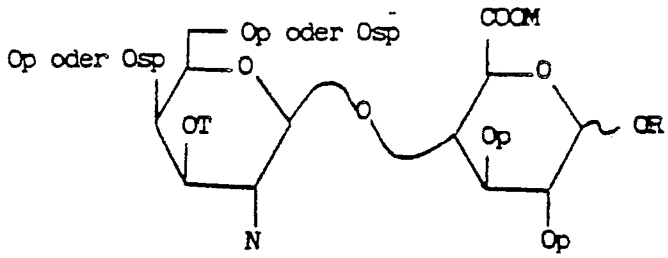
(II)

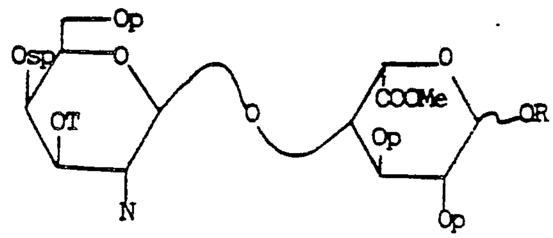
(III)

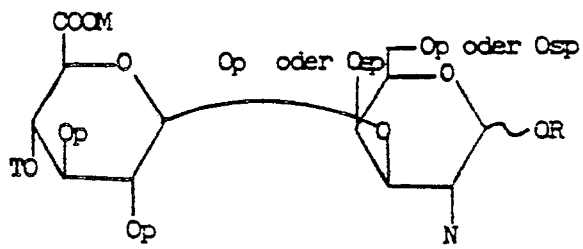
(IV)

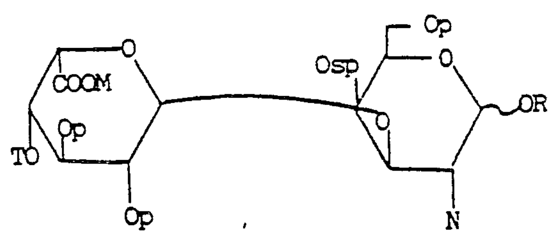
(V)

dans lesquelles :

M représente un atome d'hydrogène ou un radical alcoyle, en particulier méthyle,

sp un groupe acyle, en particulier acétyle,

p, un groupe alcoyle substitué, en particulier benzyle,

R, un groupe acyle, en particulier un groupe acétyle, un radical alcoyle, en particulier méthyle ou alcoyle substitué, notamment benzyle, ou

OR un halogénure, en particulier un bromure, ou encore un radical imidoyle,

N, un groupe azido ou phtalimidoyle,

T, peut représenter soit le groupe t représentant un radical acyle, en particulier acétyle, un radical acyle halogéné, en particulier, un radical monochloro ou trichloroacétyle, soit le groupe p représentant un radical alcoyle substitué, en particulier le radical benzyle, le cas échéant lui-même paraméthoxy ou encore un atome d'hydrogène.

20. Oligosaccharides intermédiaires selon la revendication 17, correspondant aux produits dont les groupes protecteurs ont été partiellement éliminés en cours de synthèse, ces produits comportant, en particulier, un groupe —OH à la place des groupes sp.

21. Oligosaccharides intermédiaires comprenant

une structure trisaccharidique, en particulier correspondant à l'une des formules (VI) à (IX)

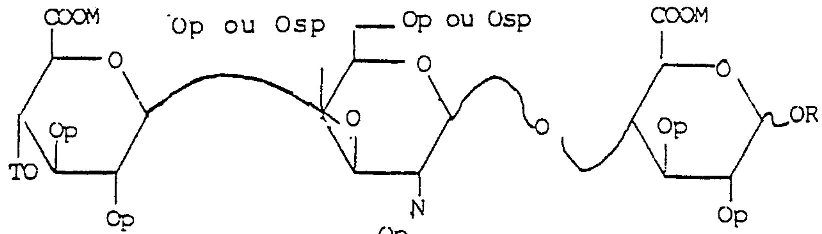

(VI)

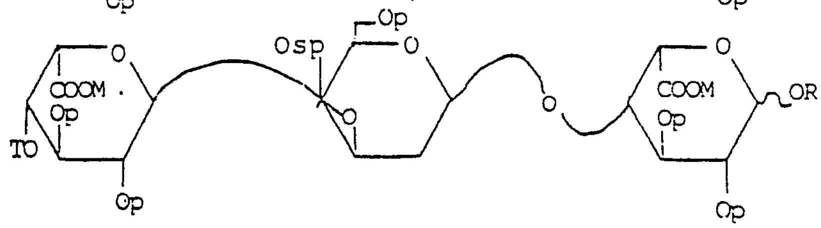

(VII)

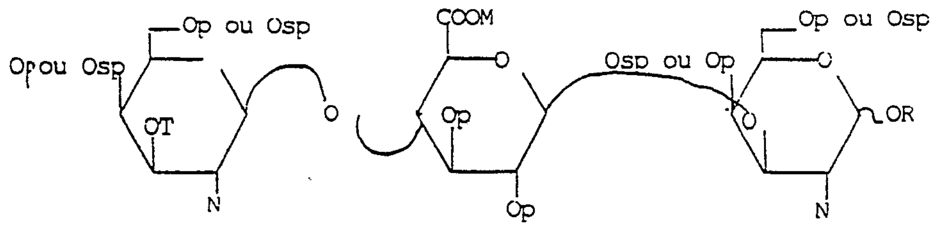

(VIII)

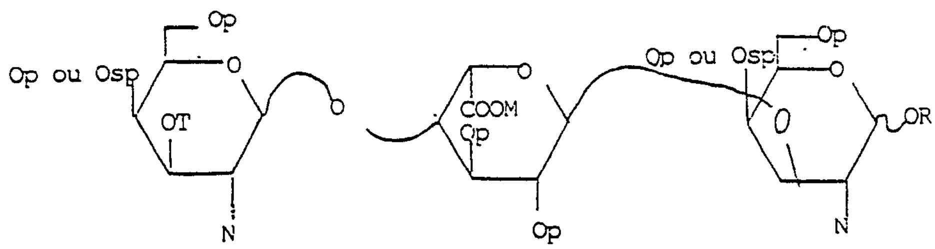

(IX)

dans lesquelles les substituants présentent les significations données dans la revendication 19, ou

une structure tétrasaccharidique, en particulier la structure (X) répondant à la formule suivante :

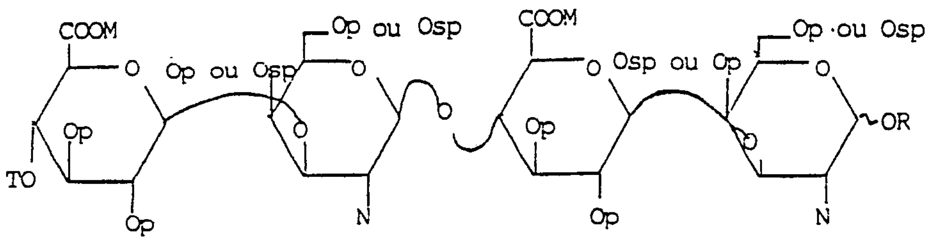

(X)

dans laquelle les différents symboles présentent les significations ci-dessus.

22. Oligosaccharides correspondant aux produits selon l'une quelconque des revendications 17 à 21, mais dans lesquels un, plusieurs ou tous les groupes —OH sont libérés au cours du procédé de synthèse et/ou comprenant un ou plusieurs groupes fonctionnels, ces groupements fonctionnels étant constitués, de préférence, par des esters, et se présentant plus spécialement sous forme d'anions minéraux, en particulier des esters sulfates ou des esters phosphates, ces groupes fonctionnels étant portés par une ou plusieurs fonctions alcool primaire et/ou alcool secondaire et/ou amine primaire.

23. Oligosaccharides selon la revendication 22, caractérisés en ce qu'ils comportent

des motifs x substitués en position 6 et/ou 3 par des esters, avantageusement sous forme de sels avec un cation minéral ou organique, en particulier un cation métallique, notamment un cation alcalin, notamment du sodium, ou encore un cation dérivé d'une base organique azotée, par exemple, du triéthylammonium, ledits motifs x comportant également de préférence en position 2 un groupe fonctionnel amino primaire, avantageusement substitué par un groupe tel qu'un sulfate ou un groupe acyle, en particulier acétyle, ces oligosaccharides comportant en outre de préférence :

des motifs y ou z avec des groupes carboxyle libres ou sous forme de sels avec un cation minéral ou organique tel que défini ci-dessus, ou encore protégé comme indiqué ci-dessus, et plus spécialement comportant des motifs z avec un groupe sulfate en position 2 et/ou des groupes sulfate sur les motifs y, les fonctions hydroxyle des cycles pyraniques de ces motifs x, y, ou z étant soit libres, soit protégées par des groupements permanents de type alcoyle, en particulier par des groupes méthyle.

24. Oligosaccharides selon la revendication 22, correspondant aux produits de formule I à X mais

dans lesquels les groupes —sp sont remplacés par des anions et de préférence renferment des groupes —NH-acyl- en particulier, —NH-acétyle, ou des groupes —$NHSO_3^-$ sur les motifs galactosamine.

25. Oligosaccharides selon l'une des revendications 17 à 24, mais renfermant des groupes : —OH libres à la place des groupes —Op.

26. Utilisation des oligosaccharides selon l'une quelconque des revendications 23 à 25 comme réactifs biologiques et/ou composés de référence.

27. Compositions pharmaceutiques, caractérisées en ce qu'elles renferment une quantité efficace d'un oligosaccharide biologiquement actif selon la revendication 24, en association avec un véhicule inerte.

28. Compositions pharmaceutiques selon la revendication 27, caractérisées en ce que le véhicule inerte est approprié pour l'administration par voie orale, et qu'elles se présentent sous forme de gélules gastrorésistantes, des comprimés ou tablettes, des pilules, ou encore sous forme de liposomes ou de solutions buvables, renfermant avantageusement de 50 mg à 5 g par unité de poids, de préference 100 à 250 mg pour des gélules, tablettes et pilules et de 1 à 5 g pour les solutés buvables.

29. Compositions pharmaceutiques selon la revendication 27, caractérisées en ce que les oligosaccharides sont associés à des excipients appropriés pour l'administration par voie rectale et se présentent sous forme de suppositoires ou encore elles se présentent sous des formes appropriées par l'administration par voie topique et sont constituées par des aérosols ou des pommades.

30. Compositions pharmaceutiques selon la revendication 27, caractérisées en ce qu'elles se présentent sous forme de solution injectable, stérile ou stérilisable, pour l'administration par voie intraveineuse intramusculaire ou sous-cutanée, ces solutions renfermant avantageusement 50 à 250 mg/ml d'oligosaccharides, de préférence 100 à 150, par exemple de 150 mg par ml, lorsque ces solutions sont destinées à l'injection par voie sous-cutanée ou qu'elles contiennent de 25 à 250, notamment de 150 mg par ml d'oligosaccharides, lorsqu'elles sont destinées à l'injection par voie intraveineuse ou par perfusion, ou en variante elles se présentent sous forme de soluté buvable renfermant avantageusement de 500 mg à 5 g de produits actifs.

**Claims**

1. A process for the organic synthesis of oligosaccharides comprising [D-galactosamine]-[D-glucuronic acid or L-iduronic acid] enchainments, such as encountered in dermatane-sulfates, chondroitins, chondrosine or chondroitin-sulfates, which comprises :

using, in a glycosylation reaction, two products terminated or constituted respectively by a A unit of galactosamine structure, and a U unit of D-glucuronic or L-iduronic acid structure, one of the units A or U being an alcohol in which the —OH group of the alcohol function occupies any position 3 or 4, the other unit possessing an activated anomeric carbon, substituted by a reactive group compatible with the other groups present on the units, all the other positons of A and U, excepted the one the anomeric carbon of which is activated and the one occupied by the OH group of the alcoholic function, bearing either amino or carboxyl groups, or precursors of such groups, or —OH groups, said groups occupying determined positions, the amino and carboxyl groups when they are present, being respectively blocked by protective groups of amino and carboxyl function, the —OH groups being blocked by at least two types of protective groups, sequentially removable enabling desired groups on given positions to be introduced, and then —OH groups to be liberated on other positons, said glycosylation reaction giving :

either a 1,3 linkage with an alpha stereospecificity, between an L-iduronic acid and a D-galactosamine, or a 1,3 linkage with a beta stereospecificity between a D-glucuronic acid unit and a D-galactosamine unit,

a 1,4 linkage with a beta stereospecificity, between a D-galactosamine unit and a D-glucuronic acid or L-iduronic acid unit,

optionally repeating the glycosylation step to elongate the oligosaccharidic chain,

sequencially eliminating the —OH protective groups by introducing desired substitution groups on specific positions, then liberating the —OH groups on other specific positions as well as the amino and carboxyl groups.

2. A process according to claim 1, wherein the products with A and U units comprise several types of —OH protective groups, namely (1) one or more semi-permanent groups, i. e. groups removable in the first place after the glycosylation reactions when the carbohydrate skeleton contains the desired number of residues without removing or modifying other groups present, and (2) one or more permanent groups, i. e. groups which are able to maintain the —OH radicals in a protected form during the introduction of functional groups in the place of the semi-permanent groups, said protective groups being chosen between radicals such as acyl, alkyl, optionally substitutes alkyl or aryl.

3. A process according to claim 1 or 2, wherein the —OH protective groups are selected from the group comprising acyl radicals, particularly acetyl, alkyl, substituted alkyl such as benzyl, and for two neibourghing positions, acetals or ketals, for example benzylidene, another protective form consisting in blocking two —OH groups under epoxy and/or 1,6-anhydro bridge form.

4. A process according to any one of the preceding claims, wherein the products used in the

glycosylation reaction contain several types of protective groups, the protective groups may already being occupying certain positions on the products applied in the glycosylation reaction or alternatively being introduced from other groups once the glucide skeleton is constituted, this modification comprising, for example, the use for glycosylation of a substance A in which the —OH groups at the 2 and 3 positions and at the 1 and 6 positions are blocked in anhydrous form, respectively 2,3-exposide and 1,6-anhydro, the opening of the epoxide function by the sodium azide enabling the introduction, at the 2 position, of an $N_3$ group which hence constitutes a precursor of an amine function.

5. A process according to any one of the preceding claims, wherein the —OH radicals of the starting materials intended to be sulphated are protected by acyl groups, in particular acetyl, whilst the —OH radicals intended to be liberated at the end of the synthesis are protected by a permanent group such as the benzyl group.

6. A process according to any one of claims 1 to 5, wherein A comprises at the 2 position a nitrogen group advantageously constituted by groups such as $—N_3$ or —N-phthalimido, or any other group constituting a precursor of the amine function or of an amine derivative, in particular of $—NHSO_3^-$ or of —NH-acyl, more especially $—NH—COCH_3$.

7. A process according to any one of claims 1 to 6, wherein the carboxyl functions or the U units, are blocked by groups inert with respect to reactions used for the replacement of the protective groups and removable at the end of the synthesis to liberate the carboxyl groups, possibly for the purposes of salt formation, these protective groups of carboxyl function being selected advantageously from among alkyl radicals or aryl radicals.

8. A process according to claim 1, wherein the A and U units of the sequence formed include temporary protective groups, that is to say groups capable of selectively blocking a position of the A or U unit intended to take part in a novel glycosylation reaction, these groups being removable in the presence of the other groups present on the units of the starting products by recreating an alcohol.

9. A process according to claim 8, wherein for a U-A disaccharide lengthening towards the right, the temporary group is present on the U unit and for lengthening to the left on the A unit, enabling the obtention as desired, by successively carrying out glycosylation reaction of enchainment $U_w A_x U_y A_z$ in which the sum of the indices is comprised between 2 and 12, these values being included in the range, where w and y cannot be nul simultaneously, regular enchainments being of the type U $(AU)_n$, $(AU)_n$ A, $(UA)_n$ or again (AU) with n equal to 1 to 6.

10. A process according to any one of claims 1 to 9, wherein the alcohol function of one of the units A or U involved in the glucide sequence already constituted is advantageously liberated from its temporary protective group, for example :

from an allyl group, by a treatment such as one comprising first the use of an isomerising agent such as Pd, Rh and Ir derivatives, in particular rhodium tris-triphenyl-phosphine chloride (I), or again potassium tertio-butoxide, then under acid conditions, in particular with a mixture of mercuric oxide and mercuric chloride, or

by saponification from an —O-acyl group, in particular —O-acetyl, O-chloracetyl or —O-levulinoyl, these radicals being romovable, for example, by means of thiourea in a solvent medium, advantageously at a temperature higher than 80 °C, preferably of the order of 100 °C.

11. A process according to any one of claims 1 to 10, wherein the above alcohol is reacted with a reactive derivative such as a halogenide, advantageously a chloride or a bromide, an imidate or an orthoester,

the condensation reaction between the halide and the alcohol being advantageously of the Koenings-Knorr type and carried out in a solvent medium, more especially in an organic solvent, particularly of the dichloromethane or dichloroethane type, advantageously in the presence of a catalyst generally a silver or mercury salt, for example, silver trifluoromethane sulphonate, commonly called silver triflate, silver carbonate, silver oxide, mercuric bromide or mercuric cyanide, and also with a proton acceptor such as sym-collidine and an extractor for the water possibly present and/or for the halohydric acid formed, for example 4 A molecular sieves, at room temperature or again at a lower temperature which can reach 0 °C or less, in an atmosphere of an inert gas such as nitrogen or argon, or alternatively for forming covalent bonds between alcohols of various structures and an L-idose precursor of the L-iduronic acid, the condensation reaction is carried out by using as catalyst mercuric derivatives, in particular cyanide and/or mercuric bromide, molecular sieves particularly 4 A a molecular sieves, in an organic solvent selected according to the reactivity of the alcohol,

the condensation with an orthoester such as 1,2-O-methoxy-ethylidene group being preferably carried out at a temperature above 100 °C in a solvent medium of the chlorobenzene type or any other solvent whose boiling point exceeds 100 °C and is advantageously between 100 and 150 °C in the presence of a catalyst such as 2,6-dimethyl pyridinium perchlorate,

the condensation with an imidate being carried out at low temperature, more especially at a temperature below or equal to about 0 °C, in a solvent medium, such as dichloromepthane, in the presence of a 4 A molecular sieve and a catalyst such as boron trifluoride etherate.

12. A process according to claim 1, wherein the glucide chain formed is subjected to one or more chemical reactions in order to introduce a type of given functional groups or, successively, several types of group and then to form, if desired, derivatives of said functional groups.

13. A process according to claim 12, wherein the functionalisation step is effected by eliminating only certain protective groups and/or certain precursor groups of the amino derivatives or again the whole of the protective groups and/or of the precursor groups and by introducing in their place a given type of substituent or successively different substituents, then by releasing a portion or all of the —OH groups still blocked, if desired.

14. A process according to claim 12 or 13 wherein the functionalization step is selectively carried out to successively introduce on the chain, several types of substituents, particularly sulfate groups, on determined positions on the units, to form on position 2 of A units, an amine derivative and on position 6 of U units, an acid derivative, the —OH functions being then liberated on other positions, said functionalization step being carried out by using derivatives in which the semi-permanent groups occupy positions intended to be sulphated and are constituted by —O-acetyl groups, the positions corresponding to an —OH group intended to be liberated being occupied by permanent groups constituted by benzyl groups and the position 2 of A units are substituted by groups such as $N_3$ OR NH—COO—$CH_2$—$C_6H_5$ and the positions 6 of U units are occupied by carboxyl groups protected by an alkyl radical, particularly methyl.

15. A process according to claim 14, wherein the functionalisation step comprises :

the selective introduction of the sulphate groups after having eliminated the —O-acetyl blocking group by a saponification reaction carried out by means of a strong base such as soda, preferably at a temperature below ambient temperature and more especially close to 0 °C, the product resulting from the hydrolysis being then subjected to the action of an alkylation agent in order to introduce, on the carboxyl group, the protected alkyl groups which are found to be removed on hydrolysis, said alkylation being followed by a sulphation treatment to introduce sulphate groups at the positions released by hydrolysis and left free after the action of the alkylation agent, said sulphation comprising the utilisation of a sulphation agent, such as a trimethylamine/$SO_3$-complex, in a solvent medium, more especially in a solvent such as dimethylformamide, preferably at a temperature higher than room temperature, generally in the vicinity of 50 °C, which corresponds to a reaction time of about 12 hours,

the liberation of the —OH groups blocked by the benzyl radicals, by removal of the benzyl groups, by catalytic hydrogenation under conditions compatible with the maintenance of the sulphate groups and the conversion of the nitrogenous groups into amino functional groups, preferably under hydrogen pressure in the presence of a catalyst of the Pd/C type, in an organic solvent medium, in particular alcoholic, with water

the formation of N-acetyl group by submitting the product resulting from the hydrogenation reaction to an acetylation agent such as acetic anhydride, the reaction being advantageously carried out at a basic pH, in particular close to 8 in an aqueous medium, or of N-sulphate group by means of a sulphation agent of the above-indicated type, at a pH higher than 9, advantageously of the order of 9-10

the liberation of the carboxyl group by addition of a strong base

the salification of the carboxyl group by using for example exchange resins with the desired cation, particularly with sodium, potassium, lithium, magnesium, calcium.

16. A process according to any one of the preceding claims, comprising the use in place of one or several A or U units of a sugar constituting a structural analog of an A or U unit, such as a neutral sugar or a desoxy-sugar.

17. Oligosaccharides constituting intermediates in the process according to any one of the preceding claims, comprising a chain based on binary units of structure $(A\text{-}U)_n$ or $(U\text{-}A)_n$ corresponding to enchainments x-y, (or the reverse) x, y and z having respectively the formulae

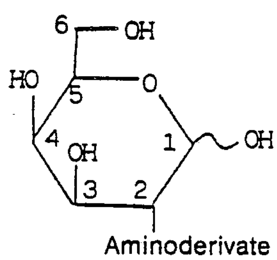

Aminoderivate

(x) D-galactosamine

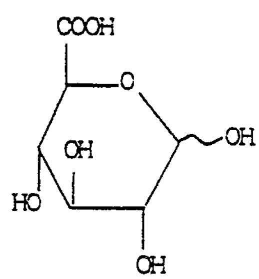

(y) D-glucuronic acid

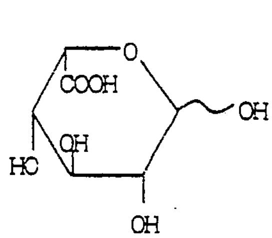

(z) L-iduronic acid

n being a number from 1 to 6, (1) said binary unit being completely protected an possessing either a reactive group on the anomeric carbon of the unit at the reducing end, or a single free —OH group on the unit at the non-reducing end, this —OH group occupying the 3, 4 or 6 position in the case of an A unit and the 2, 3 or 4 positions in the case of U units, or (2) being constituted by completely protected units such as obtained at the end of the glycosylation step, or (3) comprising products in which one or several —OH groups are liberated, said intermediate oligosaccharides optionally containing one or several consecutive x or again y units and/or one or several units of neutral sugars and/or several desoxy-sugars in their structure.

18. Oligosaccharides according to claim 17, possessing the structure of fragments of dermatane-sulfate, chondroitin-sulphates, chondroitins or chondrosine and comprising z1 $\xrightarrow{\alpha}$ 3x, x1 $\xrightarrow{\beta}$ 4y or z and y1 $\xrightarrow{\alpha}$ 3x linkages.

19. Oligosaccharides according to claim 18, including at least one disaccharidic unit having a structure of the type x1 $\xrightarrow{\beta}$ 4y of the formula I :

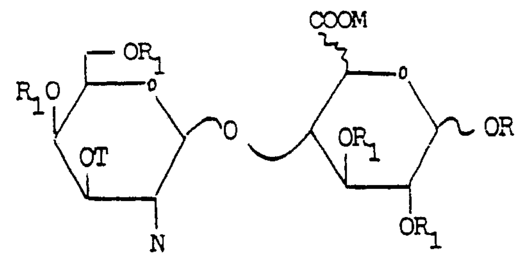

(I)

or one disaccharidic unit having a structure of the type z1 $\xrightarrow{\alpha}$ 3x ou y1 $\xrightarrow{\alpha}$ 3x of formula Ia :

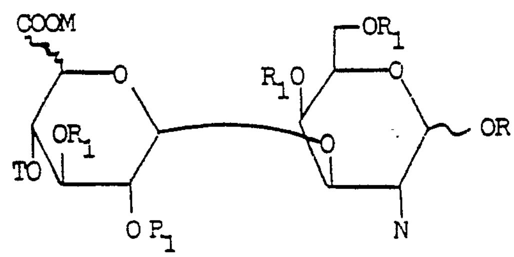

in which :

the $R_1$ radicals, identical or different from one another, if necessary conjointly with R, represent a protective group, in particular a sp semi-permanent group or a p permanent group,

T, a temporary group t, or a permanent group p, or a hydrogen atom,

N, is a nitrogenous group amine or amine derivative precursor,

R, an aliphatic or aromatic radical, particularly an alkyl radical comprising from 1 to 4 carbon atoms, or again R a substituted alkyl radical, particularly a benzyl group, or OR represents a reactive group such as a halide and

M, a group blocking the acid function, these various symbols having the above-given meanings, preferred disaccharidic units having the following formulae :

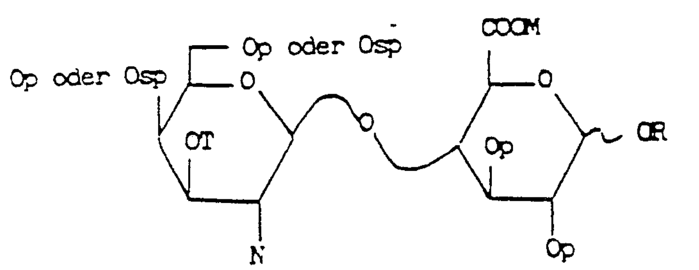

(II)

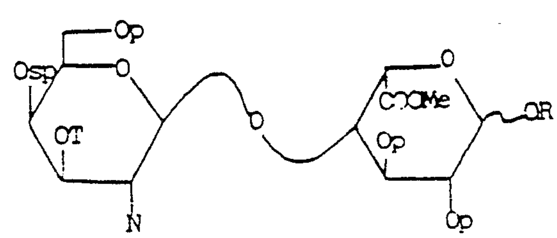

(III)

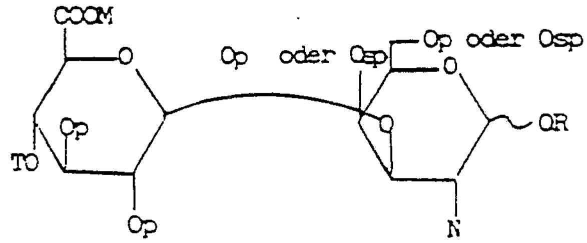

(IV)

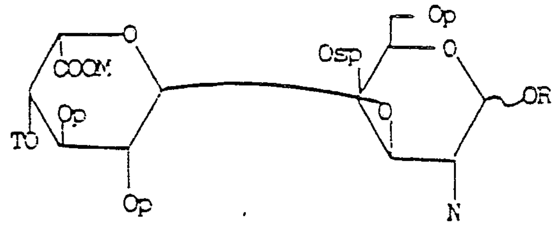

(V)

in which :

M represents a hydrogen atom or an alkyl radical, particularly methyl,

sp an acyl group, in particular acetyl,

p, a substituted alkyl group, in particular benzyl,

R, an acyl group at alpha or beta, in particular an acetyl group, an alkyl radical, in particular methyl or substituted alkyl, particularly benzyl, or

OR a halogenide, in particular a bromide, or again an imidoyl radical,

N, an azide or phthalimidoyl group,

T, the group t representing an acyl radical, in particular acetyl, a halogenated acyl radical, in particular, a monochloro or trichloroacetyl radical, or the group p representing a substituted alkyl radical in particular the benzyl radical, as the case may be itself paramethoxy or again a hydrogen atom in order of chain formation of said units being optionally reversed.

20. Intermediate oligosaccharides according to claim 17, corresponding to the products from which the protective groups have been partially removed in the course of synthesis, said products including in particular —OH groups in place of the sp groups.

21. Intermediate products comprising :

a trisaccharidic structure more especially corresponding to one of the formulae VI to IX :

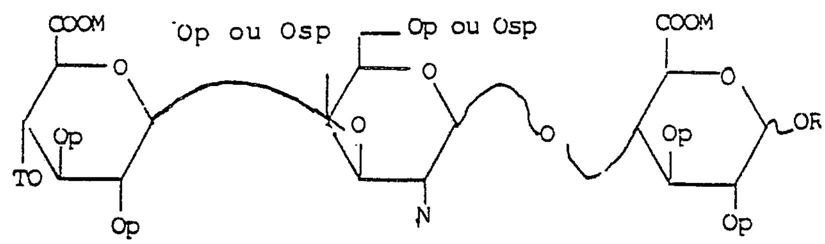

(VI)

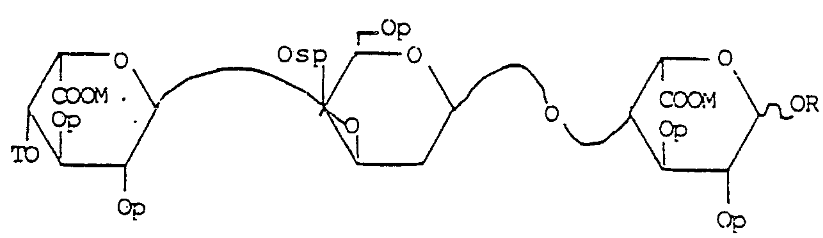

(VII)

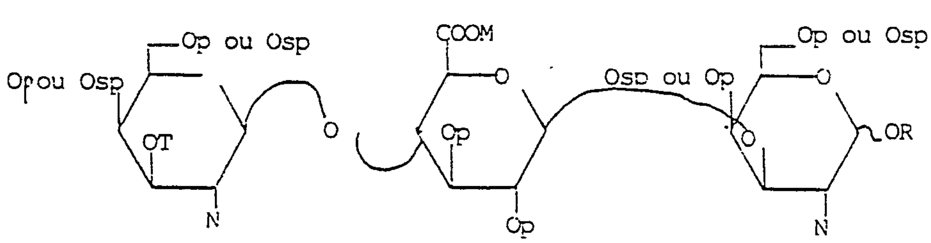

(VIII)

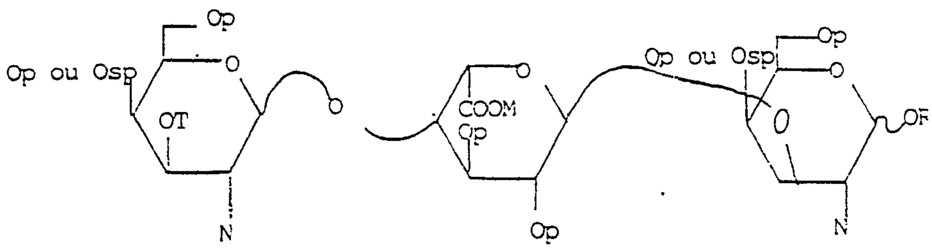

(IX)

wherein the substituents have the meanings given in claim 19,

a tetrasaccharidic structure, particularly the structure corresponding to the following formula (X) :

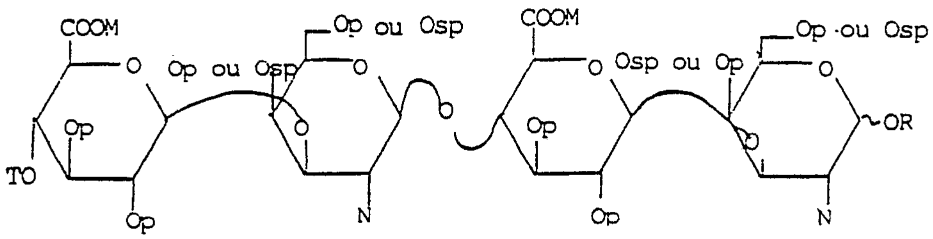

(X)

wherein the various substituents have the above meanings.

22. Oligosaccharides corresponding to the products according to any one of claims 17 to 21 but in which one, several or all the —OH groups are liberated in the course of synthesis and/or including one or several functional groups, said functional groups being constituted preferably by esters and occurring more especially in the form of inorganic anions, particularly by sulphate esters or phosphate esters, these functional groups being borne by one or several primary alcohol and/or secondary alcohol and/or primary amine functions.

23. Oligosaccharides according to claim 22, including :

x units substituted at the 6 and/or 3 positions by esters, advantageously in the form of salt with an inorganic or organic cation, in particular a metal cation, particularly an alkali cation especially sodium, or again a cation derived from a nitrogenous organic base, for example triethylammonium, said x units having also preferably at the 2 position primary amino functional group, advantageously substituted by a group such as a sulphate or an acyl group, particularly an acetyl group, and, further including preferably

units y or z with carboxyl groups free or in the form of salt with an organic or inorganic cation such as defined above, or again protected as above mentioned, and more preferably including units z comprising a sulphate group at the 2 position and/or sulphate groups on the y units, the hydroxyl functions of the pyran rings of said units x, y or z being either free, or protected by permanent groups of the alkyl type, in particular by methyl groups.

24. Oligosaccharides according to claim 22, corresponding to the products of formulae (I) to (X) but in which the —sp groups are replaced by anions, and preferably including NH-acyl groups, in particular —$NHCOCH_3$, or $NHSO_3^-$ groups on the galactosamine units.

25. A process according to any one of claims 17 to 24 but containing free —OH groups in place of the —Op groups.

26. The use of the oligosaccharides according to any one of claims 23 to 25 as biological reagents and/or reference products.

27. Pharmaceutical compositions comprising an effective amount of a biologically active oligosaccharide according to claim 24 in association with an inert carrier.

28. Pharmaceutical compositions according to claim 27, wherein the inert vehicle is suited for administration by the oral route and that they are under the form of gastroresistant capsules, pellets or tablets, pills, or again presented in liposome form or of drinkable solutions, containing advantageously from 50 mg to 5 g by weight unit, preferably 100 to 250 mg for capsules, tablets and pills and 1 to 5 g for drinkable solutions.

29. Pharmaceutical compositions according to claim 27, wherein the oligosaccharides are in association with suitable excipients for rectal administration, and are under the form of suppositories, or again under suited forms for topically administration and are constituted by aerosols or pommades.

30. Pharmaceutical compositions according to claim 27, characterized in that they are under the form of sterile or sterilizable injectable pharmaceutical compositions for administration both intravenously and intramuscularly or subcutaneously, said solutions containing advantageously 50 to 250 mg/ml of oligosaccharides, preferably from 100 to 150, for example of 150 mg/ml, when these solutions are intended for subcutaneous injections or they contain for example from 25 to 250, particularly 150 mg/ml,

of oligosaccharides when they are intended for injection intravenously or by perfusion, or alternatively they are under the form of drinkable solutions containing advantageously 500 mg to 5 g of active principles.

**Patentansprüche**

1. Verfahren zur organischen Synthese von Oligosacchariden mit [D-Galactosamin]-[D-glucuronsäure- oder L-iduronsäure-]-Ketten oder Ketten umgekehrten Aufbaues, wie man sie in Dermatan-Sulfat, den Chondroitinen, dem Chondrosin oder den Chondroitinsulfaten antrifft, dadurch gekennzeichnet, daß man

bei einer Glykosylierungsreaktion zwei Verbindungen, die einen endständigen Rest A mit D-Galactosaminstruktur und einen endständigen Rest U mit D-Glucuronsäurestruktur oder L-Iduronsäurestruktur tragen bzw. aus solchen Resten aufgebaut sind, wobei einer der Reste A oder U ein Alkohol ist, bei dem die Alkohol-OH-Gruppe in der 3- oder 4-Stellung steht, und der andere Rest ein aktiviertes anomeres Kohlenstoffatom aufweist, welches durch eine mit den anderen an den Resten vorhandenen Gruppen verträgliche, reaktive Gruppe substituiert ist, und sämtliche anderen Stellungen von A und U mit Ausnahme jener, deren anomeres Kohlenstoffatom aktiviert ist, und jener, die durch die Alkohol-OH-Gruppe besetzt ist, entweder Aminogruppen oder Carboxylgruppen oder Precursoren dieser Gruppen oder auch OH-Gruppen tragen, wobei diese Gruppen bestimmte Stellungen besetzen, etwa vorhandene Aminogruppen oder Carboxylgruppen durch Schutzgruppen für Aminogruppen bzw. Carboxylgruppen blockiert sind, die OH-Gruppen durch mindestens zwei Arten von Schutzgruppen blockiert sind, die nacheinander abgespalten werden können, und die Einführung von gewünschten Gruppen in bestimmte Stellungen und dann die Freisetzung der OH-Gruppen an anderen Stellungen ermöglichen, einsetzt, wobei diese Glykosylierungsreaktion :

entweder eine 1,3-Bindung mit α-Stereospezifität zwischen einem L-Iduronsäurerest und einem D-Galactosaminrest, oder eine 1,3-Bindung mit einer β-Stereospezifität zwischen einem D-Glucuronsäurerest und einem D-Galactosaminrest, oder eine 1,4-Bindung mit einer β-Stereospezifität zwischen einem D-Galactosaminrest und einem D-Glucuron- oder L-Iduronsäurerest ergibt,

daß man gegebenenfalls die Glykosylierungsmaßnahme wiederholt, um die Oligosaccharidkette zu verlängern,

und daß man die Schutzgruppen der OH-Gruppen unter Einführung der gewünschten Substituentengruppen an spezifischen Stellungen und dann unter Freisetzung der OH-Gruppen an anderen spezifischen Stellungen sowie der Aminogruppen und Carboxylgruppen nacheinander entfernt.

2. Verfahren nach Anspruch 1, dadurch gekennzeichnet, daß die eingesetzten Produkte mit den Resten A und U mehrere Arten von Schutzgruppen für OH-Reste aufweisen, d. h. (1) eine oder mehrere semipermanente Gruppen, nämlich Gruppen, die nach den Glykosylierungsreaktionen dann, wenn das Kohlenhydratgerüst die gewünschte Anzahl von Resten aufweist, zuerst und ohne Abspaltung oder Änderung von anderen vorhandenen Gruppen abgespalten werden können, und (2) eine oder mehrere permanente Gruppen, nämlich Gruppen, die den Schutz der OH-Reste während der Einführung von funktionellen Gruppen an der Stelle der semi-permanenten Gruppen ermöglichen, wobei diese Schutzgruppen aus Resten, wie Acylresten, Alkylresten, gegebenenfalls substituierten Alkylresten oder Arylresten, ausgewählt sind.

3. Verfahren nach Anspruch 1 oder 2, dadurch gekennzeichnet, daß die Schutzgruppen für die OH-Gruppen aus der Gruppe ausgewählt sind, die Acylreste, insbesondere den Acetylrest, Alkylreste, substituierte Alkylreste, wie den Benzylrest, und für zwei benachbarte Stellungen Acetal- und Ketal-Gruppen, beispielsweise Benzylidengruppen, umfaßt, wobei eine weitere Form des Schutzes darin besteht, eine Blockierung der beiden OH-Gruppen in Form eines Epoxids und/oder der 1,6-Anhydrobrücke zu bewirken.

4. Verfahren nach einem der vorhergehenden Ansprüche, dadurch gekennzeichnet, daß die bei der Glykosylierungsreaktion verwendeten Produkte mehrere Arten von Schutzgruppen aufweisen, wobei die Schutzgruppen bereits bestimmte Positionen an den für die Glykosylierungsreaktion eingesetzten Produkten besetzen, oder gemäß einer Variante, ausgehend von anderen Gruppen, nach Bildung des Kohlenhydratgerüstes, eingeführt werden, wobei diese Variante beispielsweise darin besteht, bei der Glykosylierung ein Produkt A zu verwenden, bei dem die OH-Gruppen in den Stellungen 2 und 3 und in den Stellungen 1 und 6 durch Wasserabspaltung blockiert sind als 2,3-Epoxid bzw. 1,6-Anhydrid, wobei die Öffnung der Epoxidfunktion durch Natriumazid die Einführung einer $N_3$-Gruppe, die einen Precursor der Aminogruppe darstellt, in die 2-Stellung ermöglicht.

5. Verfahren nach einem der vorhergehenden Ansprüche, dadurch gekennzeichnet, daß die OH-Reste der Ausgangsprodukte, die sulfatiert werden sollen, mit Acylgruppen, insbesondere Acetylgruppen, geschützt sind, während die OH-Reste, die am Ende der Synthese freigesetzt werden sollen, durch eine permanente Gruppe, wie die Benzylgruppe, geschützt sind.

6. Verfahren nach einem der Ansprüche 1 bis 5, dadurch gekennzeichnet, daß A in der 2-Stellung eine stickstoffhaltige Gruppe, vorteilhafterweise eine Gruppe der Formeln $—N_3$ oder $—NHCOO—CH_2—C_6H_5$ oder irgendeine andere Gruppe, die einen Precursor für eine Aminogruppe oder

ein Derivat einer Aminogruppe darstellt, insbesondere eine Gruppe der Formel —$NHSO_3$— oder eine —NH-Acylgruppe, im spezielleren eine Gruppe der Formel —NH—$COCH_3$, enthält.

7. Verfahren nach einem der Ansprüche 1 bis 6, dadurch gekennzeichnet, daß die Carboxylfunktionen der Reste U durch Gruppen blockiert sind, die gegenüber den Reaktionen inert sind, die zum Ersatz der schützenden und abspaltbaren Gruppen am Ende der Synthese zwecks Freisetzung der Carboxylgruppen und gegebenenfalls zwecks Salzbildung durchgeführt werden, wobei diese Schutzgruppen der Carboxylfunktion vorteilhafterweise aus Alkylresten oder Arylresten ausgewählt sind.

8. Verfahren nach Anspruch 1, dadurch gekennzeichnet, daß die Reste A und U der gebildeten Sequenz temporäre Schutzgruppen aufweisen, d.s. Gruppen, die selektiv eine Stellung des Restes A oder U, an der eine erneute Glykosylierungsreaktion durchgeführt werden soll, blockieren können, wobei diese Gruppen in Gegenwart der anderen, an diesen Resten der Ausgangsmaterialien vorhandenen Gruppen unter Wiederbildung eines Alkohols abspaltbar sind.

9. Verfahren nach Anspruch 8, dadurch gekennzeichnet, daß man zur Verlängerung einer Disaccharideinheit U-A nach rechts einen Rest U, der eine temporäre Gruppe aufweist, und zur Verlängerung nach links einen Rest A, der die genannte temporäre Gruppe besitzt, einsetzt, so daß man durch aufeinanderfolgende Glykosylierungsreaktionen Ketten der Formel $U_wA_xU_yA_z$ bilden kann, worin die Summe der Indizes zwischen 2 und 12 liegt, und diese Werte in dem Intervall eingeschlossen sind, wobei w und y nicht gleichzeitig Null sein können, und die normalen Ketten folgende Typen umfassen : $U(AU)_n$, $(AU)_nA$, $(UA)_n$ oder auch $(AU)_n$, worin $1 \leq n \leq 6$ ist.

10. Verfahren nach einem der Ansprüche 1 bis 9, dadurch gekennzeichnet, daß die Alkoholfunktion eines der Reste A oder U, der in der bereits gebildeten Kohlenhydratsequenz vorhanden ist, mit Vorteil aus ihrer temporären Schutzgruppe freigesetzt wird, beispielsweise

ausgehend von einer Allylgruppe durch eine Art von Behandlung, die die Verwendung eines Isomerisierungsmittels, wie Derivate von Pd, Rh und Ir, insbesondere Tris-triphenyl-phosphin-rhodium(I)-chlorid, oder auch Kalium-tert.butoxid, und dann Säurebedingungen umfaßt, insbesondere mit einer Mischung aus Quecksilber(II)-oxid und Quecksilber(II)-chlorid, oder

durch Verseifung, ausgehend von einer O-Acylgruppe, insbesondere einer O-Acetylgruppe oder einer O-Chloracetylgruppe, wobei diese Reste zur Freisetzung einer OH-Funktion, beispielsweise mit Hilfe von Thioharnstoff in einem Lösungsmittelmedium, vorzugsweise bei einer Temperatur von über 80 °C, in bevorzugterem Maße von etwa 100 °C, abgespalten werden.

11. Verfahren nach einem der Ansprüche 1 bis 10, dadurch gekennzeichnet, daß man den genannten Alkohol mit einem reaktiven Derivat, wie einem Halogenid, vorzugsweise einem Chlorid oder einem Bromid, einem Imidat oder einem Orthoester, umsetzt, wobei

die Kondensationsreaktion zwischen dem Halogenid und dem Alkohol vorzugsweise eine Koenigs-Knorr-Kondensation ist, die in einem Lösungsmittelmedium und insbesondere einem organischen Lösungsmittel, namentlich vom Typus Dichlormethan oder Dichlorethan, durchgeführt wird, und zwar

vorzugsweise in Gegenwart eines Katalysators, im allgemeinen eines Silber- oder Quecksilber-Salzes, beispielsweise Silbertrifluormethansulfonat, im allgemeinen als Silbertriflat bezeichnet, Silbercarbonat, Silberoxid, Quecksilber(II)-bromid oder Quecksilber(II)-cyanid, und gleichermaßer in Gegenwart eines Protonenakzeptors, wie sym-Collidin, sowie eines Akzeptors für gegebenenfalls vorhandenes Wasser und/oder die gebildete Halogenwasserstoffsäure, beispielsweise in Gegenwart von 0,4 nm-Molekularsieben,

bei Raumtemperatur oder einer darunter liegenden Temperatur, die 0 °C oder weniger betragen kann, unter einer Inertgasatmosphäre, wie Stickstoff oder Argon,

oder gemäß einer Variante zur Bildung von kovalenten Bindungen zwischen den Alkoholen verschiedener Struktur und eines L-Iduronsäureprecursors, durch Kondensationsreaktion unter Verwendung von Quecksilber(II)-derivaten als Katalysator, insbesondere von Quecksilber(II)-cyanid und Quecksilber(II)-bromid, eines Molekularsiebes, insbesondere eines 0,4 nm-Molekularsiebes, in einem organischen Lösungsmittel, welches in Abhängigkeit von der Reaktivität des Alkohols ausgewählt wird,

wobei die Kondensation mit einem Orthoester, wie einer 1,2,O-Methoxy-ethyliden-Gruppe, vorzugsweise bei einer Temperatur von über 100 °C in einem Lösungsmittelmedium vom Typus des Chlorbenzols oder eines analogen Lösungsmittels mit einem Siedepunkt von über 100 °C, und vorzugsweise zwischen 100° und 150 °C, in Gegenwart eines Katalysators, wie 2,6-Dimethylpyridiniumperchlorat, bewirkt wird, und

die Kondensation mit einem Imidat bei einer niedrigen Temperatur, insbesondere bei einer Temperatur unter oder gleich etwa 0 °C, in einem Lösungsmittelmedium, wie Dichlormethan, in Gegenwart eines 0,4 nm-Molekularsiebes und eines Katalysators, wie Bortrifluorid-etherat, durchgeführt wird.

12. Verfahren nach Anspruch 1, dadurch gekennzeichnet, daß die gebildete Kohlenhydratkette einer oder mehreren chemischen Reaktionen unterworfen wird, um einen gegebenen Typus von funktioneller Gruppe oder nacheinander mehrere Typen von Gruppen einzuführen, und gewünschtenfalls dann Derivate dieser funktionellen Gruppen zu bilden.

13. Verfahren nach Anspruch 12, dadurch gekennzeichnet, daß die Stufe der Bildung der funktionellen Gruppen in der Weise durchgeführt wird, daß nur bestimmte Schutzgruppen und/oder bestimmte Precursorgruppen der Aminoderivate oder auch die Gesamtheit der Schutzgruppen und/oder der

Precursorgruppen abgespalten werden und an ihrer Stelle ein gegebener Typus von Substituent oder nacheinander verschiedene Substituenten eingeführt werden, und schließlich gewünschtenfalls ein Teil oder die Gesamtheit der noch blockierten OH-Gruppen freigesetzt wird.

14. Verfahren nach Anspruch 12 oder 13, dadurch gekennzeichnet, daß die Stufe der Einführung der funktionellen Gruppen selektiv in der Weise erfolgt, daß nacheinander mehrere Arten von Substituenten, insbesondere Sulfatgruppen, in die Kette, und zwar an bestimmten Stellungen der Reste A und U, eingeführt werden, um in der 2-Stellung der Reste A ein Aminoderivat, und in der 6-Stellung der Reste U ein Säurederivat zu bilden, worauf die OH-Funktionen an den anderen Stellungen freigesetzt werden, wobei diese Stufe der Bildung der funktionellen Gruppen in der Weise durchgeführt wird, daß Derivate verwendet werden, in denen die semi-permanenten Gruppen jene Stellungen besetzen, die sulfatiert werden sollen und durch O-Acetylgruppen gebildet werden, und in denen diejenigen Stellungen, die einer OH-Gruppe entsprechen und die freigesetzt werden sollen, durch permanente Gruppen besetzt sind, die durch Benzylgruppen gebildet sind, und die 2-Stellungen der Reste A durch Gruppen, wie solche der Formeln $-N_3$ oder $-NH-COO-CH_2-C_6H_5$, substituiert sind, und die 6-Stellungen der Reste U durch Carboxylgruppen besetzt sind, die durch einen Alkylrest, insbesondere einen Methylrest, geschützt sind.

15. Verfahren nach Anspruch 14, dadurch gekennzeichnet, daß die Stufe der Bildung der funktionellen Gruppen die folgenden Maßnahmen umfaßt

die selektive Einführung von Sulfatgruppen nach der Abspaltung der O-Acetyl-Blockierungsgruppen mit Hilfe einer Verseifungsreaktion, die mit einer starken Base, wie Natriumhydroxid, vorzugsweise bei einer Temperatur unter Raumtemperatur und insbesondere in der Nähe von 0 °C, durchgeführt wird, worauf das bei der Hydrolyse gebildete Produkt der Einwirkung eines Alkylierungsmittels unterworfen wird, um an der Carboxylgruppe Alkyl-Schutzgruppen einzuführen, die im Verlaufe der Hydrolyse abgespalten worden sind, wobei diese Alkylierung gefolgt wird von einer Sulfatierungsbehandlung zur Einführung von Sulfatgruppen an den durch die Hydrolyse freigesetzten Stellungen und den nach der Einwirkung des Alkylierungsmittels freigelassenen Stellungen, wobei die Sulfatierung die Anwendung eines Sulfatierungsmittels, wie eines Trimethylamin/$SO_3$-Komplexes, in einem Lösungsmittelmedium, insbesondere in einem Lösungsmittel, wie Dimethylformamid, vorzugsweise bei einer Temperatur über Raumtemperatur und im allgemeinen in der Nähe von 50 °C, was einer Reaktionsdauer von etwa 12 Stunden entspricht, umfaßt ;

die Freisetzung der durch Benzylreste blockierten OH-Gruppen durch Abspaltung der Benzylreste, vorteilhafterweise durch katalytische Hydrierung unter Bedingungen, die mit der Beibehaltung der Sulfatgruppen und der Umwandlung der stickstoffhaltigen Gruppen in funktionelle Aminogruppen verträglich sind, vorzugsweise unter Wasserstoffdruck in Gegenwart eines Katalysators des Typus Pd/C, in einem organischen Lösungsmittelmedium, insbesondere einem alkoholischen, mit Wasser versetzten Lösungsmittelmedium,

die Bildung von N-Acetylgruppen durch Behandlung des bei der Hydrierungsreaktion gebildeten Produktes mit einem Acetylierungsmittel, wie Essigsäureanhydrid, wobei diese Reaktion vorzugsweise bei einem basischen pH-Wert, insbesondere in der Nähe von 8, in einem wässerigen Medium durchgeführt wird ; oder die Bildung von N-Sulfatgruppen mit Hilfe eines Sulfatierungsmittels des oben angegebenen Typus, bei einem pH-Wert von über 9, vorzugsweise im Bereich von 9 bis 10,

die Freisetzung der Carboxylgruppen durch Zugabe einer starken Base,

die Salzbildung an den Carboxylgruppen unter Verwendung von beispielsweise Ionenaustauscherharzen, die das gewünschte Kation, insbesondere Natrium oder auch Kalium, Lithium, Magnesium oder Calcium, tragen.

16. Verfahren nach einem der vorhergehenden Ansprüche, dadurch gekennzeichnet, daß man anstelle eines oder mehrerer Reste A oder U einen Zucker einsetzt, der ein Strukturanalogon des Restes A oder U darstellt, wie einen neutralen Zucker oder einen Desoxy-Zucker.

17. Oligosaccharide, welche Zwischenprodukte bei dem Verfahren nach einem der vorhergehenden Ansprüche darstellen, dadurch gekennzeichnet, daß sie eine Kette auf der Grundlage von binären Resten der Struktur $(A\text{-}U)_n$ oder $(U\text{-}A)_n$, die den Verkettungen x-y oder x-z (oder umgekehrt) entsprechen, enthalten, wobei x, y und z den Strukturen :

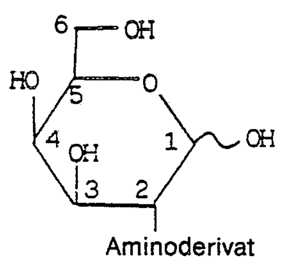

Aminoderivat

(x) D-Galactosamin

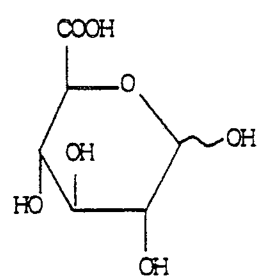

(y) D-Glucuronsäure

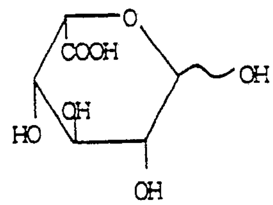

(z) L-Iduronsäure

entsprechen, worin n eine Zahl von 1 bis 6 darstellt, wobei diese binären Reste (1) vollständig geschützt sind und entweder eine reaktive Gruppe am anomeren Kohlenstoffatom des Restes am reduzierenden Ende oder eine einzige freie OH-Gruppe an dem Rest an dem nicht-reduzierenden Ende aufweisen, wobei diese OH-Gruppe die Stellung 3, 4 oder 6 im Falle eines Restes A und die Stellung 2, 3 oder 4 im Falle eines Reste U besetzt, oder (2) durch vollständig geschützte Reste gebildet sind, wie man sie am Ende der Glykosylierungsstufe erhält, oder (3) Produkte umfassen, bei denen eine oder mehrere OH-Gruppen freigesetzt sind, wobei diese Zwischenprodukt-Oligosaccharide gegebenenfalls einen oder mehrere aufeinanderfolgende Reste x, y oder z und/oder mehrere Reste von neutralen Zuckern und/oder mehrere Desoxy-Zucker in ihrer Struktur aufweisen.

18. Oligosaccharide nach Anspruch 17, dadurch gekennzeichnet, daß sie die Struktur von Dermatan-Sulfat-, Chondroitin-Sulfat-, Chondroitin- oder Chondrosin-Fragmenten besitzen und Bindungen z1 $\xrightarrow{\alpha}$ 3x, x1 $\xrightarrow{\beta}$ 4y oder z und y1 $\xrightarrow{\beta}$ 3x aufweisen.

19. Oligosaccharide nach Anspruch 18, dadurch gekennzeichnet, daß sie wenigstens einen Disaccharidrest mit einer Struktur des Typus x1 $\xrightarrow{\beta}$ 4y oder z, entsprechend der Formel I :

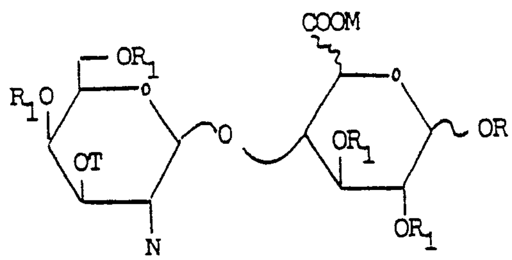

oder einen Disaccharidrest einer Struktur vom Typus z1 $\xrightarrow{\alpha}$ 3x oder y1 $\xrightarrow{\beta}$ 3x der Formel Ia :

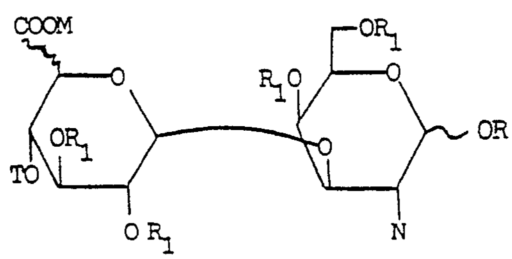

enthalten, worin :

die Reste $R_1$, die gleichartig oder voneinander verschieden sein können, gegebenenfalls zusammen mit R, eine Schutzgruppe, insbesondere eine semi-permanente Gruppe sp oder eine permanente Gruppe p,

T eine temporäre Gruppe t oder eine permanente Gruppe p oder ein Wasserstoffatom,

N eine Stickstoffhaltige Gruppe, die ein Precursor eines Amins oder eines Aminderivates ist,

R einen aliphatischen oder aromatischen Rest, insbesondere einen Alkylrest mit 1 bis 4 Kohlenstoffatomen, oder auch R eine substituierte Alkylgruppe, insbesondere eine Benzylgruppe oder OR eine reaktive Gruppe, wie ein Halogenid,

M eine die Säurefunktion blockierende Gruppe bedeuten, wobei die bevorzugten Disaccharidreste den folgenden Formeln entsprechen :

(Siehe Formeln Seite 42 f.)

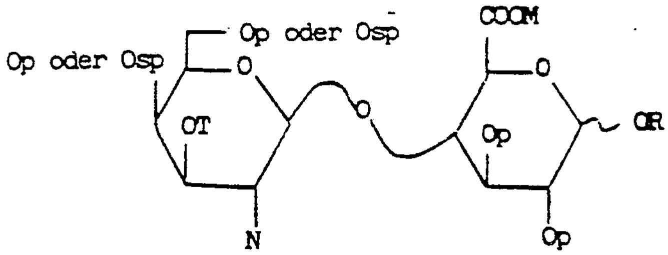

(II)

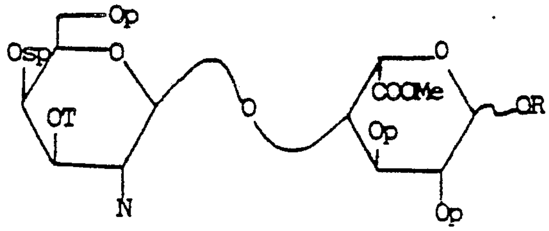

(III)

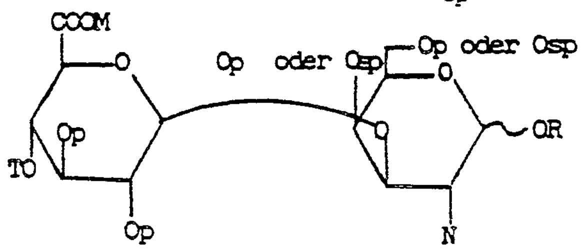

(IV)

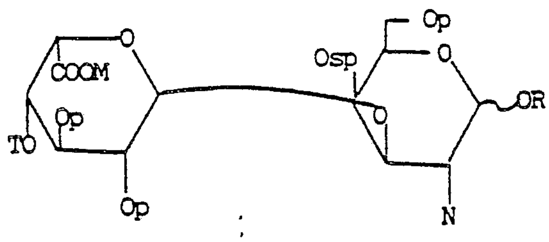

(V)

in welchen :

M für ein Wasserstoffatom oder eine Alkylgruppe, insbesondere eine Methylgruppe, steht,

sp für eine Acylgruppe, insbesondere eine Acetylgruppe, steht,

p für eine substituierte Alkylgruppe, insbesondere eine Benzylgruppe, steht,

R für eine Acylgruppe, insbesondere eine Acetylgruppe, eine Alkylgruppe, insbesondere eine Methylgruppe, oder eine substituierte Alkylgruppe, insbesondere eine Benzylgruppe, steht, oder

OR ein Halogenid, insbesondere ein Bromid, oder auch einen Imidoylrest bedeutet,

N für eine Azidogruppe oder Phthalimidoyl steht, und

T entweder die Gruppe t darstellen kann, die eine Acylgruppe, insbesondere eine Acetylgruppe, eine halogenierte Acylgruppe, insbesondere eine Monochlor- oder Trichloracetylgruppe, bedeutet, oder die Gruppe p darstellen kann, welche eine substituierte Alkylgruppe, insbesondere die Benzylgruppe, die gegebenenfalls p-methoxysubstituiert sein kann, oder auch ein Wasserstoffatom bedeutet.

20. Zwischenprodukt-Oligosaccharide nach Anspruch 17, entsprechend den Produkten, deren Schutzgruppen im Laufe der Synthese teilweise entfernt worden sind, wobei diese Produkte insbesondere eine —OH-Gruppe anstelle der sp-Gruppe enthalten.

21. Zwischenprodukt-Oligosaccharide, enthaltend eine Trisaccharidstruktur, insbesondere entsprechend einer der Formeln (VI) bis (IX)

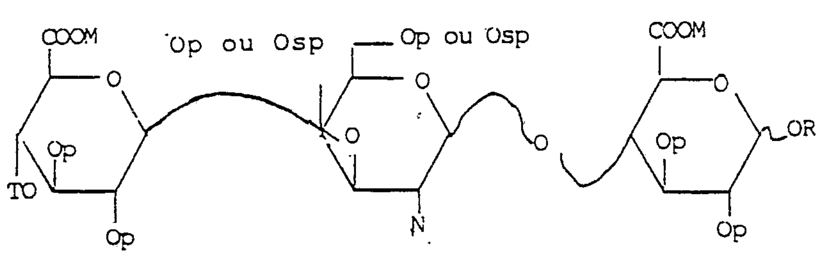

(VI)

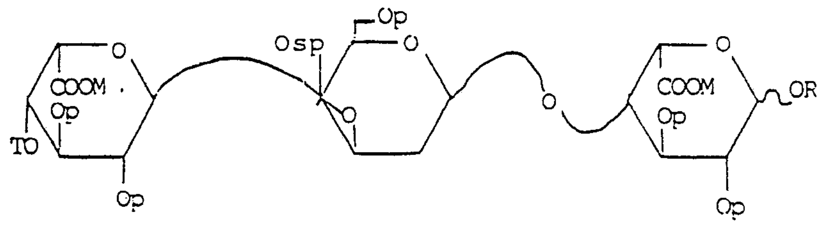

(VII)

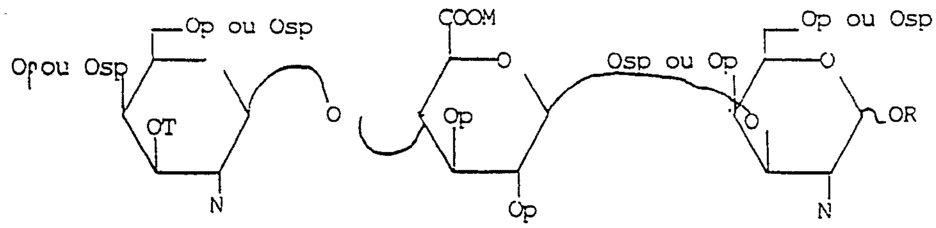

(VIII)

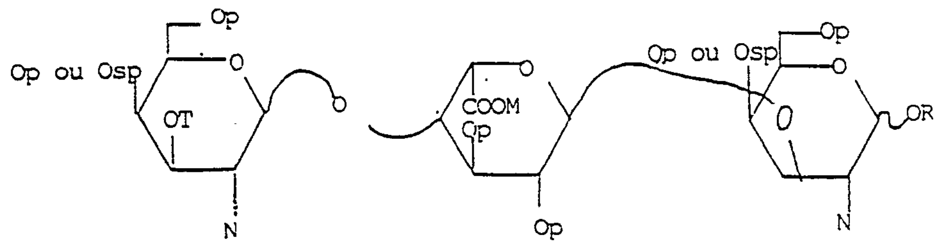

(IX)

in welchen die Substituenten die in Anspruch 19 angegebenen Bedeutungen haben, oder
eine Tetrasaccharidstruktur, insbesondere der Struktur (X) entsprechend der folgenden Formel :

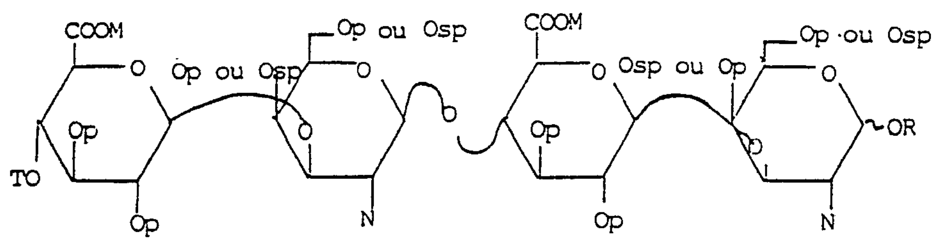

(X)

in welcher die verschiedenen Symbole die oben angegebenen Bedeutungen haben.

22. Oligosaccharide, entsprechend Produkten nach einem der Ansprüche 17 bis 21, bei denen jedoch eine, mehrere oder sämtliche OH-Gruppen im Verlaufe des Syntheseverfahrens freigesetzt worden sind, und/oder die eine oder mehrere funktionelle Gruppen aufweisen, wobei diese funktionellen Gruppen vorzugsweise durch Ester gebildet sind, und die insbesondere in Form von anorganischen Anionen, insbesondere von Sulfatestern oder Phosphatestern, vorliegen, wobei diese funktionellen Gruppen durch eine oder mehrere primäre Alkoholfunktionen und/oder sekundäre Alkoholfunktionen und/oder primäre Aminofunktionen getragen werden.

23. Oligosaccharide nach Anspruch 22, dadurch gekennzeichnet, daß sie
Reste x aufweisen, die in der 6- und/oder 3-Stellung durch Ester substituiert sind, vorzugsweise in Form von Salzen mit einem anorganischen oder organischen Kation, insbesondere einem Metallkation, namentlich einem Alkalikation, insbesondere einem von Natrium, oder auch einem von einer organischen, stickstoffhaltigen Base, abgeleiteten Kation, beispielsweise einem Triethylammoniumkation, welche Reste x weiterhin vorzugsweise in der 2-Stellung eine funktionelle, primäre Aminogruppe tragen, die vorteilhafterweise durch eine Gruppe, wie eine Sulfat- oder eine Acylgruppe, insbesondere eine Acetylgruppe, substituiert ist, welche Oligosaccharide weiterhin vorzugsweise :
Reste y oder z tragen, die Carboxylgruppen aufweisen, welche frei sind oder in Form von Salzen mit einem anorganischen oder organischen Kation, wie es oben definiert wurde, vorliegen, oder die, wie oben angegeben, geschützt sind, und insbesondere Reste z mit einer Sulfatgruppe in der 2-Stellung und/oder Sulfatgruppen an den Resten y aufweisen, wobei die Hydroxylgruppen der Pyranringe dieser Reste x, y oder z entweder frei sind oder durch permanente Gruppen des Typus Alkylgruppen, insbesondere durch Methylgruppen, geschützt sind.

24. Oligosaccharide nach Anspruch 22, entsprechend den Produkten der Formeln (I) bis (X), bei denen aber die Gruppen sp durch Anionen ersetzt sind, und welche vorzugsweise an den Galactosaminresten —NH-Acyl-Gruppen, insbesondere —NH-Acetyl-Gruppen, oder —$NHSO_3^-$-Gruppen, aufweisen.

25. Oligosaccharide nach einem der Ansprüche 17 bis 24, jedoch enthaltend freie OH-Gruppen anstelle der Gruppen -Op.

26. Verwendung der Oligosaccharide nach einem der Ansprüche 23 bis 25 als biologische Reagentien und/oder Referenzpräparate.

27. Pharmazeutische Zubereitungen, dadurch gekennzeichnet, daß sie eine wirksame Menge eines biologisch aktiven Oligosaccharids nach Anspruch 24 in Kombination mit einem inerten Trägermaterial enthalten.

28. Pharmazeutische Zubereitungen nach Anspruch 27, dadurch gekennzeichnet, daß das inerte Trägermaterial zur Verabreichung auf oralem Wege geeignet ist, und daß die Zubereitungen in Form von magenresistenten Geleepastillen, als Preßlinge oder Tabletten, als Pillen oder auch in Form von Liposomen oder von Trinklösungen vorliegen, die vorteilhafterweise 50 mg bis 5 g je Gewichtseinheit, vorzugsweise 100 bis 250 mg bei den Geleepastillen, Tabletten und Pillen, und 1 bis 5 g bei den Trinklösungen enthalten.

29. Pharmazeutische Zubereitungen nach Anspruch 27, dadurch gekennzeichnet, daß die Oligosaccharide mit Exzipienzien verbunden sind, die für die Verabreichung auf rektalem Wege geeignet sind, und in Form von Suppositorien vorliegen, oder auch in Form von zur topischen Verabreichung geeigneten Formen vorliegen und aus Aerosolen oder Salben bestehen.

30. Pharmazeutische Zubereitungen nach Anspruch 27, dadurch gekennzeichnet, daß sie in Form einer Sterilen oder sterilisierbaren Injektionslösung zur Verabreichung auf intravenösem, intramuskulären oder subkutanem Wege vorliegen, wobei diese Lösungen vorteilhafterweise 50 bis 250 mg/ml Oligosaccharide, vorzugsweise 100 bis 150, z. B. 150 mg je ml, enthalten, wenn diese Lösungen für die Injektion auf subkutanem Wege bestimmt sind, oder daß sie 25 bis 250, insbesondere 150, mg je ml Oligosaccharide enthalten, wenn sie zur Injektion auf intravenösem Wege oder durch Perfusion bestimmt sind, oder für den Fall, daß sie in Form von Trinklösungen vorliegen, vorzugsweise 500 mg bis 5 g der Wirkprodukte enthalten.

Fig. 1

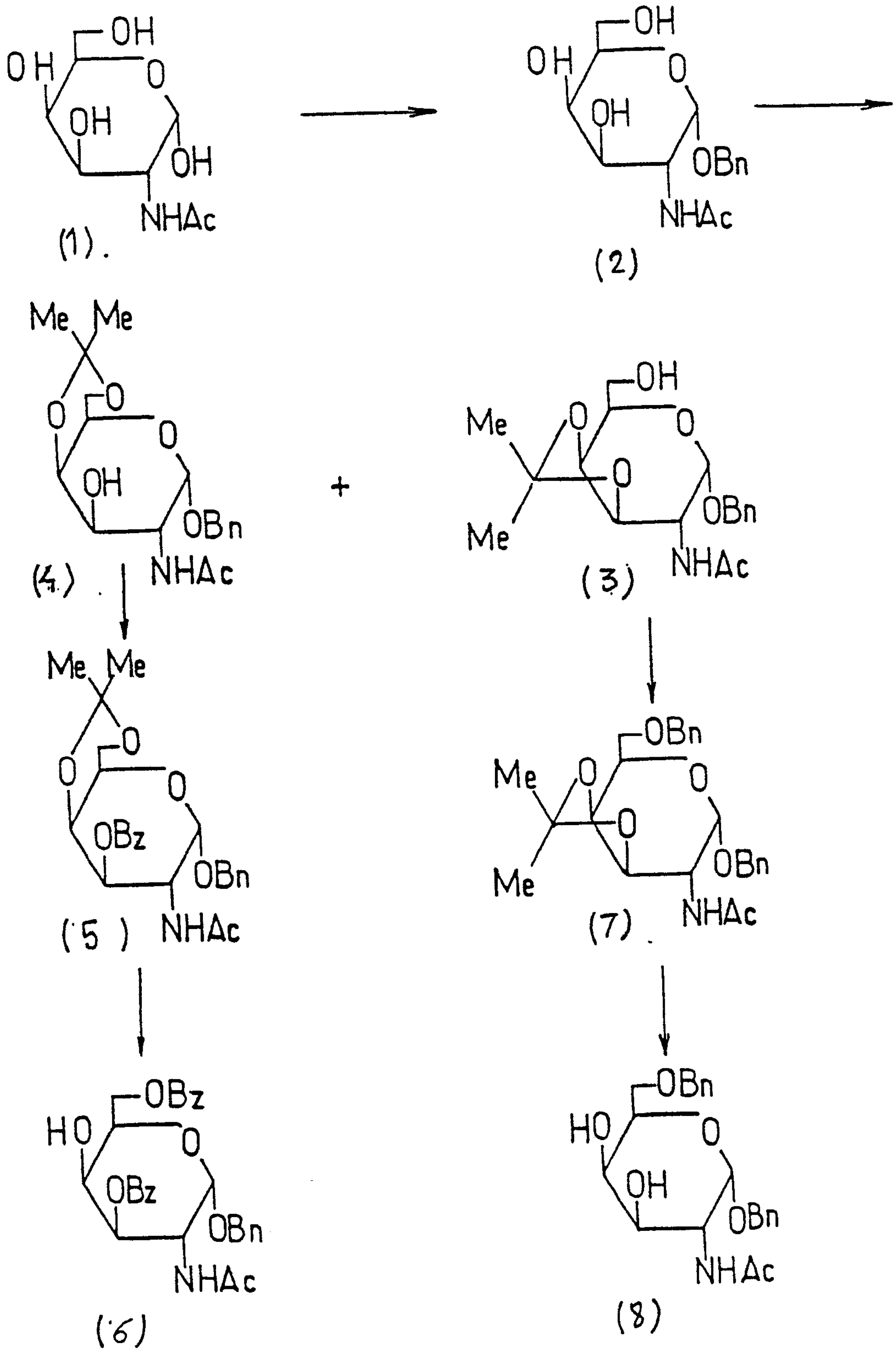

(1)

(2)

(4)

(3)

(5)

(7)

(6)

(8)

Fig. 2

(8) 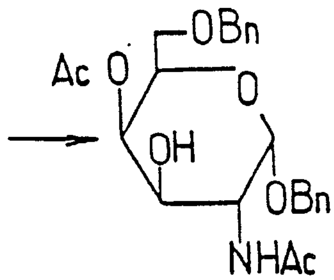 + 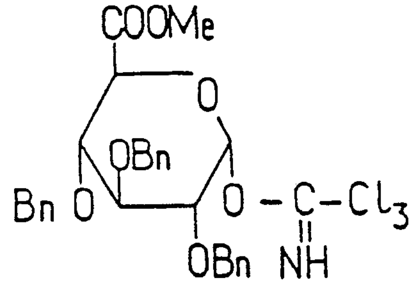

(9) (10)

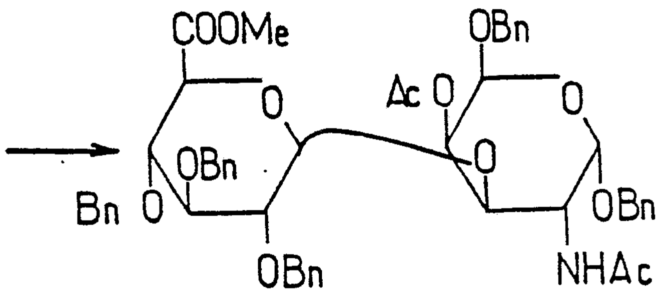 +

(11)

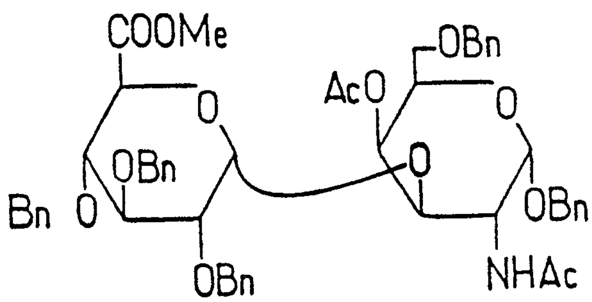

(12)

Fig. 3

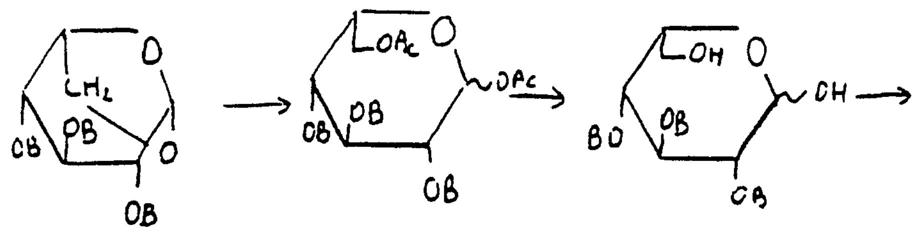

(13) (14) (15)

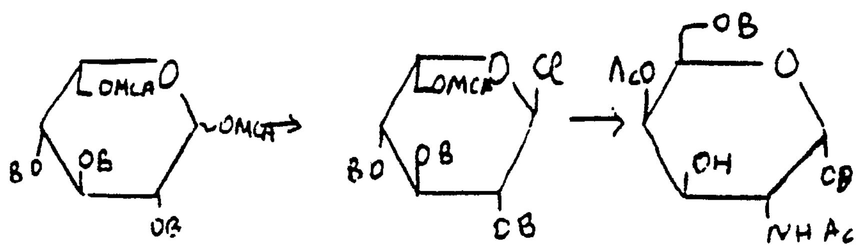

(16) (17) (18)

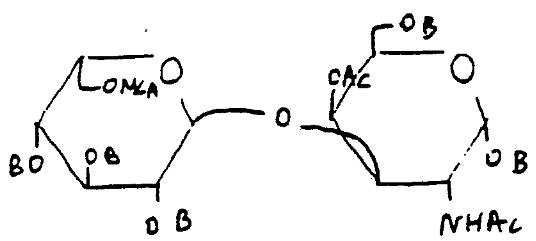

(19)

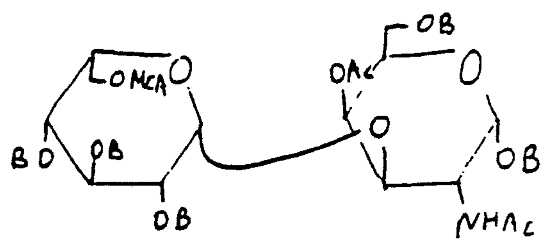

(20)

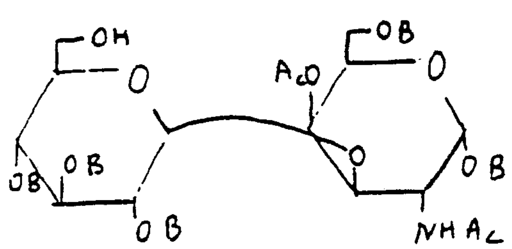

(21)

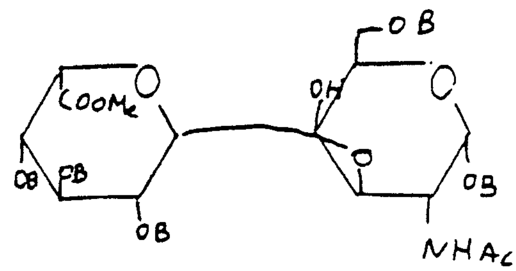

(22)

Fig. 4

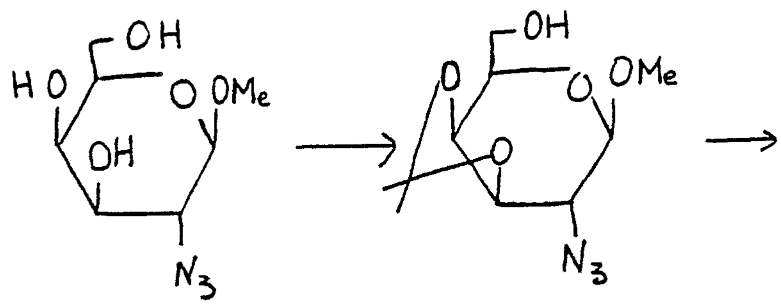

(23) (24)

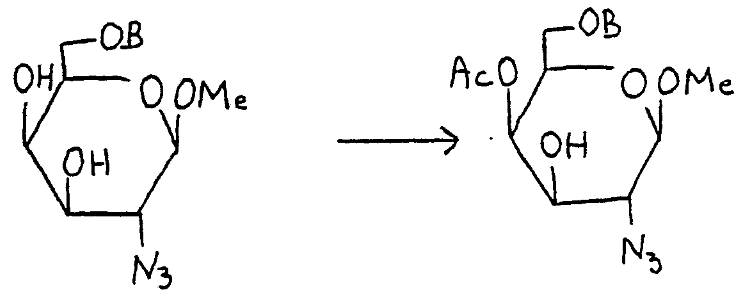

(25) (26)

Fig.5

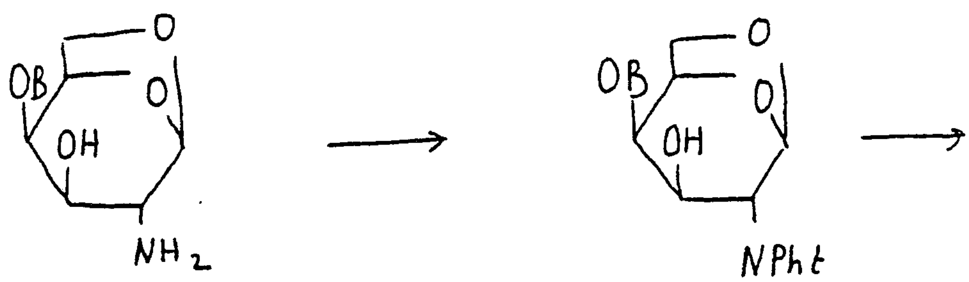

(27) (28)

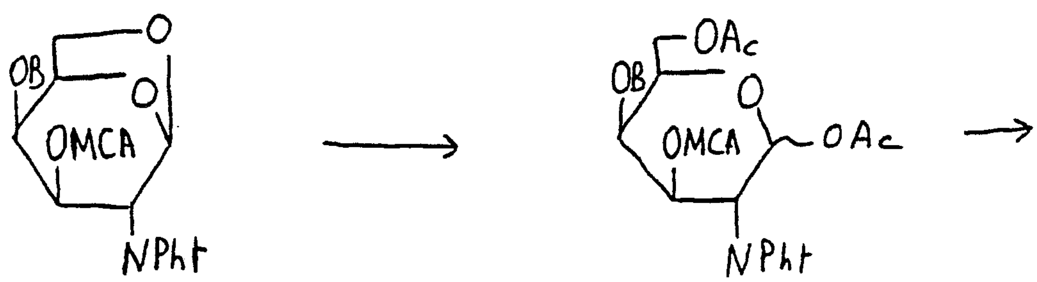

(29) (30)

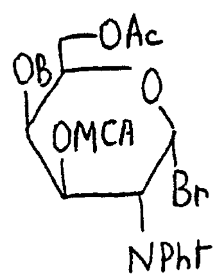

(31)

Fig. 6

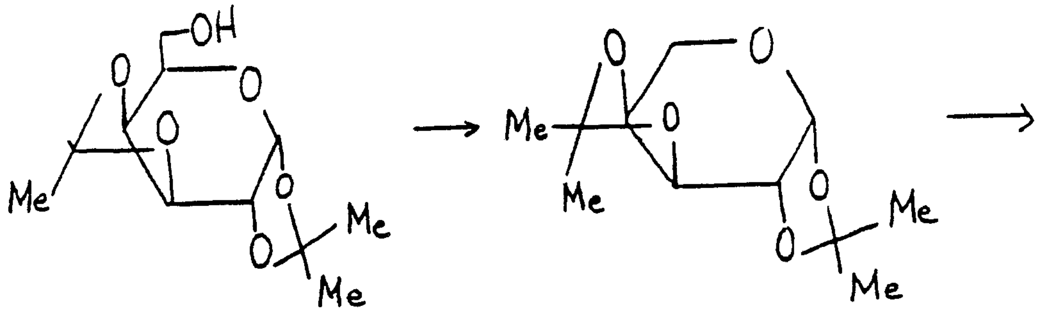

(32) (33)

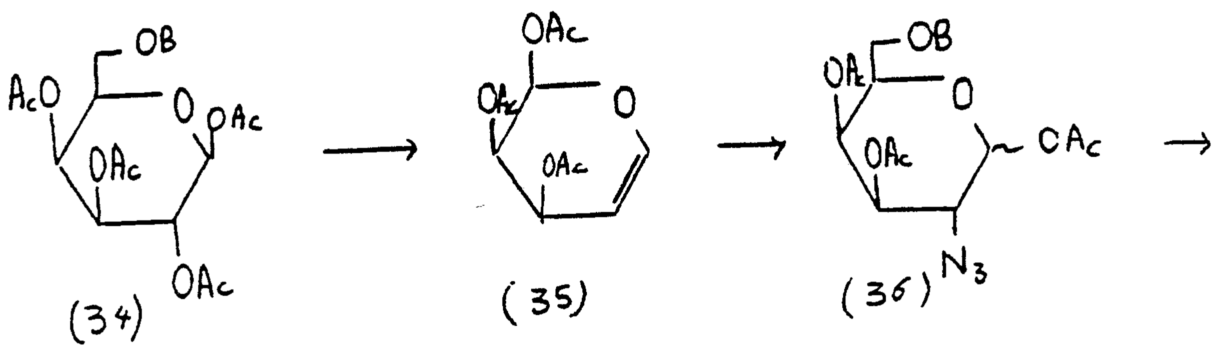

(34) (35) (36)

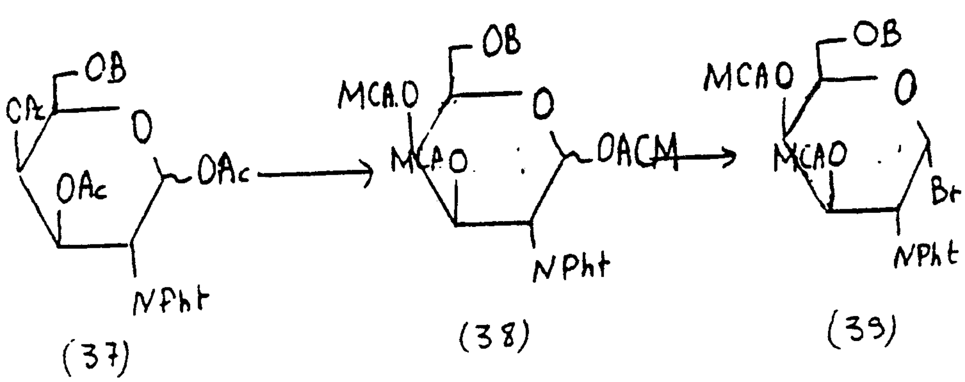

(37) (38) (39)

Fig: 7

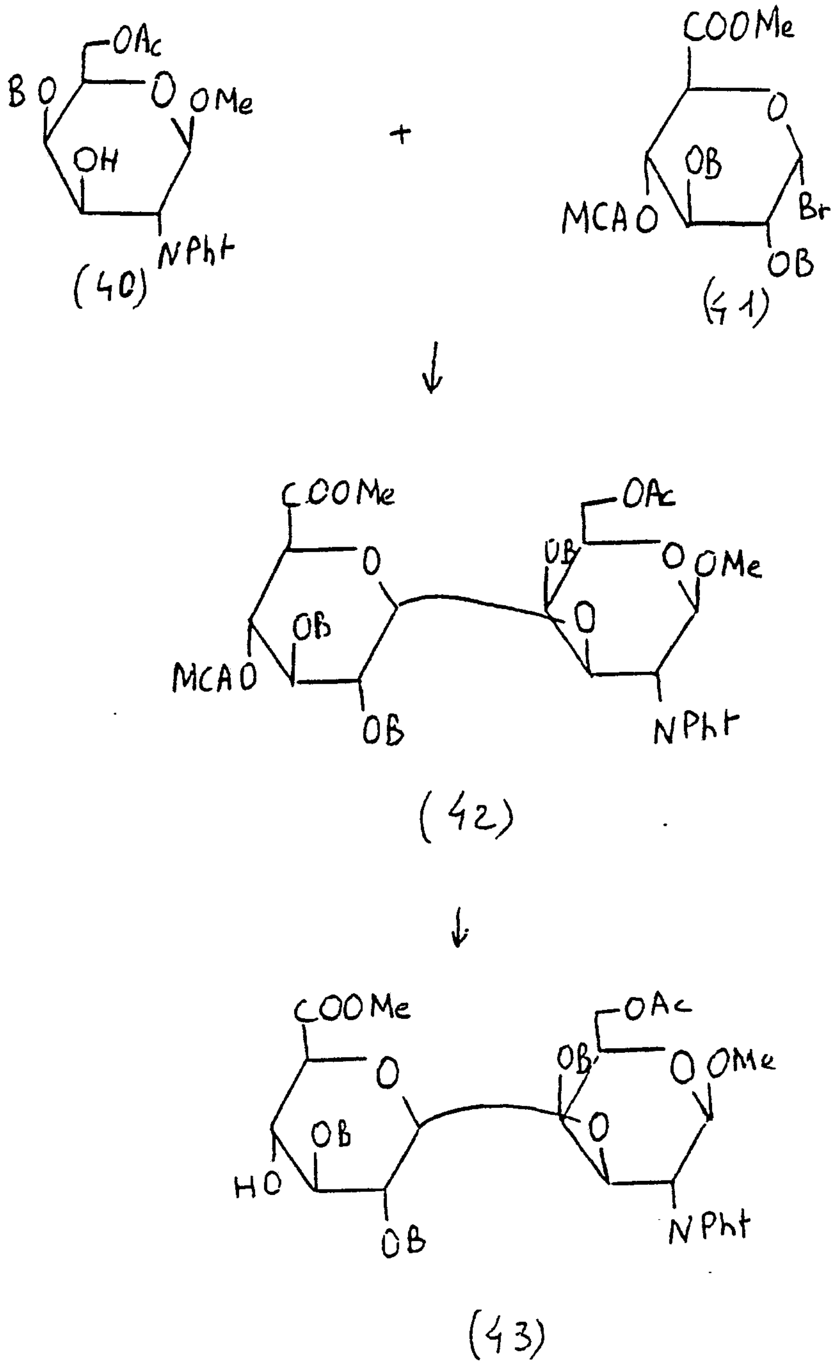

(40)

(41)

(42)

(43)

Fig. 8

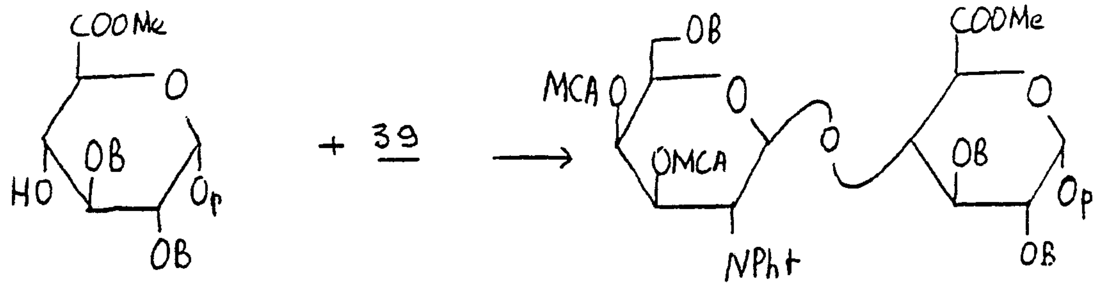


(44) (46)

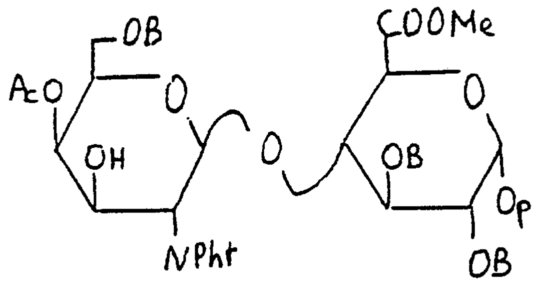

(47)

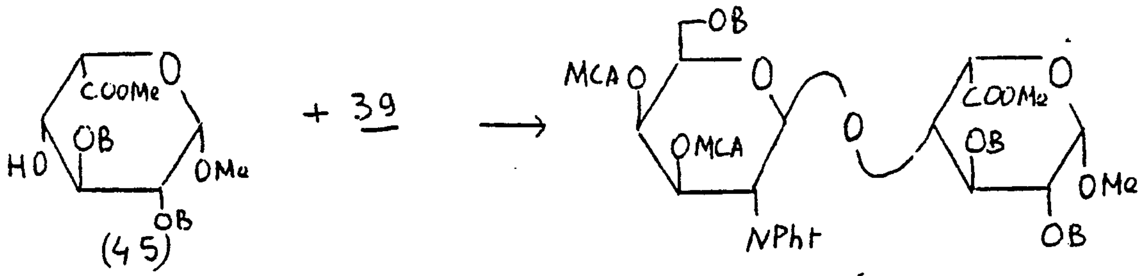


(45) (48)

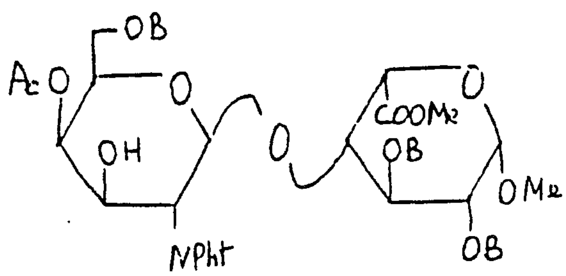

(49)

Fig. 9

27 + 44

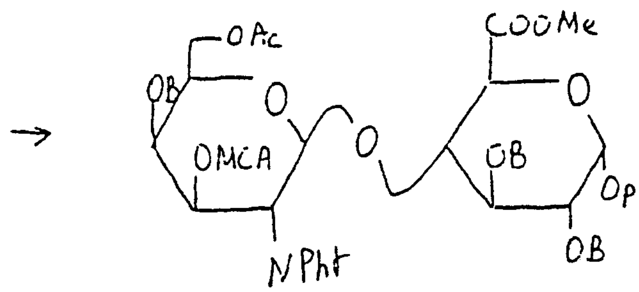

(50)

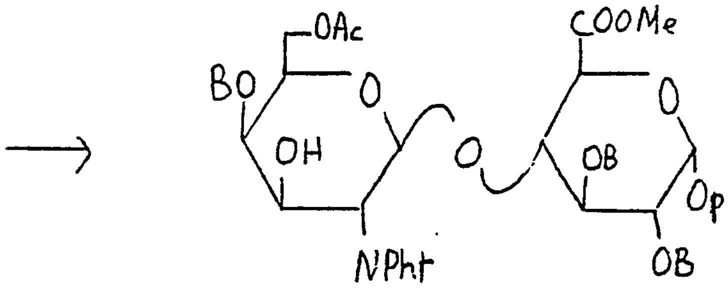

(51)

Fig. 10

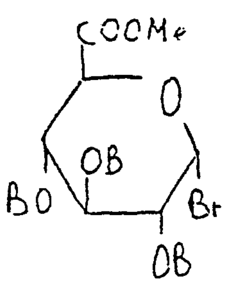

(52)

+ 29

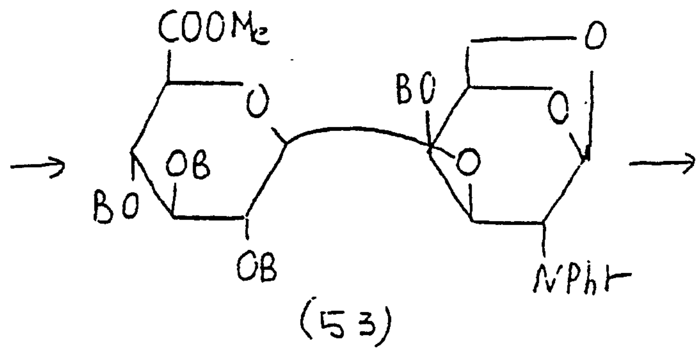

(53)

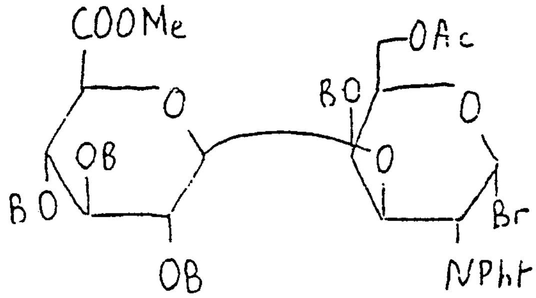

(54)

Fig. 11

54 + 44

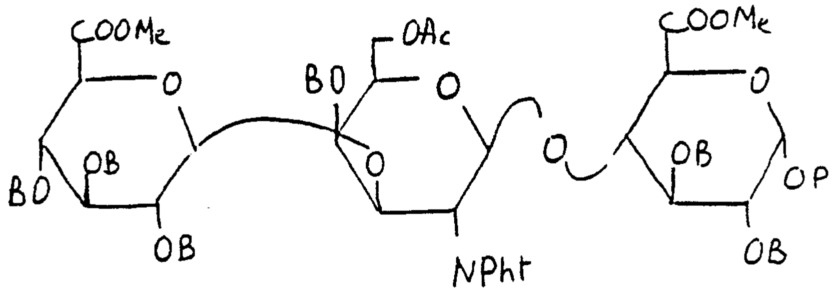

(55)

Fig. 12

54 + 43

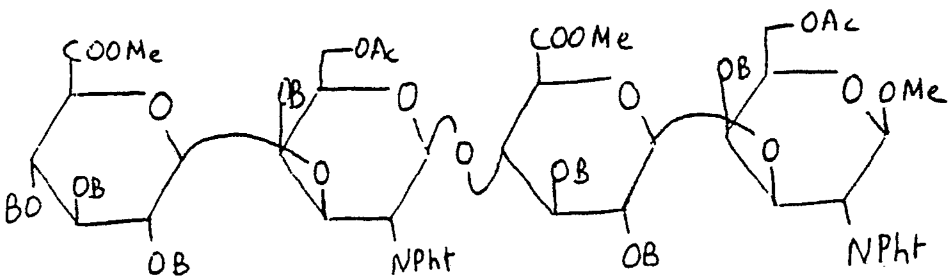

(56)